(19) Europäisches Patentamt / European Patent Office / Office européen des brevets

(11) **EP 4 501 351 B1**

(12) **EUROPEAN PATENT SPECIFICATION**

(45) Date of publication and mention of the grant of the patent:
**01.04.2026 Bulletin 2026/14**

(21) Application number: 24208680.9

(22) Date of filing: **05.02.2020**

(51) International Patent Classification (IPC):
*A61K 31/7105* (2006.01)     *A61K 35/17* (2025.01)
*A61P 35/00* (2006.01)     *C07K 14/705* (2006.01)
*C07K 14/725* (2006.01)     *C07K 16/28* (2006.01)
*C12N 15/62* (2006.01)     *C12N 15/867* (2006.01)
*A61K 39/00* (2006.01)

(52) Cooperative Patent Classification (CPC):
**A61K 31/7105; A61K 40/11; A61K 40/31; A61K 40/4264; A61P 35/00; C07K 14/705; C07K 14/7051; C07K 16/28; C12N 15/62; C12N 15/867;** A61K 2039/585; A61K 2239/31; A61K 2239/38; C07K 2319/00; C12N 2510/00

(54) **CHIMERIC ANTIGEN RECEPTOR-MODIFIED CELLS FOR THE TREATMENT OF CLDN6-EXPRESSING CANCERS**

CHIMÄRE ANTIGENREZEPTORMODIFIZIERTE ZELLEN ZUR BEHANDLUNG VON CLDN6-EXPRIMIERENDEN KREBSARTEN

CELLULES MODIFIÉES PAR UN RÉCEPTEUR ANTIGÉNIQUE CHIMÉRIQUE POUR LE TRAITEMENT DE CANCERS EXPRIMANT CLDN6

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.02.2019 PCT/EP2019/053156**

(43) Date of publication of application:
**05.02.2025 Bulletin 2025/06**

(62) Document number(s) of the earlier application(s) in accordance with Art. 76 EPC:
**20702482.9 / 3 920 939**

(73) Proprietor: **BIONTECH CELL & GENE THERAPIES GMBH**
**55131 Mainz (DE)**

(72) Inventors:
• **SAHIN, Ugur**
  **55131 Mainz (DE)**
• **OEHM, Petra**
  **55131 Mainz (DE)**
• **RENGSTL, Benjamin**
  **55131 Mainz (DE)**
• **REINHARD, Katharina**
  **55131 Mainz (DE)**
• **MICHEL, Kristina**
  **55131 Mainz (DE)**

(74) Representative: **Schnappauf, Georg**
**ZSP Patentanwälte PartG mbB**
**Hansastraße 32**
**80686 München (DE)**

(56) References cited:
WO-A1-2015/150327     WO-A1-2016/150400
WO-A1-2016/180467     WO-A1-2016/180778

Remarks:
The complete document including Reference Table(s) and the Sequence Listing(s) can be downloaded from the EPO website

**Description**

BACKGROUND

Adoptive cell transfer (ACT) based immunotherapy can be broadly defined as a form of passive immunization with previously sensitized T cells that are transferred to non-immune recipients or to the autologous host after *ex vivo* expansion from low precursor frequencies to clinically relevant cell numbers. The use of genetic engineering approaches to insert antigen-targeted receptors of defined specificity into T cells has greatly extended the potential capabilities of ACT. Chimeric antigen receptors (CARs) are a type of antigen-targeted receptor composed of intracellular T cell signaling domains fused to extracellular antigen binding domains, most commonly single-chain variable fragments (scFvs) from monoclonal antibodies. CARs directly recognize cell surface antigens, independent of MHC-mediated presentation.

Attempts in treating cancer by using genetically modified T cells to target antigens expressed on tumor cells through the expression of CARs have met with very limited success. Despite significant responses in patients with B cell malignancies, successful clinical responses after targeting of solid tumors using CAR T cells with various specificities is much more limited. CAR T cell therapy for solid tumors is faced with numerous challenges. These include physical barriers, the immunosuppressive tumor microenvironment and importantly the lack of truly specific and safe tumor targets.

Thus, there is an urgent need in the art for effective compositions and methods for treatment of cancer using CARs. The present invention addresses this need.

In order to implement a CAR based therapy for the treatment of solid cancers, we selected the oncofetal antigen CLDN6 (Claudin 6) that has all features of an ideal target for CAR based therapy. CLDN6 is a tetraspin membrane protein that is involved in the formation of primitive tight junctions during organogenesis and therefore, is exclusively expressed at significant levels during fetal development and is absent in adult healthy tissues, but highly overexpressed in different high medical need cancers including ovarian, endometrial, testicular and lung cancers.

CLDN6 has drugable extracellular loops and cell surface levels are high enough to allow for recognition by CAR T cells. Furthermore, CLDN6 expression correlates with disease progression as it could be detected with higher frequency in metastatic lesions and de-differentiated cells suggesting a role in oncogenic processes. Safety of CLDN6 targeting is suggested by a clinical phase I/II trial using the anti-CLDN6 monoclonal antibody IMAB027 in advanced ovarian carcinoma patients (OVAR, NCT02054351), where no IMAB027-related adverse events were detected.

Based on the results of our *in vitro* and *in vivo* experiments we selected a second generation CAR with a 4-1BB domain (CLDN6-CAR-CD8h-BBz) as lead structure for preclinical and clinical testing. We could demonstrate highly specific and sensitive recognition of CLDN6 expressing target cells as well as a high potential for survival and repetitive stimulation of CAR T cells.

We further evaluated the *in vivo* anti-tumoral potential of the CLDN6-CAR using an ovarian carcinoma xenograft model and could demonstrate that the adoptive transfer of CLDN6-CAR-transduced T cells resulted in complete eradication of advanced tumors. Moreover, these results could be reproduced with cryopreserved CAR T cells that were generated at the GMP facility.

As it could be demonstrated in the past, that the clinical outcome of CAR T cell therapy is positively correlated with the persistence of infused CAR T cells in the body (Robbins et al. (2004) J Immunol. 173(12):7125-30, Huang et al. (2005) 28(3):258-67), we combined the CLDN6-CAR therapy with our innovative CAR *in-vivo* expansion concept using CAR antigen encoding liposome formulated mRNA (WO 2016/180778).

Finally, we could demonstrate in different tumor models that the combination of adoptively transferred CAR T cells together with $RNA_{(LIP)}$-based vaccination accelerates ongoing anti-tumoral responses and can also restore anti-tumoral efficacy of insufficient CAR T cell doses. WO 2015/150327 discloses Claudin-6-specific immunoreceptors (T cell receptors and artificial T cell receptors (chimeric antigen receptors; CARs)) and T cell epitopes which are useful for immunotherapy.

WO 2016/180467 discloses methods and means for enhancing the effect of T cells engineered to express chimeric antigen receptors (CARs).

WO 2016/150400 discloses immune effector cells and preparation method and application of targeted CLDN6 for treating tumor cells.

SUMMARY OF THE CLAIMED INVENTION

The present invention provides a chimeric antigen receptor (CAR) molecule comprising:

i) a CLDN6 antigen binding domain, which comprises the amino acid sequence of SEQ ID NO: 35;

ii) a hinge domain, which comprises the amino acid sequence of SEQ ID NO: 27;

iii) a transmembrane domain, which comprises the amino acid sequence of SEQ ID NO: 28; and

iv) an intracellular domain that comprises a 4-1BB costimulatory domain comprising the amino acid sequence of SEQ ID NO: 30, and a CD3-zeta signaling domain comprising the amino acid sequence of SEQ ID NO: 31..

The present invention further provides a nucleic acid encoding the CAR molecule of the invention. In one embodiment, the nucleic acid is DNA or RNA.

The present invention further provides a vector comprising the nucleic acid of the invention. In one embodiment, the vector is selected from the group consisting of a DNA vector, an RNA vector, a plasmid, a lentivirus vector, an adenoviral vector, and a retrovirus vector. In one embodiment, the vector further comprises a promoter. In one embodiment, the promoter is chosen from an EF-1 promoter, a CMV IE gene promoter, an EF-1$\alpha$ promoter, an ubiquitin C promoter, or a phosphoglycerate kinase (PGK) promoter.

The present invention further provides an immune effector cell, comprising:

the CAR molecule of the invention;
the nucleic acid of the invention; or
the vector of the invention.

In one embodiment, the immune effector cell is genetically modified to express the CAR. In one embodiment, the immune effector cell is selected from the group consisting of a T cell, a Natural Killer (NK) cell, and a cytotoxic T lymphocyte (CTL). In one embodiment, the immune effector cell is a CD8+ T cell. In one embodiment, the immune effector cell is a human cell. The present invention further provides a population of immune effector cells comprising multiple immune effector cells of the invention.

In one embodiment of the immune effector cell of the invention or the population of immune effector cells of the invention the cells lack expression or have low expression of a functional TCR or a functional HLA.

The present invention further provides a method of making an immune effector cell or a population of immune effector cells of the invention, comprising introducing the nucleic acid of the invention or the vector of the invention into an immune effector cell, under conditions such that the CAR molecule is expressed.

The present invention further provides the nucleic acid of the invention, the vector of the invention, the immune effector cell of the invention, or the population of immune effector cells of the invention for use in a method of treating a cancer expressing

CLDN6, comprising providing to the subject an effective amount of an immune effector cell of the invention or a population of immune effector cells of the invention. The immune effector cell or the population of immune effector cells of the invention may be generated ex vivo and administered to the subject or the immune effector cell of the invention or the population of immune effector cells of the invention may be generated in the subject.

In one embodiment, the cancer is selected from the group consisting of ovarian cancer, lung cancer, gastric cancer, breast cancer, hepatic cancer, pancreatic cancer, skin cancer, melanomas, head neck cancer, sarcomas, bile duct cancer, renal cell cancer, and urinary bladder cancer.

The present invention further provides the nucleic acid of the invention, the vector of the invention, the immune effector cell of the invention, or the population of immune effector cells of the invention for use in a method of providing anti-tumor immunity in a subject comprising providing to the subject an effective amount of the immune effector cell of the invention or the population of immune effector cells of the invention. The immune effector cell of the invention or the population of immune effector cells of the invention may be generated ex vivo and administered to the subject or the immune effector cell of the invention or the population of immune effector cells of the invention may be generated in the subject. In one embodiment, the tumor is a CLDN6 expressing tumor.

In one embodiment, the immune effector cell of the invention or the population of immune effector cells of the invention is autologous or allogeneic to the subject.

In one embodiment, the method further comprises administering an agent that increases the efficacy of the immune effector cell of the invention or the population of immune effector cells of the invention. In one embodiment, said agent is chosen from one or more of:

a protein phosphatase inhibitor;
a kinase inhibitor;
a cytokine;
an inhibitor of an immune inhibitory molecule; or
an agent that decreases the level or activity of a TREG cell.

In one embodiment, the method further comprises the step of contacting the immune effector cell of the invention or the population of immune effector cells of the invention, either ex vivo or in the subject, with a cognate antigen molecule binding to the CLDN6 antigen binding domain. In one embodiment, the cognate antigen molecule is selected from the group consisting of CLDN6 or a fragment thereof, or a variant of CLDN6 or a CLDN6 fragment. In one embodiment, the immune effector cell of the invention or the population of immune effector cells of the invention is contacted with the cognate antigen molecule under conditions such that expansion and/or activation of the immune effector cell of the invention or the population of immune effector cells of the invention occurs. In one embodiment, the step of contacting the immune effector cell of the invention or the population of immune effector cells of the invention with the cognate antigen molecule takes place *in vivo* or *ex vivo*.

In one embodiment, the method comprises the step of administering the cognate antigen molecule or a nucleic acid coding therefor to the subject. In one embodiment, the nucleic acid encoding the cognate antigen molecule is expressed in cells of the subject to provide the cognate antigen molecule. In one embodiment, expression of the cognate antigen molecule is at the cell surface. In one embodiment, the nucleic acid encoding the cognate antigen molecule is transiently expressed in cells of the subject. In one embodiment, the nucleic encoding the cognate antigen molecule is RNA. In one embodiment, the immune effector cell of the invention or the population of immune effector cells of the invention and/or the cognate antigen molecule or the nucleic acid coding therefor are administered systemically. In one embodiment, after systemic administration of the nucleic acid encoding the cognate antigen molecule, expression of the nucleic acid encoding the cognate antigen molecule in spleen occurs. In one embodiment, after systemic administration of the nucleic acid encoding the cognate antigen molecule, expression of the nucleic acid encoding the cognate antigen molecule in antigen presenting cells, preferably professional antigen presenting cells occurs. In one embodiment, the antigen presenting cells are selected from the group consisting of dendritic cells, macrophages and B cells. In one embodiment, after systemic administration of the nucleic acid encoding the cognate antigen molecule, no or essentially no expression of the nucleic acid encoding the cognate antigen molecule in lung and/or liver occurs. In one embodiment, after systemic administration of the nucleic acid encoding the cognate antigen molecule, expression of the nucleic acid encoding the cognate antigen molecule in spleen is at least 5-fold the amount of expression in lung.

In one embodiment, the nucleic acid encoding the cognate antigen molecule is formulated in a delivery vehicle. In one embodiment, the delivery vehicle comprises particles. In one embodiment, the delivery vehicle comprises at least one lipid. In one embodiment, the at least one lipid comprises at least one cationic lipid. In one embodiment, the lipid forms a complex with and/or encapsulates the nucleic acid encoding the cognate antigen molecule. In one embodiment, the lipid is comprised in a vesicle encapsulating the nucleic acid encoding the cognate antigen molecule. In one embodiment, the nucleic acid encoding the cognate antigen molecule is formulated in liposomes.

The present invention further provides the nucleic acid of the invention, the vector of the invention, the immune effector cell of the invention, or the population of immune effector cells of the invention for use as a medicament.

The present invention further provides the nucleic acid of the invention, the vector of the invention, the immune effector cell of the invention, or the population of immune effector cells of the invention for use in the treatment of a cancer expressing CLDN6.

The present invention further provides a kit comprising the nucleic acid of the invention, the vector of the invention, the immune effector cell of the invention, or the population of immune effector cells of the invention. In one embodiment, the kit further comprises a cognate antigen molecule binding to the CLDN6 antigen binding domain or a nucleic acid coding therefor. In one embodiment, the kit further comprises instructions for use of the kit in the methods disclosed herein.

The invention is limited by the appended claims.

BRIEF DESCRIPTION OF THE DRAWINGS

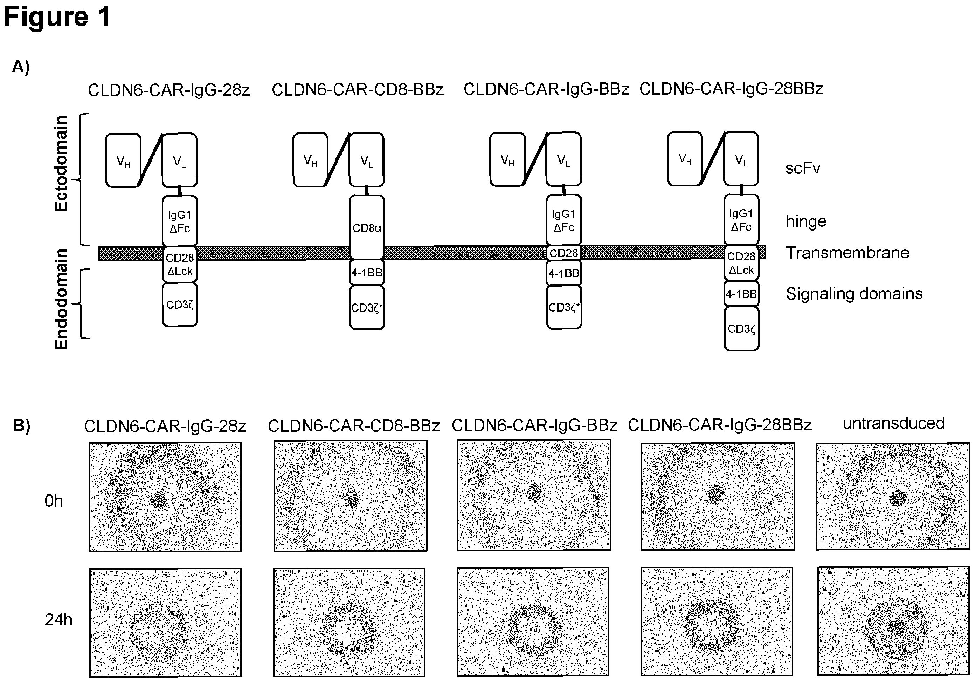

**Figure 1: Generation and characterization of different CLDN6-CARs.**
A) Four different CLDN6-targeting CARs have been designed and generated based on the variable domains of the heavy ($V_H$) and the light ($V_L$) chain of the CLDN6-specific antibody IMAB206-C46S. IgG1ΔFc: human IgG1 hinge-CH2-CH3 Fc domain with mutated IgG Fcγ receptor (FcγR) binding sites to prevent activation by FcγR expressing innate immune cells (Hombach A. et al., (2010) Gene Therapy 17, 1206-1213); CD28ΔLck: CD28 transmembrane and cytoplasmic domain with a deletion into the Lck binding moiety of the CD28 endodomain abrogating IL-2 induction upon CAR engagement to prevent unwanted Treg cell expansion at the tumor site (Kofler D.M. et al., (2011) Molecular Therapy 19 (4), 760-767); 4-1BB: 4-1BB costimulatory endodomain; CD3ζ: CD3ζ signaling domain; CD3ζ*: CD3ζ with mutation Q14→K; CD8α: human CD8α hinge domain. B) Functional testing of different CLDN6-CARs in a PA1-SC12-A2-eGFP tumor spheroid assay. Lysis of tumor spheroids was analyzed based on eGFP expression after 24 h of coculture (E:T = 10:1) using the IncuCyte® live-cell imaging system. Images of wells were scanned with 4x objective lenses at start of co-culture (0h) and 24h thereafter and represent tumor spheroid killing of technical triplicates.

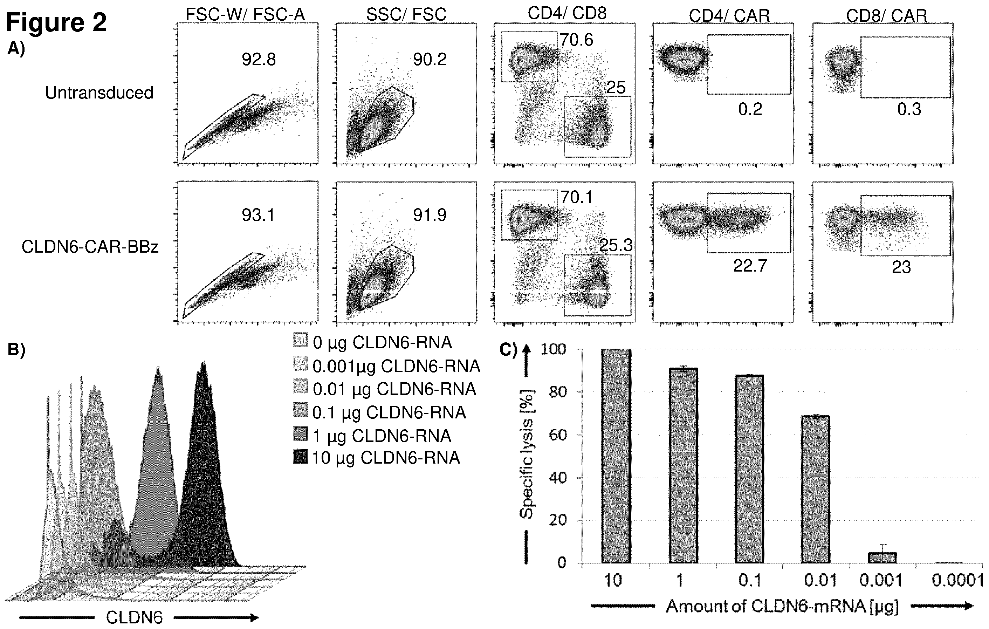

**Figure 2: Dose-dependent CAR-mediated recognition and lysis of CLDN6-expressing target cells.**

A) CLDN6-CAR-BBz surface expression was analyzed on transduced T cells after staining with a fluorochrome-conjugated IMAB206-idiotype-specific antibody. Untransduced T cells served as a negative control. Cells were gated on single CD4[+] or CD8[+] lymphocytes. Indicated numbers represent the frequency of parent population (in %). B) CLDN6 surface expression on Colo699-N cells transfected with titrated amounts of CLDN6-RNA was analyzed by flow cytometry. C) The specific lysis of RNA-transfected Colo699-N cells by CLDN6-CAR-BBz transduced T cells was analyzed after 12 h co-culture with an E:T ratio of 20:1 using the xCELLigence device. Data are presented as mean ±SD of technical triplicates.

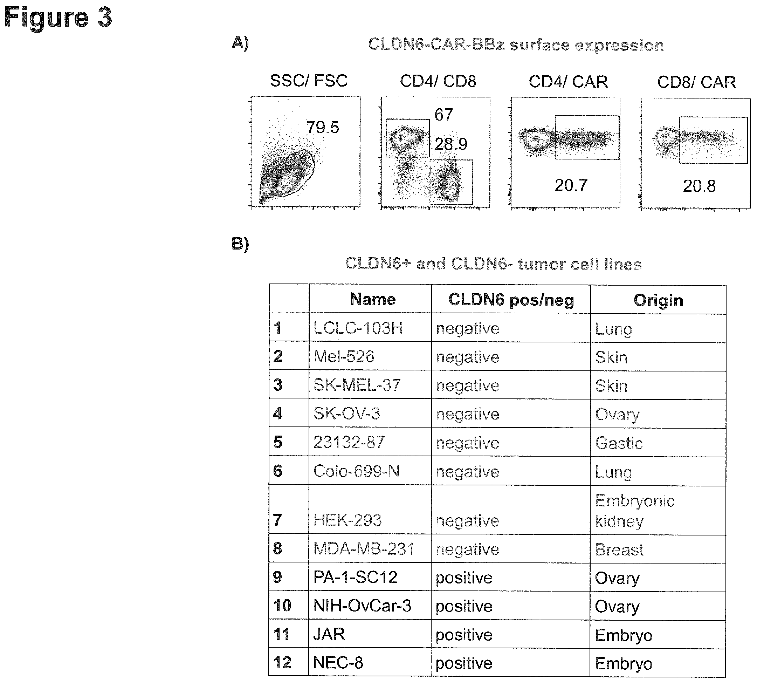

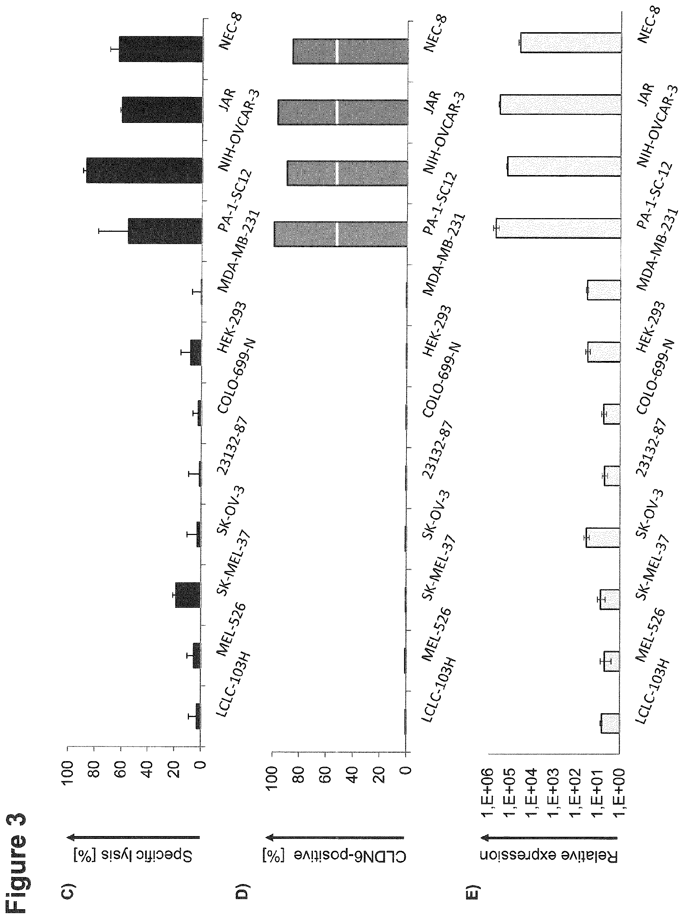

**Figure 3: Specific CLDN6-CAR-mediated lysis of CLDN6-expressing tumor cell lines.**
CLDN6-CAR-BBz transduced human T cells were analyzed by flow cytometry and co-cultured with a panel of CLDN6-postitive and -negative human tumor cell lines of different origin. A) CAR surface expression was assessed by flow cytometry before co-culture was initiated. Indicated numbers represent the frequency of parent population (in %). B) using an E:T ratio of 10:1. C) The specific lysis was analyzed using the xCELLigence system after 12 h co-culture according to the formula % lysis = (CI eGFP -CI effector)/CI eGFP *100; CI: cell index. Data represent mean±SD of technical triplicates D) CLDN6 surface expression on tumor cell lines was analyzed by flow cytometry after staining with a CLDN6-specific antibody. Percentage of parent population is depicted. E) Relative CLDN6 mRNA expression levels in tumor cells lines used in (A-D) as assessed by qRT-PCR were calculated after normalization to the housekeeping gene *HPRT1.* Bars represent mean±SD of technical triplicates.

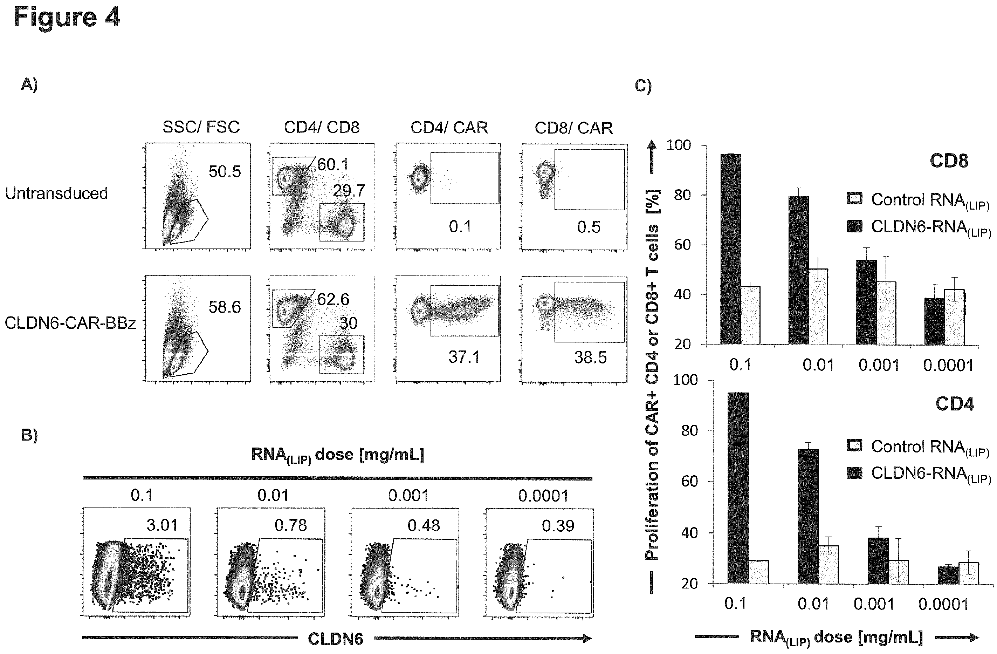

**Figure 4: Dose-dependent proliferation mediated by CLDN6-CAR-BBz in response to CLDN6-expressing target cells.**
CAR-transduced T cells were labeled with CFSE and co-cultured with autologous DCs transfected with titrated amounts of CLDN6-RNA lipoplexes (RNA$_{(LIP)}$). Proliferation was analyzed based on CFSE after 5 days of co-culture and staining with fluorochrome-conjugated antibodies against CD4, CD8 and the CAR. A) CAR surface expression was assessed by flow cytometry before co-culture was initiated. Indicated numbers represent the frequency of parent population (in %). B) CLDN6 expression on the surface of transfected DCs was assessed by flow cytometry using a fluorochrome-conjugated CLDN6-specific antibody. C) Specific proliferation was analyzed by flow cytometry based on the dilution of the CFSE proliferation dye. Bars show the percentage of proliferating CAR-expressing CD8[+] and CD4[+] T cells.

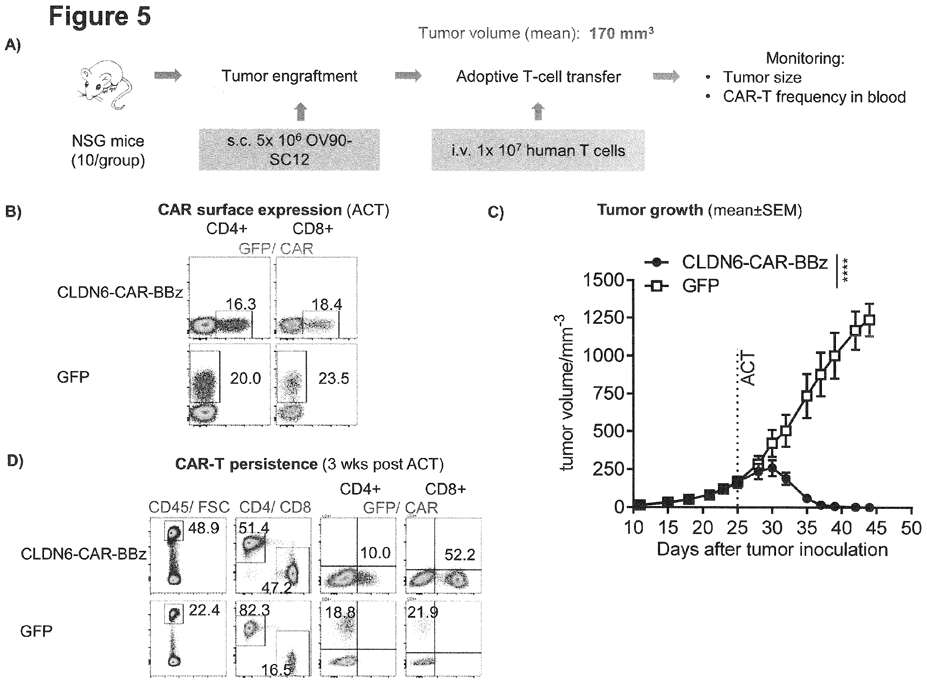

**Figure 5: Anti-tumoral activity CLDN6-CAR-BBz transduced T cells in an advanced ovarian cancer (OV90) xenograft tumor model.**
5x10[6] OV90-SC12 tumor cells were subcutaneously engrafted 25 days prior to adoptive cell transfer (ACT) of a single dose of 1x10[7] i.v. administered CLDN6-CAR-BBz or eGFP transduced human T cells (n=10/group). At this time point of T cell treatment, mice already presented with advanced tumors 170 mm[3] in avarage. Tumor volume was determined three times weekly using a caliper and calculated according to the formula V = ½ (length x width[2]) with maximum length and width of tumors. Animals were sacrificed when the tumor volume exceeded 1500 mm[3] or when the tumor ulcerated. A) Schematic overview of the conducted mouse experiment. B) Transgene expression (GFP or CAR) of transduced human CD4[+] and CD8[+] T cells was analyzed by flow cytometry at the day of ACT. Indicated numbers represent the frequency of parent population (in %). C) Mean tumor volumes in CLDN6-CAR-BBz and eGFP-T cells treated animals after ACT (indicated as dotted line) until day 44 are shown. Data are presented as mean±SEM all mice/group. D) T cell persistence and CAR surface expression was analyzed in peripheral blood 3 weeks after ACT using flow cytometry. Representative dot plots are shown. Numbers indicated the frequency of parental populations.

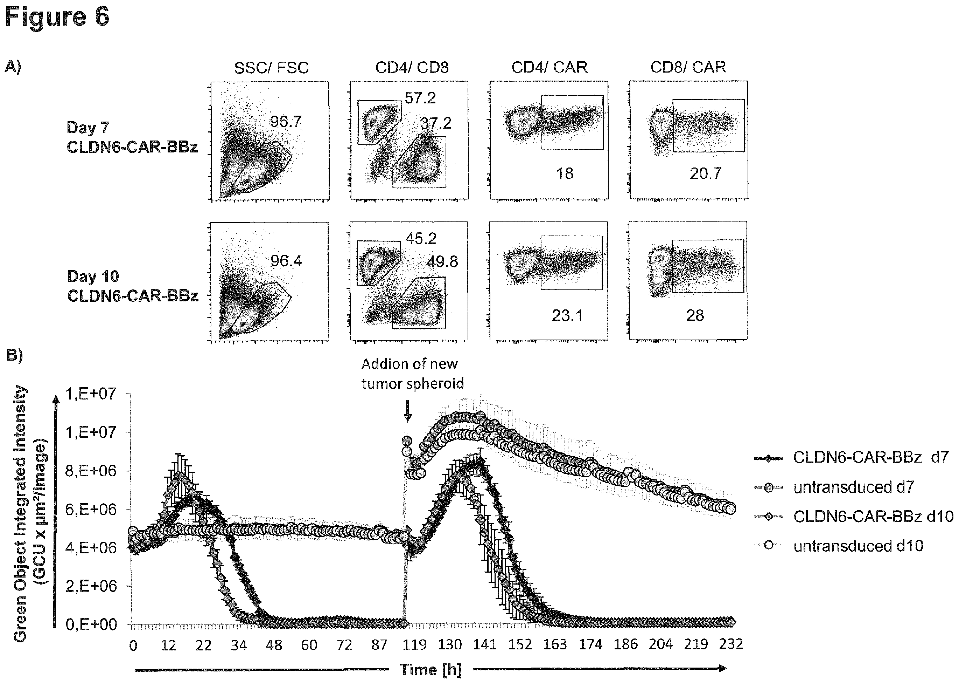

**Figure 6: Repetitive elimination of tumor spheroids by 7-day and 10-day cultured CLDN6-CAR T cells.**
CLDN6-CAR-transduced T cells generated within 7 and 10 days, respectively, were evaluated for their potential to repetitively kill CLDN6 and eGFP expressing tumor spheroids as assessed by live-cell imaging. A) CAR surface expression of 7-day and 10-day cultured CLDN6-CAR T cells was analyzed on transduced T cells after staining with a fluorochrome-conjugated IMAB206-idiotype-specific antibody. Untransduced T cells served as a negative control. Cells were gated on single CD4[+] or CD8[+] lymphocytes. Indicated numbers represent the frequency of parent population (in %). B) Functional testing of 7-day and 10-day cultured CLDN6-CAR T cells was performed using the PA1-SC12-A2-eGFP tumor spheroid assay. Lysis of tumor spheroids was analyzed based on eGFP expression using the IncuCyte® live-cell imaging system over a total time period of 10 days. After 5 days a new tumor spheroid was added. Values represent the green object integrated intensities of technical triplicates as mean±SD.

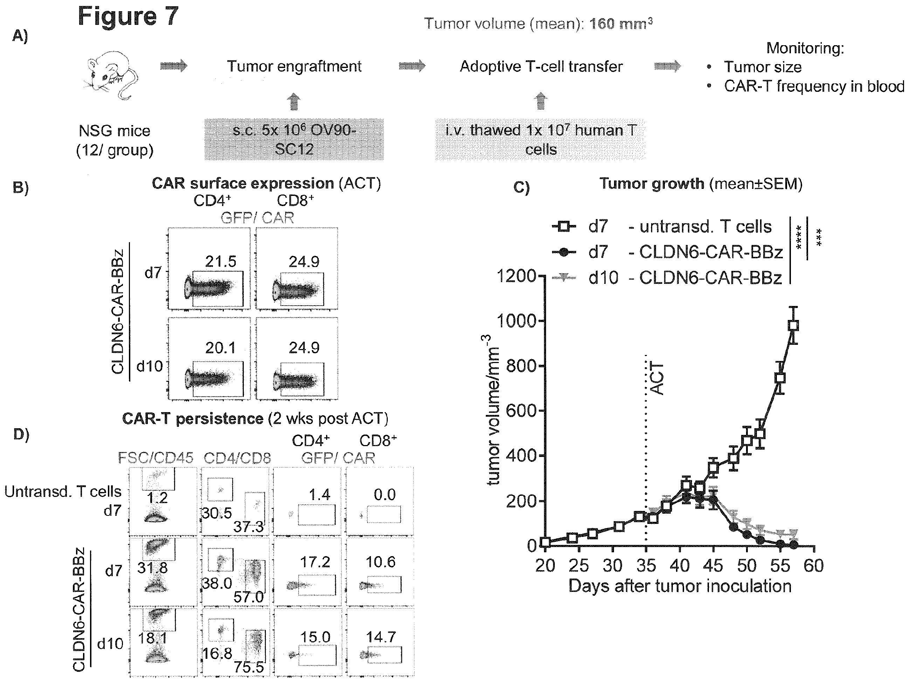

**Figure 7: Anti-tumoral Efficacy of GMP-Manufactured CLDN6-CAR T cells**
5x10[6] OV90-SC12 tumor cells were subcutaneously engrafted 35 days prior to adoptive transfer of a single dose either 1x10[7] CLDN6-CAR-BBz or non-transduced human T cells *i.v.* (n=12/group). Used T cell products were manufactured at the GMP facility in an either standard (ten *in vitro* cultured days) or in a shortened manufacturing procedure (harvested already after seven days). Non-transduced T cells, which were treated in the same way as transduced T cells, were used as negative control. Indicated T cell products were thawed, washed with PBS and directly injection into the mice, which already presented with advanced tumors of 160 mm$^3$ in average. Tumor volume was determined three times weekly using a caliper and calculated according to the formula V = ½ (length x width$^2$) with maximum length and width of tumors. Animals were sacrificed when the tumor volume exceeded 1500 mm$^3$ or when the tumor ulcerated. A) Schematic overview of the conducted mouse experiment. B) The CAR surface expression on transduced human CD4$^+$ and CD8$^+$ T cells was analyzed by flow cytometry at the day of ACT. Indicated numbers represent the frequency of parent population (in %). C) Tumor volumes in CLDN6-CAR-BBz and control T cells-treated animals until day 57 are shown. Data are presented as mean±SEM of all mice/group. D) T cell persistence and CAR surface expression was analyzed in peripheral blood 2 weeks after ACT using flow cytometry. Representative dot plots are shown. Numbers indicated the frequency of parental populations.

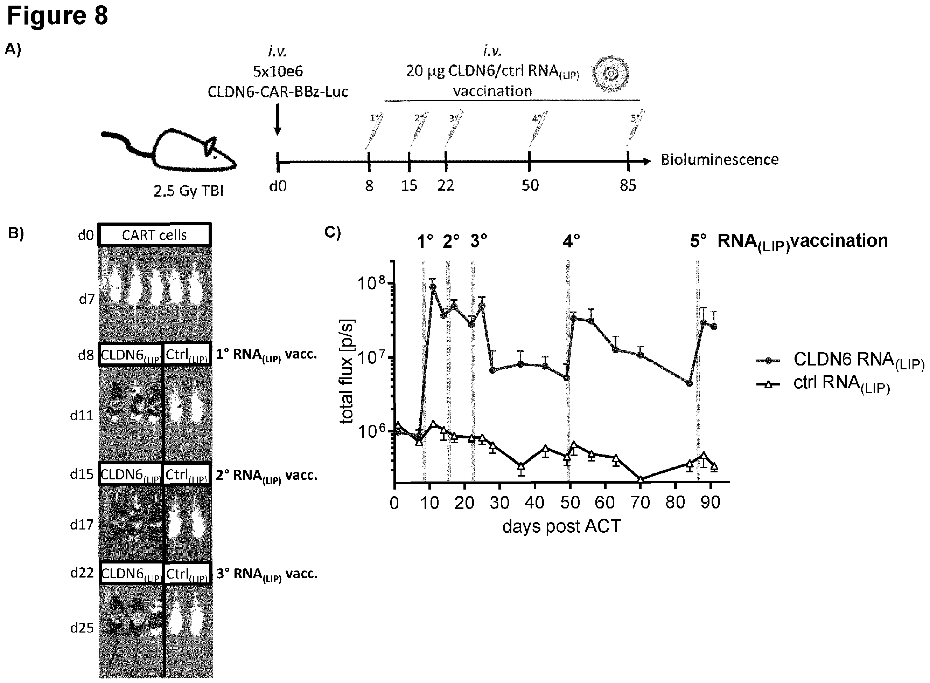

**Figure 8: *In vivo* expansion via RNA$_{(LIP)}$ leads to enhanced persistence of CLDN6-CAR-BBz T cells**

2.5 Gy irradiated (XRAD320) C57BL/6BrdCrHsd-*Tyr$^c$* mice (n = 2-3/group) were *i.v.* engrafted with 5 x 10$^6$ CLDN6-CAR-BBz-Luc-GFP transduced C57BI/6-Thy1.1$^+$ T cells. 8 days after ACT, mice received hCLDN6 or Oval (ctrl RNA) encoding mRNA lipoplex vaccination (RNA$_{(LIP)}$; 20 μg, *i.v.*) followed by *i.p.* administration of nucleoside-modified-formulated RNA encoding murine albumin (1 μg/ mRNA/ mouse). Vaccination was repeated at day 15, 22, 50 and 85. Sequential bioluminescence imaging was performed to monitor expansion and persistence on day 1 (baseline) up to day 92 after ACT. A) Schematic overview of the conducted mouse experiment. B) Example of bioluminescence imaging of mice in lateral position at the indicated time point after ACT and treatment with antigen RNA$_{(LIP)}$. Off-color images represent light intensity (black, least intense; white up to dark-grey, most intense) which was superimposed over the greyscale reference images. C) Quantification of bioluminescence during and after the expansion rounds with CLDN6-RNA$_{(LIP)}$/ctrl-RNA$_{(LIP)}$ in the presence of indicated nucleoside-modified-formulated cytokine RNAs are shown (mean+/- s.e.m.). Gray, vertical lines indicate the time point of **RNA$_{(LIP)}$** vaccination.ACT: adoptive T cell transfer, TBI: total body irradiation, BLI: bioluminescence imaging, Luc: effective firefly luciferase, BBz: 4-1BB; z: CD3zeta.

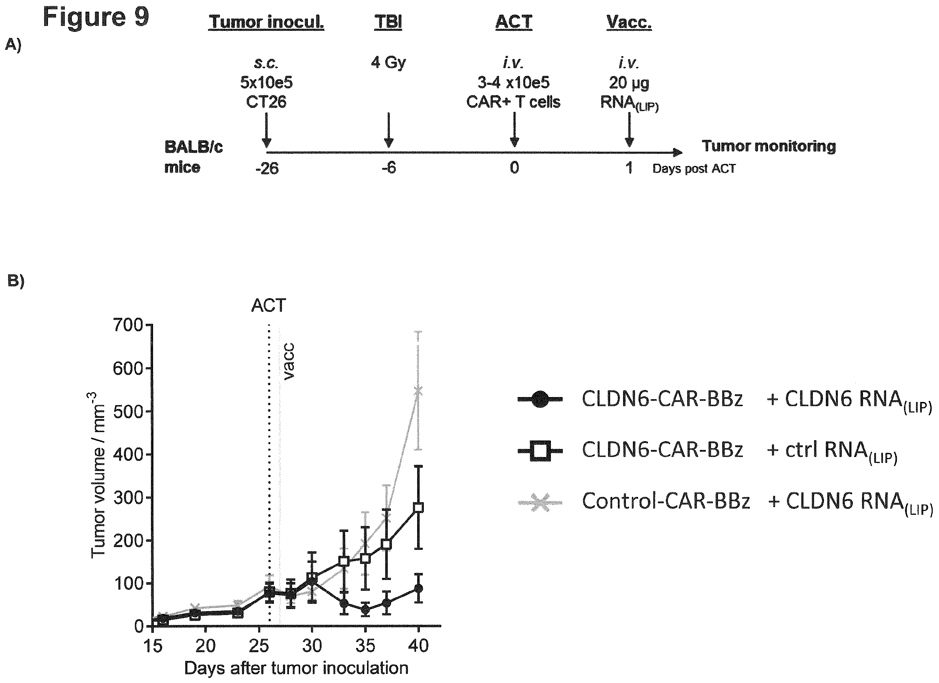

**Figure 9: Improved anti-tumoral activity of *in vivo* expanded CLDN6-CAR-BBz T cells**

5x10$^5$ CT26 tumor cells were subcutaneously engrafted into Balb/c mice 20 days before 4 Gy total body irradiation and 26 days prior to ACT of a single dose of 1x10$^6$ CLDN6-CAR-BBz or ctrl-CAR-BBz transduced Balb/c-Thy1.1+ T cells i.v. (n=10/group). At time point of T cell treatment, mice presented with established tumors of approx. 80 mm$^3$ in avargae. Tumor volume was determined three times weekly using a caliper and calculated according to the formula V = ½ (length x width$^2$) with maximum length and width of tumors. A) Schematic overview of the conducted mouse experiment. B) Mean tumor volumes in CLDN6-CAR-BBz and ctrl-CAR-BBz-T cells treated animals until day 40 are shown. Data are presented as mean±SEM all mice/group. ACT is indicated as dotted line and RNA$_{(LIP)}$ treatment as grey line.

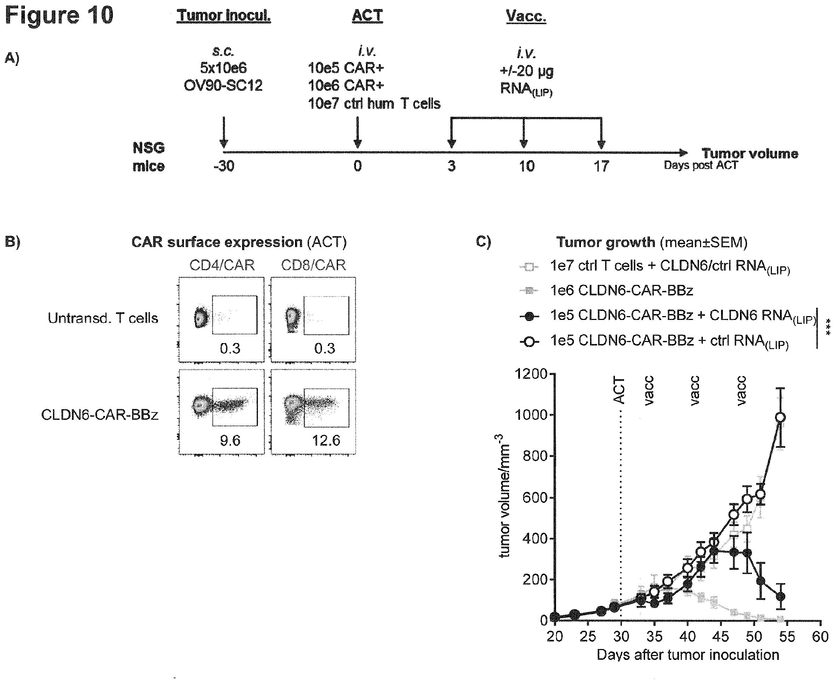

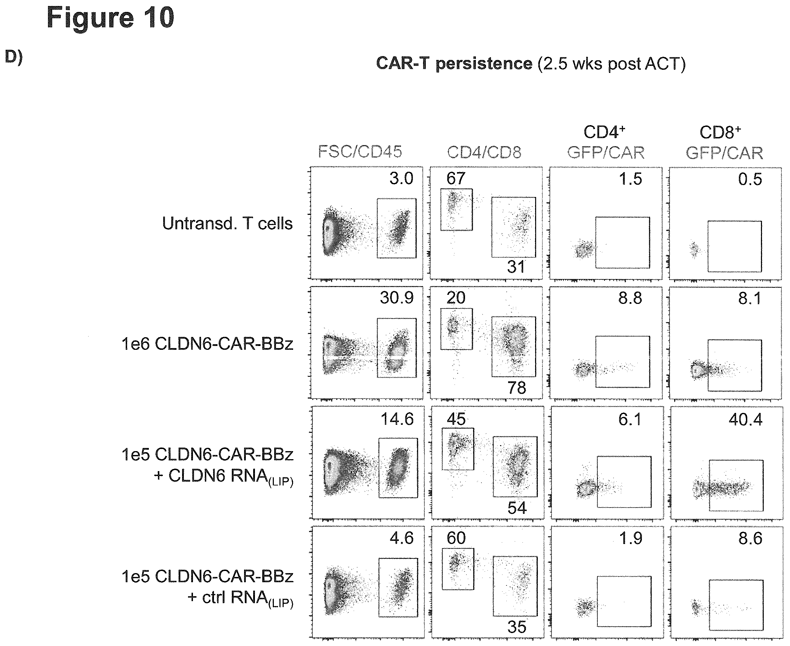

**Figure 10: Restored anti-tumoral efficacy of low-dose *in vivo* expanded CLDN6-CAR T cell** 5x10$^6$ OV90-SC12 tumor cells were subcutaneously engrafted 30 days prior to ACT of a single dose of either low-dose (1x10$^5$) or high-dose (1x10$^6$) CLDN6-CAR-BBz or 1x10$^7$ non-transduced human T cells *i.v.* (n=9/group). Used T cell products were manufactured according to the shortened transduction process (harvested already after 7 days) at the GMP facility. Non-transduced T cells, which were treated in the same way as transduced T cells, were used as negative control. Indicated T cell products were thawed, washed with PBS and directly injection into tumor-bearing mice. In addition, mice that received a low-dose of CAR T cells were further vaccinated with 20μg RNA$_{(LIP)}$ either encoding CLDN6 or a control antigen as indicated. Tumor volume was determined three times weekly using a caliper and calculated according to the formula V = ½ (length x width$^2$) with maximum length and width of tumors. Animals were sacrificed when the tumor volume exceeded 1500 mm$^3$ or when the tumor ulcerated. A) Schematic overview of the conducted mouse experiment. B) The CAR surface expression on transduced human CD4$^+$ and CD8$^+$ T cells was analyzed by flow cytometry at the day of adoptive transfer. Indicated numbers represent the frequency of parent population (in %). C) Tumor growth curves of animals treated with different doses of CLDN6-CAR-BBz +/- RNA$_{(LIP)}$ or control T cells. Data are presented as mean±SEM of all mice/group. ACT is indicated as dotted line and RNA$_{(LIP)}$ treatment as grey lines. D) T cell persistence and CAR surface expression was analyzed in peripheral blood 2.5 weeks after ACT using flow cytometry. Representative dot plots are shown. Numbers indicated the frequency of parental populations.

GENERAL TEACHING

The present disclosure provides teachings which in some respects go beyond the invention as such which is defined exclusively by the appended claims. The teachings are provided to place the actual invention in a broader technical context and to illustrate possible related technical developments. Such additional technical information which does not fall within the scope of the appended claims, is not part of the invention. In particular, the terms "embodiment" and "aspect" are not to be construed as necessarily referring to an embodiment of the invention, unless the "embodiment" or "aspect" in question falls within the scope of the claims.

The disclosure relates to compositions and methods for treating cancer including but not limited to solid tumors. The present disclosure relates to a strategy of adoptive cell transfer of cells such as T cells transduced to express a CAR. CARs are molecules that combine specificity for a desired antigen (e.g., tumor antigen) which preferably is antibody-based with a T cell receptor-activating intracellular domain to generate a chimeric protein that exhibits a specific cellular immune activity (e.g., a specific anti-tumor cellular immune activity). Preferably, a cell can be genetically modified to stably express a CAR on its surface, conferring novel antigen specificity that is MHC independent. The present disclosure relates generally to T cells genetically modified to stably express a desired CAR. T cells expressing a CAR are referred to herein as CAR T cells or CAR-modified T cells.

A CAR of the disclosure combines a CLDN6 antigen binding domain, preferably a domain of a specific antibody, and an intracellular domain that comprises a 4-1BB costimulatory domain and a domain of the CD3-zeta chain into a single chimeric protein. In one embodiment, the CAR of the disclosure comprises an extracellular domain comprising a CLDN6 antigen binding domain, a transmembrane domain, and an intracellular domain that comprises a 4-1BB costimulatory domain and a domain of the CD3-zeta chain. In one embodiment, the transmembrane domain is not naturally associated with one of the domains in the CAR. In one embodiment, the transmembrane domain is naturally associated with one of the domains in the CAR. In one embodiment, the transmembrane domain is modified by amino acid substitution to avoid binding of such domains to the transmembrane domains of the same or different surface membrane proteins to minimize interactions with other members of the receptor complex. Preferably, the transmembrane domain is derived from CD8$\alpha$.

The disclosure further provides CAR T cells and methods of their use for adoptive therapy. In one embodiment, the CAR T cells of the disclosure can be generated by introducing a retroviral such as lentiviral vector comprising a desired CAR, for example a CAR comprising anti-CLDN6, CD8$\alpha$ hinge and transmembrane domain, and 4-1BB and CD3zeta signaling domains, into the cells. The CAR T cells of the disclosure are preferably able to replicate in *vivo* resulting in long-term persistence that can lead to sustained tumor control.

In one embodiment the disclosure relates to administering a genetically modified T cell expressing a desired CAR for the treatment of a patient having cancer or at risk of having cancer. Preferably, autologous cells are used in the treatment. In one embodiment, autologous PBMCs are collected from a patient in need of treatment and T cells are activated and expanded using the methods described herein and known in the art and then infused back into the patient.

In one embodiment, the disclosure relates generally to the treatment of a patient having or at risk of developing CLDN6 expressing cancer. The disclosure includes using T cells expressing an anti-CLDN6-CAR including both CD3-zeta and the 4-1BB costimulatory domain. The CAR T cells of the disclosure can undergo robust *in vivo* T cell expansion, in particular if contacted with their cognate antigen, and can persist at high levels for an extended amount of time. In some instances, the CAR T cells of the disclosure infused into a patient can eliminate cancer cells *in vivo* in patients.

Definitions

Although the present disclosure is described in detail below, it is to be understood that this disclosure is not limited to the particular methodologies, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

In the following, the elements of the present disclosure will be described. These elements are listed with specific embodiments, however, it should be understood that they may be combined in any manner and in any number to create additional embodiments. The variously described examples and preferred embodiments should not be construed to limit the present disclosure to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Unless defined otherwise, all technical and scientific terms used herein have the same meaning as commonly understood by one of ordinary skill in the art to which the disclosure pertains. Although any methods and materials similar or equivalent to those described herein can be used in the practice for testing of the present disclosure, the preferred materials and methods are described herein. In describing and claiming the present disclosure, the following terminology will be used.

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e., the subject-matter consists in the inclusion of a stated member, integer or step or group of members, integers or steps.

The articles "a" and "an" are used herein to refer to one or to more than one (i.e., to at least one) of the grammatical object of the article. By way of example, "an element" means one element or more than one element.

"About" as used herein when referring to a measurable value such as an amount, a temporal duration, and the like, is meant to encompass variations of $\pm 20\%$ or $\pm 10\%$, more preferably $\pm 5\%$, even more preferably $\pm 1\%$, and still more preferably $\pm 0.1\%$ from the specified value.

The term "antibody", as used herein, refers to an immunoglobulin molecule which binds, preferably specifically binds with an antigen. Antibodies can be intact immunoglobulins derived from natural sources or from recombinant sources and can be immunoreactive portions or fragments of intact immunoglobulins. Antibodies are typically tetramers of immunoglobulin molecules. The antibodies in the present disclosure may exist in a variety of forms including, for example, polyclonal antibodies, monoclonal antibodies, Fv, Fab and $F(ab)_2$, as well as single chain antibodies and humanized antibodies (Harlow et al., 1999, In: Using Antibodies: A Laboratory Manual, Cold Spring Harbor Laboratory Press, NY; Harlow et al., 1989, in: Antibodies: A Laboratory Manual, Cold Spring Harbor, New York; Houston et al., 1988, Proc. Natl. Acad. Sci. USA 85:5879-5883; Bird et al., 1988, Science 242:423-426).

Antibodies expressed by B cells are sometimes referred to as the BCR (B cell receptor) or antigen receptor. The Five members included in this class of proteins are IgA, IgG, IgM, IgD, and IgE. IgA is the primary antibody that is present in body secretions, such as saliva, tears, breast milk, gastrointestinal secretions and mucus secretions of the respiratory and genitourinary tracts. IgG is the most common circulating antibody. IgM is the main immunoglobulin produced in the primary immune response in most subjects. It is the most efficient immunoglobulin in agglutination, complement fixation, and other antibody responses, and is important in defense against bacteria and viruses. IgD is the immunoglobulin that has no known antibody function, but may serve as an antigen receptor. IgE is the immunoglobulin that mediates immediate hypersensitivity by causing release of mediators from mast cells and basophils upon exposure to allergen.

The term "antibody fragment" refers to a portion of an intact antibody and typically comprises the antigenic determining variable regions of an intact antibody.

Examples of antibody fragments include, but are not limited to, Fab, Fab', F(ab')2, and Fv fragments, linear antibodies, scFv antibodies, and multispecific antibodies formed from antibody fragments.

An "antibody heavy chain", as used herein, refers to the larger of the two types of polypeptide chains present in antibody molecules in their naturally occurring conformations.

An "antibody light chain", as used herein, refers to the smaller of the two types of polypeptide chains present in antibody molecules in their naturally occurring conformations, $\kappa$ and $\lambda$ light chains refer to the two major antibody light chain isotypes.

The term "antigen" or "Ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be naturally occurring or recombinant antigens.

In the context of the present disclosure, the term "tumor antigen" refers to antigens that are common to specific hyperproliferative disorders such as cancer.

Claudins are integral membrane proteins located within the tight junctions of epithelia and endothelia. Claudins are predicted to have four transmembrane segments with two extracellular loops, and N- and C-termini located in the cytoplasm. The first extracellular loop, termed EC1 or ECL1, consists on average of 53 amino acids, and the second extracellular loop, termed EC2 or ECL2, consists of around 24 amino acids. The Claudin (CLDN) family of transmembrane proteins plays a critical role in the maintenance of epithelial and endothelial tight junctions and might also play a role in the maintenance of the cytoskeleton and in cell signalling.

Claudin 6 (CLDN6) is an oncofetal gene expressed in murine and human stem cells as well as embroid bodies committed to the epithelia cell fate (Turksen, K. et al. (2001) Dev Dyn 222, 292-300; Anderson WJ. et al. (2008) Dev Dyn 237, 504-12; Turksen K. et al. (2002) Development, 129, 1775-84; Assou S. et al. (2007) Stem Cells 25, 961-73). As a tumor-associated antigen it can be classified as a differentiation antigen due to its expression during early stage of epidermal morphogenesis where it is crucial for epidermal differentiation and barrier formation. Additionally expression was observed in epithelial tissues or neonatal normal epithelial tissue of tongue, skin, stomach and breast (Abuazza G. et al. (2006), Am J Physiol Renal Physiol 291, 1132-1141; Troy T.C. et al. (2007), Molecular Biotechnology 36, 166-74; Zhao L. et al. (2008), Am J Physiol Regul Integr Comp Physiol 294, 1856-1862). Besides that, own data also reveal low or very low expression of CLDN6 in human placenta, urinary bladder, endometrium, prostate and the peripheral nerve and frequent overexpression of CLDN6 in different cancers. CLDN6 has been demonstrated to be overexpressed in tumors, including pediatric brain tumors, gastric adenocarcinomas and germ cell tumors as well as visceral carcinomas such as ovarian carcinomas. It has also been demonstrated that overexpression of CLDN6 in gastric cancer cells results in increased invasiveness, migration and proliferation suggesting that CLDN6 is a marker for poor prognosis and may play a potential role in maintaining the malignant phenotype. In addition, it has been shown that CLDN6 functions as cancer suppressor via inhibition of cell proliferation and induction of apoptosis in breast cancer cell lines.

CLDN6 has been found to be expressed, for example, in ovarian cancer, lung cancer, gastric cancer, breast cancer, hepatic cancer, pancreatic cancer, skin cancer, melanomas, head neck cancer, sarcomas, bile duct cancer, renal cell cancer, and urinary bladder cancer. CLDN6 is a particularly preferred target for the prevention and/or treatment of ovarian cancer, in particular ovarian adenocarcinoma and ovarian teratocarcinoma, lung cancer, including small cell lung cancer (SCLC) and non-small cell lung cancer (NSCLC), in particular squamous cell lung carcinoma and adenocarcinoma, gastric cancer, breast cancer, hepatic cancer, pancreatic cancer, skin cancer, in particular basal cell carcinoma and squamous cell carcinoma, malignant melanoma, head and neck cancer, in particular malignant pleomorphic adenoma, sarcoma, in particular synovial sarcoma and carcinosarcoma, bile duct cancer, cancer of the urinary bladder, in particular transitional cell carcinoma and papillary carcinoma, kidney cancer, in particular renal cell carcinoma including clear cell renal cell carcinoma and papillary renal cell carcinoma, colon cancer, small bowel cancer, including cancer of the ileum, in particular small bowel adenocarcinoma and adenocarcinoma of the ileum, testicular embryonal carcinoma, placental choriocarcinoma, cervical cancer, testicular cancer, in particular testicular seminoma, testicular teratoma and embryonic testicular cancer, uterine cancer, germ cell tumors such as a teratocarcinoma or an embryonal carcinoma, in particular germ cell tumors of the testis, and the metastatic forms thereof. In one embodiment, the cancer expressing CLDN6 is selected from the group consisting of ovarian cancer, lung cancer, metastatic ovarian cancer and metastatic lung cancer. Preferably, the ovarian cancer is a carcinoma or an adenocarcinoma. Preferably, the lung cancer is a carcinoma or an adenocarcinoma, and preferably is bronchiolar cancer such as a bronchiolar carcinoma or bronchiolar adenocarcinoma.

The term "CLDN6" preferably relates to human CLDN6, and, in particular, to a protein comprising, preferably consisting of the amino acid sequence of SEQ ID NO: 1 or SEQ ID NO: 2 of the sequence listing or a variant of said amino acid sequence. The first extracellular loop of CLDN6 preferably comprises amino acids 28 to 80, more preferably amino acids 28 to 76 of the amino acid sequence shown in SEQ ID NO: 1 or the amino acid sequence shown in SEQ ID NO: 2. The second extracellular loop of CLDN6 preferably comprises amino acids 138 to 160, preferably amino acids 141 to 159, more preferably amino acids 145 to 157 of the amino acid sequence shown in SEQ ID NO: 1 or the amino acid sequence shown in SEQ ID NO: 2. Said first and second extracellular loops preferably form the extracellular portion of CLDN6.

The term "expressed on the cell surface" or "associated with the cell surface" means that a molecule such as CLDN6 is associated with and located at the plasma membrane of a cell, wherein at least a part of the molecule faces the extracellular space of said cell and is accessible from the outside of said cell, e.g., by antibodies located outside the cell. In this context, a part is preferably at least 4, preferably at least 8, preferably at least 12, more preferably at least 20 amino acids. The association may be direct or indirect. For example, the association may be by one or more transmembrane domains, one or more lipid anchors, or by the interaction with any other protein, lipid, saccharide, or other structure that can be found on the outer leaflet of the plasma membrane of a cell. For example, a molecule associated with the surface of a cell may be a transmembrane protein having an extracellular portion or may be a protein associated with the surface of a cell by interacting with another protein that is a transmembrane protein.

"Cell surface" or "surface of a cell" is used in accordance with its normal meaning in the art, and thus includes the outside of the cell which is accessible to binding by proteins and other molecules.

"Cell-mediated immunity", "cellular immunity", "cellular immune response", or similar terms are meant to include a cellular response directed to cells characterized by expression of an antigen, in particular characterized by presentation of an antigen with class I or class II MHC. The cellular response relates to cells called T cells or T lymphocytes which act as either "helpers" or "killers". The helper T cells (also termed CD4$^+$ T cells) play a central role by regulating the immune response and the killer cells (also termed cytotoxic T cells, cytolytic T cells, CD8$^+$ T cells or CTLs) kill diseased cells such as cancer cells, preventing the production of more diseased cells.

The term "epitope" refers to an antigenic determinant in a molecule such as an antigen, i.e., to a part in or fragment of the molecule, that is recognized, i.e. bound, by the immune system, for example, that is recognized by an antibody or CAR. For example, epitopes are the discrete, three-dimensional sites on an antigen, which are recognized by the immune system. Epitopes usually consist of chemically active surface groupings of molecules such as amino acids or sugar side chains and usually have specific three dimensional structural characteristics, as well as specific charge characteristics. Conformational and non-conformational epitopes are distinguished in that the binding to the former but not the latter is lost in the presence of denaturing solvents. Preferably an epitope is capable of eliciting an immune response against the antigen or a cell expressing the antigen. Preferably, the term relates to an immunogenic portion of an antigen. An epitope of a protein such as a tumor antigen preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 9, 10, 11, 12, 13, 14, 15, 16, 17, 18, 19, 20, 21, 22, 23, 24, or 25 amino acids in length.

"Antigen processing" refers to the degradation of an antigen into procession products, which are fragments of said antigen (e.g., the degradation of a protein into peptides) and the association of one or more of these fragments (e.g.,

via binding) with MHC molecules for presentation by cells, preferably antigen presenting cells to specific T cells.

An antigen-presenting cell (APC) is a cell that displays antigen in the context of major histocompatibility complex (MHC) on its surface. T cells may recognize this complex using their T cell receptor (TCR). Antigen-presenting cells process antigens and present them to T cells. According to the disclosure, the term "antigen-presenting cell" includes professional antigen-presenting cells and non-professional antigen-presenting cells.

Professional antigen-presenting cells are very efficient at internalizing antigen, either by phagocytosis or by receptor-mediated endocytosis, and then displaying a fragment of the antigen, bound to a class II MHC molecule, on their membrane. The T cell recognizes and interacts with the antigen-class II MHC molecule complex on the membrane of the antigen-presenting cell. An additional co-stimulatory signal is then produced by the antigen-presenting cell, leading to activation of the T cell. The expression of co-stimulatory molecules is a defining feature of professional antigen-presenting cells. The main types of professional antigen-presenting cells are dendritic cells, which have the broadest range of antigen presentation, and are probably the most important antigen-presenting cells, macrophages, B cells, and certain activated epithelial cells.

The term "macrophage" refers to a subgroup of phagocytic cells produced by the differentiation of monocytes. Macrophages which are activated by inflammation, immune cytokines or microbial products nonspecifically engulf and kill foreign pathogens within the macrophage by hydrolytic and oxidative attack resulting in degradation of the pathogen. Peptides from degraded proteins are displayed on the macrophage cell surface where they can be recognized by T cells, and they can directly interact with antibodies on the B cell surface, resulting in T and B cell activation and further stimulation of the immune response. Macrophages belong to the class of antigen presenting cells. In one embodiment, the macrophages are splenic macrophages.

The term "dendritic cell" (DC) refers to another subtype of phagocytic cells belonging to the class of antigen presenting cells. In one embodiment, dendritic cells are derived from hematopoietic bone marrow progenitor cells. These progenitor cells initially transform into immature dendritic cells. These immature cells are characterized by high phagocytic activity and low T cell activation potential. Immature dendritic cells constantly sample the surrounding environment for pathogens such as viruses and bacteria. Once they have come into contact with a presentable antigen, they become activated into mature dendritic cells and begin to migrate to the spleen or to the lymph node. Immature dendritic cells phagocytose pathogens and degrade their proteins into small pieces and upon maturation present those fragments at their cell surface using MHC molecules. Simultaneously, they upregulate cell-surface receptors that act as co-receptors in T cell activation such as CD80, CD86, and CD40 greatly enhancing their ability to activate T cells. They also upregulate CCR7, a chemotactic receptor that induces the dendritic cell to travel through the blood stream to the spleen or through the lymphatic system to a lymph node. Here they act as antigen-presenting cells and activate helper T cells and killer T cells as well as B cells by presenting them antigens, alongside non-antigen specific co-stimulatory signals. Thus, dendritic cells can actively induce a T cell- or B cell-related immune response. In one embodiment, the dendritic cells are splenic dendritic cells.

According to the disclosure, the term "CAR" (or "chimeric antigen receptor") relates to an artificial receptor comprising a single molecule or a complex of molecules which recognizes, i.e. binds to, a target structure (e.g. an antigen) on a target cell such as a cancer cell (e.g. by binding of an antigen binding domain to an antigen expressed on the surface of the target cell) and may confer specificity onto an immune effector cell such as a T cell expressing said CAR on the cell surface. Preferably, recognition of the target structure by a CAR results in activation of an immune effector cell expressing said CAR. A CAR may comprise one or more protein units said protein units comprising one or more domains as described herein. The term "CAR" does not include T cell receptors.

Adoptive cell transfer therapy with CAR-engineered T cells expressing chimeric antigen receptors is a promising anti-cancer therapeutic as CAR-modified T cells can be engineered to target virtually any tumor antigen. For example, patient's T cells may be genetically engineered (genetically modified) to express CARs specifically directed towards antigens on the patient's tumor cells, then infused back into the patient.

The term "anti-tumor" as used herein, refers to a biological effect which can be manifested by a decrease in tumor volume, a decrease in the number of tumor cells, a decrease in the number of metastases, a prevention of the occurrence of tumor in the first place, an increase in life expectancy, or amelioration of various physiological symptoms associated with the cancerous condition.

As used herein, the term "autologous" is meant to refer to any material derived from the same individual to which it is later to be re-introduced into the individual.

"Allogeneic" refers to a graft derived from a different animal of the same species,

"Xenogeneic" refers to a graft derived from an animal of a different species.

The term "syngeneic" is used to describe anything that is derived from individuals or tissues having identical genotypes, i.e., identical twins or animals of the same inbred strain, or their tissues.

The term "cancer" as used herein is defined as disease characterized by the rapid and uncontrolled growth of aberrant cells. Cancer cells can spread locally or through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

As used herein, the terms "peptide", "polypeptide", and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR™, and the like, and by synthetic means. The term "polynucleotide" as used herein is to be interpreted broadly, and includes DNA and RNA, including modified DNA and RNA.

In the present disclosure, the term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered RNAs are considered analogs of naturally-occurring RNA.

In certain embodiments of the present disclosure, the RNA is messenger RNA (mRNA) that relates to a RNA transcript which encodes a peptide or protein. As established in the art, mRNA generally contains a 5' untranslated region (5'-UTR), a peptide coding region and a 3' untranslated region (3'-UTR). In some embodiments, the RNA is produced by *in vitro* transcription or chemical synthesis. In one embodiment, the mRNA is produced by *in vitro* transcription using a DNA template where DNA refers to a nucleic acid that contains deoxyribonucleotides.

In one embodiment, RNA is *in vitro* transcribed RNA (IVT-RNA) and may be obtained by *in vitro* transcription of an appropriate DNA template. The promoter for controlling transcription can be any promoter for any RNA polymerase. A DNA template for *in vitro* transcription may be obtained by cloning of a nucleic acid, in particular cDNA, and introducing it into an appropriate vector for *in vitro* transcription. The cDNA may be obtained by reverse transcription of RNA.

In one embodiment, the RNA may have modified ribonucleotides. Examples of modified ribonucleotides include, without limitation, 5-methylcytidine, pseudouridine and/or 1-methyl-pseudouridine.

In some embodiments, the RNA according to the present disclosure comprises a 5'-cap. In one embodiment, the RNA of the present disclosure does not have uncapped 5'-triphosphates. In one embodiment, the RNA may be modified by a 5'- cap analog. The term "5'-cap" refers to a structure found on the 5'-end of an mRNA molecule and generally consists of a guanosine nucleotide connected to the mRNA via a 5' to 5' triphosphate linkage. In one embodiment, this guanosine is methylated at the 7-position. Providing an RNA with a 5'-cap or 5'-cap analog may be achieved by *in vitro* transcription, in which the 5'-cap is co-transcriptionally expressed into the RNA strand, or may be attached to RNA post-transcriptionally using capping enzymes.

In some embodiments, RNA according to the present disclosure comprises a 5'-UTR and/or a 3'-UTR. The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR). A 5'-UTR, if present, is located at the 5' end, upstream of the start codon of a protein-encoding region. A 5'-UTR is downstream of the 5'-cap (if present), e.g. directly adjacent to the 5'-cap. A 3'-UTR, if present, is located at the 3' end, downstream of the termination codon of a protein-encoding region, but the term "3'-UTR" does preferably not include the poly(A) tail. Thus, the 3'-UTR is upstream of the poly(A) sequence (if present), e.g. directly adjacent to the poly(A) sequence.

In some embodiments, the RNA according to the present disclosure comprises a 3'-poly(A) sequence. The term "poly(A) sequence" relates to a sequence of adenyl (A) residues which typically is located at the 3'-end of a RNA molecule. According to the disclosure, in one embodiment, a poly(A) sequence comprises at least about 20, at least about 40, at least about 80, or at least about 100, and up to about 500, up to about 400, up to about 300, up to about 200, or up to about 150 A nucleotides, and in particular about 120 A nucleotides.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the noncoding strand, used as the template for transcription of a gene or cDNA, can be referred to as encoding the protein or other product of that gene or cDNA.

As used herein "endogenous" refers to any material from or produced inside an organism, cell, tissue or system.

As used herein, the term "exogenous" refers to any material introduced from or produced outside an organism, cell, tissue or system.

The term "expression" as used herein is defined as the transcription and/or translation of a particular nucleotide sequence.

"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an *in vitro* expression system. Expression vectors include all those known in the art, such as cosmids, plasmids (e.g., naked or contained in liposomes) and viruses (e.g., lentiviruses, retroviruses, adenoviruses, and adeno-associated viruses) that incorporate the recombinant polynucleotide.

"Homologous" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared X 100. For example, if 6 of 10 of the positions in two sequences are matched or homologous then the two sequences are 60% homologous. Generally, a comparison is made when two sequences are aligned to give maximum homology. Homologous sequences exhibit according to the disclosure at least 40%, in particular at least 50%, at least 60%, at least 70%, at least 80%, at least 90% and preferably at least 95%, at least 98 or at least 99% identity of the amino acid or nucleotide residues.

"Fragment", with reference to an amino acid sequence (peptide or protein), relates to a part of an amino acid sequence, i.e. a sequence which represents the amino acid sequence shortened at the N-terminus and/or C-terminus. A fragment shortened at the C-terminus (N-terminal fragment) is obtainable e.g. by translation of a truncated open reading frame that lacks the 3'-end of the open reading frame. A fragment shortened at the N-terminus (C-terminal fragment) is obtainable e.g. by translation of a truncated open reading frame that lacks the 5'-end of the open reading frame, as long as the truncated open reading frame comprises a start codon that serves to initiate translation. A fragment of an amino acid sequence comprises e.g. at least 50 %, at least 60 %, at least 70 %, at least 80%, at least 90% of the amino acid residues from an amino acid sequence. A fragment of an amino acid sequence preferably comprises at least 6, in particular at least 8, at least 12, at least 15, at least 20, at least 30, at least 50, or at least 100 consecutive amino acids from an amino acid sequence.

By "variant" or "variant protein" or "variant polypeptide" herein is meant a protein that differs from a parent protein by virtue of at least one amino acid modification. The parent polypeptide may be a naturally occurring or wild type (WT) polypeptide, or may be a modified version of a wild type polypeptide. Preferably, the variant polypeptide has at least one amino acid modification compared to the parent polypeptide, e.g. from 1 to about 20 amino acid modifications, and preferably from 1 to about 10 or from 1 to about 5 amino acid modifications compared to the parent.

By "parent polypeptide", "parent protein", "precursor polypeptide", or "precursor protein" as used herein is meant an unmodified polypeptide that is subsequently modified to generate a variant. A parent polypeptide may be a wild type polypeptide, or a variant or engineered version of a wild type polypeptide.

By "wild type" or "WT" or "native" herein is meant an amino acid sequence that is found in nature, including allelic variations. A wild type protein or polypeptide has an amino acid sequence that has not been intentionally modified.

For the purposes of the present disclosure, "variants" of an amino acid sequence (peptide, protein or polypeptide) comprise amino acid insertion variants, amino acid addition variants, amino acid deletion variants and/or amino acid substitution variants. The term "variant" includes all mutants, splice variants, posttranslationally modified variants, conformations, isoforms, allelic variants, species variants, and species homologs, in particular those which are naturally occurring.

Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible. Amino acid addition variants comprise amino- and/or carboxy-terminal fusions of one or more amino acids, such as 1, 2, 3, 5, 10, 20, 30, 50, or more amino acids. Amino acid deletion variants are characterized by the removal of one or more amino acids from the sequence, such as by removal of 1, 2, 3, 5, 10, 20, 30, 50, or more amino acids. The deletions may be in any position of the protein. Amino acid deletion variants that comprise the deletion at the N-terminal and/or C-terminal end of the protein are also called N-terminal and/or C-terminal truncation variants. Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in peptide and protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids. In one embodiment, conservative amino acid substitutions include substitutions within the following groups:

glycine, alanine;
valine, isoleucine, leucine;
aspartic acid, glutamic acid;
asparagine, glutamine;
serine, threonine;
lysine, arginine; and
phenylalanine, tyrosine.

Preferably the degree of similarity, preferably identity between a given amino acid sequence and an amino acid sequence which is a variant of said given amino acid sequence will be at least about 60%, 65%, 70%, 80%, 81%, 82%, 83%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.
"Sequence similarity" indicates the percentage of amino acids that either are identical or that represent conservative amino acid substitutions. "Sequence identity" between two amino acid sequences indicates the percentage of amino acids that are identical between the sequences.
The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).
The percentage identity is calculated by determining the number of identical positions between the two sequences being compared, dividing this number by the number of positions compared and multiplying the result obtained by 100 so as to obtain the percentage identity between these two sequences.
The term "functional variant", as used herein, refers to a variant molecule or sequence that comprises an amino acid sequence that is altered by one or more amino acids compared to the amino acid sequence of the parent molecule or sequence and that is still capable of fulfilling one or more of the functions of the parent molecule or sequence, e.g., binding to a target molecule or contributing to binding to a target molecule. In one embodiment, a functional variant either alone or in combination with other elements competes for binding to a target molecule with the parent molecule or sequence. In other words, the modifications in the amino acid sequence of the parent molecule or sequence do not significantly affect or alter the binding characteristics of the molecule or sequence. In different embodiments, binding of the functional variant may be reduced but still significantly present, e.g., binding of the functional variant may be at least 50%, at least 60%, at least 70%, at least 80%, or at least 90% of the parent molecule or sequence. However, in other embodiments, binding of the functional variant may be enhanced compared to the parent molecule or sequence.

An amino acid sequence (peptide, protein or polypeptide) "derived from" a designated amino acid sequence (peptide, protein or polypeptide) refers to the origin of the first amino acid sequence. Preferably, the amino acid sequence which is derived from a particular amino acid sequence has an amino acid sequence that is identical, essentially identical or homologous to that particular sequence or a fragment thereof. Amino acid sequences derived from a particular amino acid sequence may be variants of that particular sequence or a fragment thereof.

As used herein, an "instructional material" or "instructions" includes a publication, a recording, a diagram, or any other medium of expression which can be used to communicate the usefulness of the compositions and methods of the disclosure. The instructional material of the kit of the disclosure may, for example, be affixed to a container which contains the compositions of the disclosure or be shipped together with a container which contains the compositions. Alternatively, the instructional material may be shipped separately from the container with the intention that the instructional material and the compositions be used cooperatively by the recipient.

"Isolated" means altered or removed from the natural state. For example, a nucleic acid or a peptide naturally present in a living animal is not "isolated", but the same nucleic acid or peptide partially or completely separated from the coexisting materials of its natural state is "isolated". An isolated nucleic acid or protein can exist in substantially purified form, or can exist in a non-native environment such as, for example, a host cell.

In the context of the present disclosure, the following abbreviations for the commonly occurring nucleic acid bases are used. "A" refers to adenosine, "C" refers to cytosine, "G" refers to guanosine, "T" refers to thymidine, and "U" refers to uridine.

A "lentivirus" as used herein refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses. Vectors derived from lentiviruses offer the means to achieve significant levels of gene transfer *in vivo*.

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

The term "overexpressed" tumor antigen or "overexpression" of the tumor antigen is intended to indicate an abnormal level of expression of the tumor antigen in a cell from a disease area like a solid tumor within a specific tissue or organ of the patient relative to the level of expression in a normal cell from that tissue or organ.

The term "promoter" as used herein is defined as a DNA sequence recognized by the synthetic machinery of the cell, or introduced synthetic machinery, required to initiate the specific transcription of a polynucleotide sequence.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only when an inducer which corresponds to the promoter is present in the cell.

A "tissue-specific" promoter is a nucleotide sequence which, when operably linked with a polynucleotide encodes or specified by a gene, causes the gene product to be produced in a cell substantially only if the cell is a cell of the tissue type corresponding to the promoter.

By the term "specifically binds", as used herein, is meant a molecule such as an antibody or CAR which recognizes a specific antigen, but does not substantially recognize or bind other molecules in a sample or in a subject. For example, an antibody that specifically binds to an antigen from one species may also bind to that antigen from one or more other species. But, such cross-species reactivity does not itself alter the classification of an antibody as specific. In another example, an antibody that specifically binds to an antigen may also bind to different allelic forms of the antigen. However, such cross reactivity does not itself alter the classification of an antibody as specific. In some instances, the terms "specific binding" or "specifically binding", can be used in reference to the interaction of an antibody, a protein, or a peptide with a second chemical species, to mean that the interaction is dependent upon the presence of a particular structure (e.g., an antigenic determinant or epitope) on the chemical species; for example, an antibody recognizes and binds to a specific protein structure rather than to proteins generally. If an antibody is specific for epitope "A", the presence of a molecule containing epitope A (or free, unlabeled A), in a reaction containing labeled "A" and the antibody, will reduce the amount of labeled A bound to the antibody.

The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The phrase "under transcriptional control" or "operatively linked" as used herein means that the promoter is in the correct location and orientation in relation to a polynucleotide to control the initiation of transcription by RNA polymerase and expression of the polynucleotide.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like.

Description

The present disclosure provides compositions and methods for treating cancer among other diseases. The cancer may be a solid tumor, a primary or a metastasizing tumor. In one embodiment, the cancer is a CLDN6 expressing cancer. In one embodiment, the cancer is selected from the group consisting of ovarian cancer, in particular ovarian adenocarcinoma and ovarian teratocarcinoma, lung cancer, including small cell lung cancer (SCLC) and non-small cell lung cancer (NSCLC), in particular squamous cell lung carcinoma and adenocarcinoma, gastric cancer, breast cancer, hepatic cancer, pancreatic cancer, skin cancer, in particular basal cell carcinoma and squamous cell carcinoma, malignant melanoma, head and neck cancer, in particular malignant pleomorphic adenoma, sarcoma, in particular synovial sarcoma and carcinosarcoma, bile duct cancer, cancer of the urinary bladder, in particular transitional cell carcinoma and papillary carcinoma, kidney cancer, in particular renal cell carcinoma including clear cell renal cell carcinoma and papillary renal cell carcinoma, colon cancer, small bowel cancer, including cancer of the ileum, in particular small bowel adenocarcinoma and adenocarcinoma of the ileum, testicular embryonal carcinoma, placental choriocarcinoma, cervical cancer, testicular cancer, in particular testicular seminoma, testicular teratoma and embryonic testicular cancer, uterine cancer, germ cell tumors such as a teratocarcinoma or an embryonal carcinoma, in particular germ cell tumors of the testis, and the metastatic forms thereof. In one embodiment, the cancer expressing CLDN6 is selected from the group consisting of ovarian cancer, lung cancer, metastatic ovarian cancer and metastatic lung cancer. Preferably, the ovarian cancer is a carcinoma or an adenocarcinoma. Preferably, the lung cancer is a carcinoma or an adenocarcinoma, and preferably is bronchiolar cancer such as a bronchiolar carcinoma or bronchiolar adenocarcinoma.

The present disclosure provides a chimeric antigen receptor (CAR) comprising an extracellular and intracellular domain. The extracellular domain comprises a target-specific binding element otherwise referred to as an antigen binding moiety or domain. The intracellular domain or otherwise the cytoplasmic domain comprises a 4-1BB costimulatory signaling region and a CD3-zeta chain portion. The 4-1BB costimulatory signaling region refers to a portion of the CAR comprising an intracellular domain of the costimulatory molecule 4-1BB. Costimulatory molecules are cell surface molecules other than antigens receptors or their ligands that are required for an efficient response of lymphocytes to antigen.

Between the extracellular domain and the transmembrane domain of the CAR, or between the cytoplasmic domain and the transmembrane domain of the CAR, there may be incorporated a spacer domain or region. In embodiments, a spacer domain provides for flexibility of the antigen binding domain. As used herein, the term "spacer domain" generally means any oligo- or polypeptide that functions to link the transmembrane domain to, either the extracellular domain or, the cytoplasmic domain in the polypeptide chain. A spacer domain may comprise up to 300 amino acids, preferably 10 to 100 amino acids and most preferably 25 to 50 amino acids. In embodiments, a spacer domain has about 10 to 300 amino acids, about 10 to 200 amino acids, about 10 to 175 amino acids, about 10 to 150 amino acids, about 10 to 125 amino acids, about 10 to 100 amino acids, about 10 to 75 amino acids, about 10 to 50 amino acids, about 10 to 40 amino acids, about 10 to 30 amino acids, about 10 to 20 amino acids, or about 10 to 15 amino acids, and including any integer between the endpoints of any of the listed ranges. In some embodiments, the spacer domain is derived from a hinge region of an immunoglobulin like molecule. In embodiments, a spacer domain comprises all or a portion of the hinge region from a human IgG1, human IgG2, a human IgG3, or a human IgG4, and may contain one or more amino acid substitutions. In some embodiments, the spacer domain is derived from the hinge region sequence of CD8α.

In one embodiment, the disclosure provides a cell (e.g., T cell) engineered to express a CAR wherein the CAR-engineered cell exhibits an antitumor property. The CAR of the disclosure when expressed in a cell is able to redirect antigen recognition based on the antigen binding specificity of the CAR. In one embodiment, CLDN6 is expressed on cells of the cancer types disclosed herein. The CAR-engineered cell when bound to its cognate antigen, affects a tumor cell so that the tumor cell fails to grow, is prompted to die, or otherwise is affected so that the tumor burden in a patient is diminished or eliminated.

According to the disclosure, a CLDN6 antigen binding moiety is fused via a hinge domain derived from CD8α with intracellular domains comprising a combination of a 4-1BB (CD137) signaling domain and a CD3-zeta signaling domain. Inclusion of the 4-1BB (CD137) signaling domain significantly increases anti-tumor activity and *in vivo* persistence of CAR T cells compared to an otherwise identical CAR T cell not engineered to express 4-1BB (CD137) (Milone M.C. et al., (2009) Molecular Therapy 17 (8), 1453-1464).

Antigen binding moiety

The CAR of the disclosure comprises a target-specific binding element otherwise referred to as an antigen binding moiety or antigen binding domain that is generally part of the extracellular domain of the CAR. The antigen binding domain recognizes a ligand that acts as a cell surface marker on target cells associated with a particular disease state. Specifically, the CAR of the disclosure targets the tumor antigen CLDN6 on a tumor cell.

In one embodiment, the CLDN6 binding domain in the CAR of the disclosure binds specifically to CLDN6. In one embodiment, the CLDN6 to which the CLDN6 binding domain in the CAR of the disclosure binds is expressed in a cancer cell. In one embodiment, the CLDN6 is expressed on the surface of a cancer cell. In one embodiment, the CLDN6 binding domain binds to an extracellular domain or to an epitope in an extracellular domain of CLDN6. In one embodiment, the CLDN6 binding domain binds to native epitopes of CLDN6 present on the surface of living cells. In one embodiment, the CLDN6 binding domain binds to the first extracellular loop of CLDN6, preferably amino acid positions 28 to 76 of CLDN6, or the second extracellular loop of CLDN6, preferably amino acid positions 141 to 159 of CLDN6. In particular embodiments, the CLDN6 binding domain binds to an epitope on CLDN6 which is not present on CLDN9. Preferably, the CLDN6 binding domain binds to an epitope on CLDN6 which is not present on CLDN4 and/or CLDN3. Most preferably, the CLDN6 binding domain binds to an epitope on CLDN6 which is not present on a CLDN protein other than CLDN6. The CLDN6 binding domain preferably binds to CLDN6 but not to CLDN9 and preferably does not bind to CLDN4 and/or CLDN3. Preferably, the CLDN6 binding domain is specific for CLDN6. Preferably, the CLDN6 binding domain binds to CLDN6 expressed on the cell surface.

In one embodiment of the disclosure, a CLDN6 antigen binding domain comprises a variable region of a heavy chain of an immunoglobulin (VH) with a specificity for CLDN6 and a variable region of a light chain of an immunoglobulin (VL) with a specificity for CLDN6. In one embodiment, said heavy chain variable region (VH) and the corresponding light chain variable region (VL) are connected via a peptide linker, preferably a peptide linker comprising the amino acid sequence (GGGGS)3.

In one embodiment, a binding domain for CLDN6 comprises a heavy chain variable region (VH) comprising an amino acid sequence selected from the group consisting of SEQ ID NOs: 3, 5, 7 and 9 or a functional variant thereof.

In one embodiment, a binding domain for CLDN6 comprises a light chain variable region (VL) comprising an amino acid sequence selected from the group consisting of SEQ ID NOs: 4, 6, 8, 10, 23 and 24 or a functional variant thereof.

In one embodiment, a binding domain for CLDN6 comprises:

(i) a heavy chain variable region (VH) comprising an amino acid sequence of SEQ ID NO: x or a functional variant thereof, and
(ii) a light chain variable region (VL) comprising an amino acid sequence of SEQ ID NO: x+1 or a functional variant thereof;

wherein x selected from 3, 5, 7 and 9.

In one embodiment, a binding domain for CLDN6 comprises:

(i) a heavy chain variable region (VH) comprising an amino acid sequence selected from the group consisting of SEQ ID NOs: 3, 5, 7 and 9 or a functional variant thereof, and

(ii) a light chain variable region (VL) comprising an amino acid sequence selected from the group consisting of SEQ ID NOs: 4, 6, 8, 10, 23 and 24 or a functional variant thereof.

In one embodiment, a binding domain for CLDN6 comprises:

(i) a heavy chain variable region (VH) comprising the amino acid sequence of SEQ ID NO: 5 or a functional variant thereof, and

(ii) a light chain variable region (VL) comprising the amino acid sequence of SEQ ID NO: 4 or a functional variant thereof.

In one embodiment, a binding domain for CLDN6 comprises:

(i) a heavy chain variable region (VH) comprising the amino acid sequence of SEQ ID NO: 5 or a functional variant thereof, and

(ii) a light chain variable region (VL) comprising the amino acid sequence of SEQ ID NO: 23 or a functional variant thereof.

In one embodiment, a binding domain for CLDN6 comprises:

(i) a heavy chain variable region (VH) comprising the amino acid sequence of SEQ ID NO: 5 or a functional variant thereof, and

(ii) a light chain variable region (VL) comprising the amino acid sequence of SEQ ID NO: 24 or a functional variant thereof.

In certain preferred embodiments, a binding domain for CLDN6 comprises a combination of heavy chain variable region (VH) and light chain variable region (VL) selected from the following possibilities (i) to (xi):

(i) the VH comprises an amino acid sequence represented by SEQ ID NO: 3 or a functional variant thereof, or a fragment of the amino acid sequence or functional variant, and the VL comprises an amino acid sequence represented by SEQ ID NO: 4 or a functional variant thereof, or a fragment of the amino acid sequence or functional variant,
(ii) the VH comprises an amino acid sequence represented by SEQ ID NO: 5 or a functional variant thereof, or a fragment of the amino acid sequence or functional variant, and the VL comprises an amino acid sequence represented by SEQ ID NO: 6 or a functional variant thereof, or a fragment of the amino acid sequence or functional variant,
(iii) the VH comprises an amino acid sequence represented by SEQ ID NO: 7 or a functional variant thereof, or a fragment of the amino acid sequence or functional variant, and the VL comprises an amino acid sequence represented by SEQ ID NO: 8 or a functional variant thereof, or a fragment of the amino acid sequence or functional variant,
(iv) the VH comprises an amino acid sequence represented by SEQ ID NO: 9 or a functional variant thereof, or a fragment of the amino acid sequence or functional variant, and the VL comprises an amino acid sequence represented by SEQ ID NO: 10 or a functional variant thereof, or a fragment of the amino acid sequence or functional variant,
(v) the VH comprises an amino acid sequence represented by SEQ ID NO: 5 or a functional variant thereof, or a fragment of the amino acid sequence or functional variant, and the VL comprises an amino acid sequence represented by SEQ ID NO: 4 or a functional variant thereof, or a fragment of the amino acid sequence or functional variant,
(vi) the VH comprises an amino acid sequence represented by SEQ ID NO: 5 or a functional variant thereof, or a fragment of the amino acid sequence or functional variant, and the VL comprises an amino acid sequence represented by SEQ ID NO: 23 or a functional variant thereof, or a fragment of the amino acid sequence or functional variant,
(vii) the VH comprises an amino acid sequence represented by SEQ ID NO: 5 or a functional variant thereof, or a fragment of the amino acid sequence or functional variant, and the VL comprises an amino acid sequence represented by SEQ ID NO: 24 or a functional variant thereof, or a fragment of the amino acid sequence or functional variant.

In a particularly preferred embodiment, a binding domain for CLDN6 comprises the following combination of heavy chain variable region (VH) and light chain variable region (VL):
the VH comprises an amino acid sequence represented by SEQ ID NO: 5 or a functional variant thereof, or a fragment of the amino acid sequence or functional variant, and the VL comprises an amino acid sequence represented by SEQ ID NO: 24 or a functional variant thereof, or a fragment of the amino acid sequence or functional variant.
The term "fragment" refers, in particular, to one or more of the complementarity-determining regions (CDRs), preferably at least the CDR3 variable region, of the heavy chain variable region (VH) and/or of the light chain variable region (VL). In one embodiment said one or more of the complementarity-determining regions (CDRs) are selected from a set of complementarity-determining regions CDR1, CDR2 and CDR3. In a particularly preferred embodiment, the term "fragment" refers to the complementarity-determining regions CDR1, CDR2 and CDR3 of the heavy chain variable region (VH) and/or of the light chain variable region (VL).

In one embodiment a binding domain for CLDN6 comprising one or more CDRs, a set of CDRs or a combination of sets of CDRs as described herein comprises said CDRs together with their intervening framework regions. Preferably, the portion will also include at least about 50% of either or both of the first and fourth framework regions, the 50% being the C-terminal 50% of the first framework region and the N-terminal 50% of the fourth framework region. Construction of binding domains made by recombinant DNA techniques may result in the introduction of residues N- or C-terminal to the variable regions encoded by linkers introduced to facilitate cloning or other manipulation steps, including the introduction of linkers to join variable regions or join variable regions to further protein sequences including sequences as described herein.

In one embodiment a binding domain comprising one or more CDRs, a set of CDRs or a combination of sets of CDRs as described herein comprises said CDRs in a human antibody framework.

In one embodiment a binding domain for CLDN6 comprises a heavy chain variable region (VH) comprising at least one, preferably two, more preferably all three of the CDR sequences of a heavy chain variable region (VH) described herein, e.g., of a heavy chain variable region (VH) comprising an amino acid sequence selected from the group consisting of SEQ ID NOs: 3, 5, 7 and 9 or a functional variant thereof.

In one embodiment a binding domain for CLDN6 comprises a light chain variable region (VL) comprising at least one, preferably two, more preferably all three of the CDR sequences of a light chain variable region (VL) described herein, e.g., of a light chain variable region (VL) comprising an amino acid sequence selected from the group consisting of SEQ ID NOs: 4, 6, 8, 10, 23 and 24 or a functional variant thereof.

In one embodiment a binding domain for CLDN6 comprises:

(i) a heavy chain variable region (VH) comprising at least one, preferably two, more preferably all three of the CDR sequences of a heavy chain variable region (VH) of a combination of heavy chain variable region (VH) and light chain variable region (VL) described herein, e.g., a combination wherein the VH comprises an amino acid sequence represented by SEQ ID NO: 5 or a functional variant thereof, and the VL comprises an amino acid sequence represented by SEQ ID NO: 6 or a functional variant thereof, and

(ii) a light chain variable region (VL) comprising at least one, preferably two, more preferably all three of the CDR sequences of a light chain variable region (VL) of the combination of heavy chain variable region (VH) and light chain variable region (VL) described herein.

In one embodiment, a binding domain for CLDN6 comprises:

(i) a heavy chain variable region (VH) comprising at least one, preferably two, more preferably all three of the CDR sequences of a heavy chain variable region (VH) of SEQ ID NO: x, or a functional variant thereof, and
(ii) a light chain variable region (VL) comprising at least one, preferably two, more preferably all three of the CDR sequences of a light chain variable region (VL) of SEQ ID NO: x+1, or a functional variant thereof;

wherein x selected from 3, 5, 7 and 9.

In one embodiment, a binding domain for CLDN6 comprises:

(i) a heavy chain variable region (VH) comprising at least one, preferably two, more preferably all three of the CDR sequences of a heavy chain variable region (VH) of SEQ ID NO: 5, or a functional variant thereof, and
(ii) a light chain variable region (VL) comprising at least one, preferably two, more preferably all three of the CDR sequences of a light chain variable region (VL) of SEQ ID NO: 6, or a functional variant thereof.

In one embodiment, a binding domain for CLDN6 comprises:

(i) a heavy chain variable region (VH) comprising at least one, preferably two, more preferably all three of the CDR sequences of a heavy chain variable region (VH) of SEQ ID NO: 5, or a functional variant thereof, and
(ii) a light chain variable region (VL) comprising at least one, preferably two, more preferably all three of the CDR sequences of a light chain variable region (VL) of SEQ ID NO: 4, or a functional variant thereof.

The term "at least one, preferably two, more preferably all three of the CDR sequences" preferably relates to at least the CDR3 sequence, optionally in combination with the CDR1 sequence and/or the CDR2 sequence.

CDR1 of a heavy chain variable region (VH) selected from the group consisting of SEQ ID NOs: 3, 5, 7 and 9 preferably includes amino acids 26 to 33 of the sequences shown in SEQ ID NOs: 3, 5, 7 and 9, respectively. CDR2 of a heavy chain variable region (VH) selected from the group consisting of SEQ ID NOs: 3, 5, 7 and 9 preferably includes amino acids 51 to 58 of the sequences shown in SEQ ID NOs: 3, 5, 7 and 9, respectively. CDR3 of a heavy chain variable region (VH) selected from the group consisting of SEQ ID NOs: 3, 5, 7 and 9 preferably includes amino acids 97 to 106 of the sequences shown in SEQ ID NOs: 3, 5, 7 and 9, respectively.

CDR1 of a light chain variable region (VL) selected from the group consisting of SEQ ID NOs: 4, 6, 8, 10, 23 and 24 preferably includes amino acids 27 to 31 of the sequences shown in SEQ ID NOs: 4, 6, 8, 10, 23 and 24, respectively. CDR2 of a light chain variable region (VL) selected from the group consisting of SEQ ID NOs: 4, 6, 8, 10, 23 and 24 preferably includes amino acids 49 to 51 of the sequences shown in SEQ ID NOs: 4, 6, 8, 10, 23 and 24, respectively. CDR3 of a light chain variable region (VL) selected from the group consisting of SEQ ID NOs: 4, 6, 8, 10, 23 and 24 preferably includes amino acids 88 to 97 of the sequences shown in SEQ ID NOs: 4, 6, 8, 10, 23 and 24, respectively.

In one embodiment, a binding domain for CLDN6 comprises a heavy chain variable region (VH) comprising the CDR3 sequence Xaa1 Gly Xaa2 Val Xaa3, wherein Xaa1 is any amino acid, preferably an aromatic amino acid, more preferably Phe or Tyr, most preferably Tyr, Xaa2 is any amino acid, preferably an aromatic amino acid, more preferably Phe or Tyr, most preferably Tyr, and Xaa3 is any amino acid, preferably Leu or Phe, more preferably Leu. In one embodiment, a binding domain for CLDN6 comprises a heavy chain variable region (VH) comprising the CDR3 sequence Asp Xaa1 Gly Xaa2 Val Xaa3 or Xaa1 Gly Xaa2 Val Xaa3 Asp, wherein Xaal, Xaa2 and Xaa3 are as defined above. In one embodiment, a binding domain for CLDN6 comprises a heavy chain variable region (VH) comprising the CDR3 sequence Asp Xaa1 Gly Xaa2 Val Xaa3 Asp, wherein Xaal, Xaa2 and Xaa3 are as defined above. In one embodiment, a binding domain for CLDN6 comprises a heavy chain variable region (VH) comprising the CDR3 sequence Ala Arg Asp Xaa1 Gly Xaa2 Val Xaa3 Asp Tyr, wherein Xaal, Xaa2 and Xaa3 are as defined above. In one embodiment, a binding domain for CLDN6 according to the foregoing embodiments comprises a heavy chain variable region (VH) comprising the CDR1 sequence according to SEQ ID NO: 16 or a functional variant thereof and/or the CDR2 sequence according to SEQ ID NO: 17 or a functional variant thereof.

In one embodiment, a binding domain for CLDN6 comprises a light chain variable region (VL) comprising the CDR3 sequence Arg Xaa1 Xaa2 Xaa3 Pro, wherein Xaa1 is any amino acid, preferably Ser or Asn, most preferably Ser, Xaa2 is any amino acid, preferably Tyr, Ser, Ile, Asn or Thr, more preferably Tyr, Ser, or Asn, most preferably Asn, and Xaa3 is any amino acid, preferably Ser or Tyr, more preferably Tyr. In one embodiment, a binding domain for CLDN6 comprises a light chain variable region (VL) comprising the CDR3 sequence Gln Arg Xaa1 Xaa2 Xaa3 Pro Pro, wherein Xaal, Xaa2 and Xaa3 are as defined above. In one embodiment, a binding domain for CLDN6 comprises a light chain variable region (VL) comprising the CDR3 sequence Gln Gln Arg Xaa1 Xaa2 Xaa3 Pro Pro Trp Thr, wherein Xaal, Xaa2 and Xaa3 are as defined above. In one embodiment, a binding domain for CLDN6 according to the foregoing embodiments comprises a light chain variable region (VL) comprising the CDR1 sequence according to SEQ ID NO: 21 or a functional variant thereof and/or the CDR2 sequence according to SEQ ID NO: 22 or a functional variant thereof.

In one embodiment, a binding domain for CLDN6 comprises:

(i) a heavy chain variable region (VH) comprising a CDR3 sequence selected from the group consisting of Xaa1 Gly Xaa2 Val Xaa3, Asp Xaa1 Gly Xaa2 Val Xaa3, Xaa1 Gly Xaa2 Val Xaa3 Asp, Asp Xaa1 Gly Xaa2 Val Xaa3 Asp, and Ala Arg Asp Xaa1 Gly Xaa2 Val Xaa3 Asp Tyr, wherein Xaa1 is any amino acid, preferably an aromatic amino acid, more preferably Phe or Tyr, most preferably Tyr, Xaa2 is any amino acid, preferably an aromatic amino acid, more preferably Phe or Tyr, most preferably Tyr, and Xaa3 is any amino acid, preferably Leu or Phe, more preferably Leu, and
(ii) a light chain variable region (VL) comprising a CDR3 sequence selected from the group consisting of Arg Xaa1 Xaa2 Xaa3 Pro, Gln Arg Xaa1 Xaa2 Xaa3 Pro Pro, Gln Gln Arg Xaa1 Xaa2 Xaa3 Pro Pro Trp Thr, wherein Xaa1 is any amino acid, preferably Ser or Asn, most preferably Ser, Xaa2 is any amino acid, preferably Tyr, Ser, Ile, Asn or Thr, more preferably Tyr, Ser, or Asn, most preferably Asn, and Xaa3 is any amino acid, preferably Ser or Tyr, more preferably Tyr.

In one embodiment, a binding domain for CLDN6 according to the foregoing embodiments comprises (i) a heavy chain variable region (VH) comprising the CDR1 sequence according to SEQ ID NO: 16 or a functional variant thereof and/or the CDR2 sequence according to SEQ ID NO: 17 or a functional variant thereof and/or (ii) a light chain variable region (VL) comprising the CDR1 sequence according to SEQ ID NO: 21 or a functional variant thereof and/or the CDR2 sequence according to SEQ ID NO: 22 or a functional variant thereof.

In one embodiment a binding domain for CLDN6 competes for CLDN6 binding with a binding domain for CLDN6 described above and/or has the specificity for CLDN6 of a binding domain for CLDN6 described above. In these and other embodiment, a binding domain for CLDN6 may be highly homologous to a binding domain for CLDN6 described above. It is contemplated that a preferred binding domain for CLDN6 has CDR regions either identical or highly homologous to the CDR regions of a binding domain for CLDN6 described above. By "highly homologous" it is contemplated that from 1 to 5, preferably from 1 to 4, such as 1 to 3 or 1 or 2 substitutions may be made in each CDR region.

The term "compete" refers to the competition between two binding molecules for binding to a target antigen. If two binding molecules do not block each other for binding to a target antigen, such binding molecules are non-competing and this is an indication that said binding molecules do not bind to the same part, i.e. epitope, of the target antigen. It is well known to a person skilled in the art how to test for competition of binding molecules such as antibodies for binding to a target antigen. An example of such a method is a so-called cross-competition assay, which may e.g. be performed as an ELISA or by flow-cytometry. For example an ELISA-based assay may be performed by coating ELISA plate wells with one of the antibodies; adding the competing antibody and His-tagged antigen/target and detecting whether the added antibody inhibited binding of the His-tagged antigen to the coated antibody, e.g., by adding biotinylated anti-His antibody, followed by Streptavidin-poly-HRP, and further developing the reaction with ABTS and measuring the absorbance at 405 nm. For example a flow-cytometry assay may be performed by incubating cells expressing the antigen/target with an excess of unlabeled antibody, incubating the cells with a sub-optimal concentration of biotin-labelled antibody, followed by incubation with fluorescently labeled streptavidin and analyzing by flow cytometry.

Two binding molecules have the "same specificity" if they bind to the same antigen and to the same epitope. Whether a molecule to be tested recognizes the same epitope as a certain binding molecule, i.e., the binding molecules bind to the same epitope, can be tested by different methods known to the skilled person, e.g., based on the competition of the binding molecules such as antibodies for the same epitope. The competition between the binding molecules can be detected by a cross-blocking assay. For example, a competitive ELISA assay may be used as a cross-blocking assay. For example, target antigen may be coated on the wells of a microtiter plate and antigen binding antibody and candidate competing test antibody may be added. The amount of the antigen binding antibody bound to the antigen in the well indirectly correlates with the binding ability of the candidate competing test antibody that competes therewith for binding to the same epitope. Specifically, the larger the affinity of the candidate competing test antibody is for the same epitope, the smaller the amount of the antigen binding antibody bound to the antigen-coated well. The amount of the antigen binding antibody bound to the well can be measured by labeling the antibody with detectable or measurable labeling substances.

Preferably, the antigen binding moiety portion in the CAR of the disclosure is anti-CLDN6 scFV, wherein the anti-CLDN6 scFV preferably comprises the sequence set forth in SEQ ID NO: 35 or a functional variant thereof.

Transmembrane domain

The CAR of the disclosure is designed to comprise a transmembrane domain that is fused to the extracellular domain of the CAR. In one embodiment, the transmembrane domain is not naturally associated with one of the domains in the CAR. In one embodiment, the transmembrane domain is naturally associated with one of the domains in the CAR. In one embodiment, the transmembrane domain is modified by amino acid substitution to avoid binding of such domains to the transmembrane domains of the same or different surface membrane proteins to minimize interactions with other members of the receptor complex. The transmembrane domain may be derived either from a natural or from a synthetic source. Where the source is natural, the domain may be derived from any membrane-bound or transmembrane protein. Transmembrane regions of particular use in this disclosure may be derived from (i.e. comprise at least the transmembrane region(s) of) the alpha, beta or zeta chain of the T cell receptor, CD28, CD3 epsilon, CD45, CD4, CDS, CD8, CD9, CD16, CD22, CD33, CD37, CD64, CD80, CD86, CD134, CD137, CD154. Alternatively the transmembrane domain may be synthetic, in which case it will comprise predominantly hydrophobic residues such as leucine and valine. Preferably a triplet of phenylalanine, tryptophan and valine will be found at each end of a synthetic transmembrane domain.

Preferably, the transmembrane domain in the CAR of the disclosure is the CD8$\alpha$ transmembrane domain. In one embodiment, the CD8$\alpha$ transmembrane domain comprises the amino acid sequence of SEQ ID NO: 28 or a functional variant thereof.

In some instances, the CAR of the disclosure comprises the CD8$\alpha$ hinge domain which forms the linkage between the transmembrane domain and the extracellular domain. In one embodiment, the CD8$\alpha$ hinge domain comprises the amino acid sequence of SEQ ID NO: 27 or a functional variant thereof.

Cytoplasmic domain

The cytoplasmic domain or otherwise the intracellular signaling domain of the CAR of the disclosure is responsible for activation of at least one of the normal effector functions of the immune cell in which the CAR has been placed in. The term "effector function" refers to a specialized function of a cell. Effector function of a T cell, for example, may be cytolytic activity or helper activity including the secretion of cytokines. Thus the term "intracellular signaling domain" refers to the portion of a protein which transduces the effector function signal and directs the cell to perform a specialized function. While usually the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

It is known that signals generated through the TCR alone are insufficient for full activation of the T cell and that a secondary or co-stimulatory signal is also required. Thus, T cell activation can be said to be mediated by two distinct classes of cytoplasmic signaling sequence: those that initiate antigen-dependent primary activation through the TCR (primary cytoplasmic signaling sequences) and those that act in an antigen-independent manner to provide a secondary or co-stimulatory signal (secondary cytoplasmic signaling sequences).

The CAR of the disclosure comprises a primary cytoplasmic signaling sequence derived from CD3-zeta. Further, the cytoplasmic domain of the CAR of the disclosure is designed to comprise the CD3-zeta signaling domain combined with a costimulatory signaling region derived from 4-1BB.

The term "4-1BB" refers to a membrane receptor protein also termed CD137, which is a member of the tumor necrosis factor receptor (TNFR) superfamily expressed on the surface of activated T cells as a type of accessory molecule. 4-1BB has a molecular weight of 55 kDa, and is found as a homodimer.

T cell surface glycoprotein CD3-zeta chain also known as T cell receptor T3 zeta chain or CD247 is a protein that in humans is encoded by the CD247 gene. T cell receptor zeta ($\zeta$), together with T cell receptor alpha/beta and gamma/delta heterodimers and CD3-gamma, - delta, and -epsilon, forms the T cell receptor-CD3 complex. The zeta chain plays an important role in coupling antigen recognition to several intracellular signal-transduction pathways. Low expression of the antigen results in impaired immune response.

The cytoplasmic signaling sequences within the cytoplasmic signaling portion of the CAR of the disclosure may be linked to each other in a random or specified order. Optionally, a short oligo- or polypeptide linker, preferably between 2 and 10 amino acids in length may form the linkage. A glycine-serine doublet provides a particularly suitable linker.

Thus, the cytoplasmic domain in the CAR of the disclosure is designed to comprise the signaling domain of CD3-zeta and the signaling domain of 4-1BB. In one embodiment, the cytoplasmic domain in the CAR of the disclosure is designed to comprise the signaling domain of 4-1BB and the signaling domain of CD3-zeta, wherein the signaling domain of 4-1BB comprises the amino acid sequence of SEQ ID NO: 30 or a functional variant thereof and the signaling domain of CD3-zeta comprises the amino acid sequence of SEQ ID NO: 31 or a functional variant thereof.

In one embodiment, the CAR of the disclosure comprises a signal peptide which directs the nascent protein into the endoplasmic reticulum. In one embodiment, the signal peptide precedes the antigen binding domain. In one embodiment, the signal peptide is derived from an immunoglobulin such as IgG. In one embodiment, the signal peptide comprises the sequence according to SEQ ID NO: 25 or a functional variant thereof.

In one embodiment, a CAR of the disclosure comprises the following elements in the following order: NH2 - CLDN6 antigen binding domain - transmembrane domain - 4-1BB costimulatory domain - CD3-zeta signaling domain - COOH.

In one embodiment, a CAR of the disclosure comprises the following elements in the following order: NH2 - CLDN6 antigen binding domain - CD8α hinge - CD8α transmembrane domain - 4-1BB costimulatory domain - CD3-zeta signaling domain - COOH.

In one embodiment, the CAR of the disclosure comprises the amino acid sequence of SEQ ID NO: 36 or a functional variant thereof.

Vectors

The present disclosure encompasses a nucleic acid construct such as a DNA construct comprising sequences encoding a CAR of the disclosure.

The present disclosure also provides vectors in which a DNA of the present disclosure is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

In brief summary, the expression of natural or synthetic nucleic acids encoding CARs is typically achieved by operably linking a nucleic acid encoding the CAR polypeptide or portions thereof to a promoter, and incorporating the construct into an expression vector. The vectors can be suitable for replication and integration in eukaryotes. Typical cloning vectors contain transcription and translation terminators, initiation sequences, and promoters useful for regulation of the expression of the desired nucleic acid sequence.

The nucleic acid of the disclosure can be cloned into a number of types of vectors. For example, the nucleic acid can be cloned into a vector including, but not limited to a plasmid, a phagemid, a phage derivative, an animal virus, and a cosmid or a transposon. Vectors of particular interest include expression vectors, replication vectors, probe generation vectors, and sequencing vectors. Further, an expression vector may be provided to a cell in the form of a viral vector. Viral vector technology is well known in the art and is described, for example, in Sambrook et al. (2001, Molecular Cloning: A Laboratory Manual, Cold Spring Harbor Laboratory, New York), and in other virology and molecular biology manuals.

Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U,S, Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription. One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1$\alpha$ (EF-1$\alpha$). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the disclosure should not be limited to the use of constitutive promoters, inducible promoters are also contemplated as part of the disclosure. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

In order to assess the expression of a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene. Suitable expression systems are well known and may be prepared using known techniques or obtained commercially.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

Cells

The cells used in connection with the CAR system of the present disclosure and into which nucleic acids (DNA or RNA) encoding the CAR system of the present disclosure may be introduced include any cell with lytic potential, in particular lymphoid cells, and are preferably T cells, in particular cytotoxic lymphocytes, preferably selected from cytotoxic T cells, natural killer (NK) cells, and lymphokine-activated killer (LAK) cells. Upon activation, each of these cytotoxic lymphocytes triggers the destruction of target cells. For example, cytotoxic T cells trigger the destruction of target cells by either or both of the following means. First, upon activation T cells release cytotoxins such as perforin, granzymes, and granulysin. Perforin and granulysin create pores in the target cell, and granzymes enter the cell and trigger a caspase cascade in the cytoplasm that induces apoptosis (programmed cell death) of the cell. Second, apoptosis can be induced via Fas-Fas ligand interaction between the T cells and target cells. The cytotoxic lymphocytes will preferably be autologous cells, although heterologous cells or allogenic cells can be used.

The terms "T cell" and "T lymphocyte" are used interchangeably herein and include T helper cells (CD4+ T cells) and cytotoxic T cells (CTLs, CD8+ T cells) which comprise cytolytic T cells. The term "antigen-specific T cell" or similar terms relate to a T cell which recognizes the antigen to which the T cell is targeted and preferably exerts effector functions of T cells. T cells are considered to be specific for antigen if the cells kill target cells expressing an antigen. T cell specificity may be evaluated using any of a variety of standard techniques, for example, within a chromium release assay or proliferation assay. Alternatively, synthesis of lymphokines (such as interferon-y) can be measured.

As used herein, the term "NK cell" or "Natural Killer cell" refer to a subset of peripheral blood lymphocytes defined by the expression of CD56 or CD16 and the absence of the T cell receptor (CD3). As provided herein, the NK cell can also be differentiated from a stem cell or progenitor cell.

The term "effector functions" in the context of the present disclosure includes any functions mediated by components of the immune system that result, for example, in the killing of diseased cells such as tumor cells, or in the inhibition of tumor growth and/or inhibition of tumor development, including inhibition of tumor dissemination and metastasis. Preferably, the effector functions in the context of the present disclosure are T cell mediated effector functions. Such functions comprise in the case of a helper T cell (CD4$^+$ T cell) the release of cytokines and/or the activation of CD8$^+$ lymphocytes (CTLs) and/or B cells, and in the case of CTL the elimination of cells, i.e., cells characterized by expression of an antigen, for example, via apoptosis or perforin-mediated cell lysis, production of cytokines such as IFN-y and TNF-$\alpha$, and specific cytolytic killing of antigen expressing target cells.

The term "immune effector cell" or "immunoreactive cell" in the context of the present disclosure relates to a cell which exerts effector functions during an immune reaction. An "immune effector cell" preferably is capable of binding an antigen such as an antigen expressed on the surface of a cell and mediating an immune response. For example, immune effector cells comprise T cells (cytotoxic T cells, helper T cells, tumor infiltrating T cells), B cells, natural killer cells, neutrophils, macrophages, and dendritic cells. Preferably, in the context of the present disclosure, "immune effector cells" are T cells, preferably CD4$^+$ and/or CD8$^+$ T cells. According to the disclosure, the term "immune effector cell" also includes a cell which can mature into an immune cell (such as T cell, in particular T helper cell, or cytolytic T cell) with suitable stimulation. Immune effector cells comprise CD34$^+$ hematopoietic stem cells, immature and mature T cells and immature and mature B cells. The differentiation of T cell precursors into a cytolytic T cell, when exposed to an antigen, is similar to clonal selection of the immune system.

Preferably, an "immune effector cell" recognizes an antigen with some degree of specificity, in particular if present on the surface of diseased cells such as cancer cells. Preferably, said recognition enables the cell that recognizes an antigen to be responsive or reactive. If the cell is a helper T cell (CD4$^+$ T cell) such responsiveness or reactivity may involve the release of cytokines and/or the activation of CD8$^+$ lymphocytes (CTLs) and/or B cells. If the cell is a CTL such responsiveness or reactivity may involve the elimination of cells, i.e., cells characterized by expression of an antigen, for example, via apoptosis or perforin-mediated cell lysis. According to the disclosure, CTL responsiveness may include sustained calcium flux, cell division, production of cytokines such as IFN-y and TNF-$\alpha$, up-regulation of activation markers such as CD44 and CD69, and specific cytolytic killing of antigen expressing target cells. CTL responsiveness may also be determined using an artificial reporter that accurately indicates CTL responsiveness. Such CTL that recognizes an antigen and are responsive or reactive are also termed "antigen-responsive CTL" herein.

A "lymphoid cell" is a cell which, optionally after suitable modification, e.g. after transfer of a CAR, is capable of producing an immune response such as a cellular immune response, or a precursor cell of such cell, and includes lymphocytes, preferably T lymphocytes, lymphoblasts, and plasma cells. A lymphoid cell may be an immune effector cell as described herein. A preferred lymphoid cell is a T cell which can be modified to express a CAR on the cell surface. In one embodiment, the lymphoid cell lacks endogenous expression of a T cell receptor.

The terms "T cell" and "T lymphocyte" are used interchangeably herein and include T helper cells (CD4+ T cells) and cytotoxic T cells (CTLs, CD8+ T cells) which comprise cytolytic T cells. T cells belong to a group of white blood cells known as lymphocytes, and play a central role in cell-mediated immunity. They can be distinguished from other lymphocyte types, such as B cells and natural killer cells by the presence of a special receptor on their cell surface called T cell receptor (TCR). The thymus is the principal organ responsible for the maturation of T cells. Several different subsets of T cells have been discovered, each with a distinct function.

T helper cells assist other white blood cells in immunologic processes, including maturation of B cells into plasma cells and activation of cytotoxic T cells and macrophages, among other functions. These cells are also known as CD4+ T cells because they express the CD4 protein on their surface. Helper T cells become activated when they are presented with peptide antigens by MHC class II molecules that are expressed on the surface of antigen presenting cells (APCs). Once activated, they divide rapidly and secrete small proteins called cytokines that regulate or assist in the active immune response.

Cytotoxic T cells destroy virally infected cells and tumor cells, and are also implicated in transplant rejection. These cells are also known as CD8+ T cells since they express the CD8 glycoprotein at their surface. These cells recognize their targets by binding to antigen associated with MHC class I, which is present on the surface of nearly every cell of the body.

A majority of T cells have a T cell receptor (TCR) existing as a complex of several proteins. The actual T cell receptor is composed of two separate peptide chains, which are produced from the independent T cell receptor alpha and beta (TCRα and TCRβ) genes and are called α- and β-TCR chains. γδ T cells (gamma delta T cells) represent a small subset of T cells that possess a distinct T cell receptor (TCR) on their surface. However, in γδ T cells, the TCR is made up of one γ-chain and one δ-chain. This group of T cells is much less common (2% of total T cells) than the αβ T cells.

All T cells originate from hematopoietic stem cells in the bone marrow. Hematopoietic progenitors derived from hematopoietic stem cells populate the thymus and expand by cell division to generate a large population of immature thymocytes. The earliest thymocytes express neither CD4 nor CD8, and are therefore classed as double-negative (CD4-CD8-) cells. As they progress through their development they become double-positive thymocytes (CD4+CD8+), and finally mature to single-positive (CD4+CD8- or CD4-CD8+) thymocytes that are then released from the thymus to peripheral tissues.

T cells may generally be prepared *in vitro* or *ex vivo,* using standard procedures. For example, T cells may be isolated from bone marrow, peripheral blood or a fraction of bone marrow or peripheral blood of a mammal, such as a patient, using a commercially available cell separation system. Alternatively, T cells may be derived from related or unrelated humans, non-human animals, cell lines or cultures. A sample comprising T cells may, for example, be peripheral blood mononuclear cells (PBMC).

The T cells to be used according to the disclosure may express an endogenous T cell receptor or may lack expression of an endogenous T cell receptor.

The term "CAR targeted to an antigen" relates to a CAR which when present on an immune effector cell such as a T cell recognizes the antigen such as on the surface of antigen presenting cells or diseased cells such as cancer cells, such that the immune effector cell is stimulated, primed and/or expanded or exerts effector functions of immune effector cells as described above.

Sources of T cells

Prior to expansion and genetic modification of the T cells of the disclosure, a source of T cells is obtained from a subject. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present disclosure, any number of T cell lines available in the art, may be used. In certain embodiments of the present disclosure, T cells can be obtained from a unit of blood collected from a subject using any number of techniques known to the skilled artisan, such as Ficoll™ separation. In one preferred embodiment, cells from the circulating blood of an individual are obtained by apheresis. The apheresis product typically contains lymphocytes, including T cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing steps. In one embodiment of the disclosure, the cells are washed with phosphate buffered saline (PBS). In an alternative embodiment, the wash solution lacks calcium and may lack magnesium or may lack many if not all divalent cations. Again, surprisingly, initial activation steps in the absence of calcium lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, $Ca^{2+}$-free, $Mg^{2+}$-free PBS, PlasmaLyte A, or other saline solution with or without buffer. Alternatively, the undesirable components of the apheresis sample may be removed and the cells directly resuspended in culture media.

In another embodiment, T cells are isolated from peripheral blood lymphocytes by lysing the red blood cells and depleting the monocytes, for example, by centrifugation through a PERCOLL™ gradient or by counterflow centrifugal elutriation. A specific subpopulation of T cells, such as $CD3^+$, $CD28^+$, $CD4^+$, $CD8^+$, $CD45RA^+$, and $CD45RO^+$ T cells, can be further isolated by positive or negative selection techniques. For example, in one embodiment, T cells are isolated by incubation with anti-CD3/anti-CD28 (i.e., 3x28)-conjugated beads, such as DYNABEADS® M-450 CD3/CD28 T, for a time period sufficient for positive selection of the desired T cells. In one embodiment, the time period ranges from 30 minutes to 36 hours or longer. The skilled artisan would recognize that multiple rounds of selection can also be used in the context of this disclosure.

Enrichment of a T cell population by negative selection can be accomplished with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4$^+$ cells by negative selection, a monoclonal antibody cocktail typically includes antibodies to CD14, CD20, CD11b, CD16, HLA-DR, and CD8. In certain embodiments, it may be desirable to enrich for or positively select for regulatory T cells which typically express CD4$^+$, CD25$^+$, CD62L$^{hi}$ GITR$^+$, and FoxP3$^+$. Alternatively, in certain embodiments, T regulatory cells are depleted by anti-CD25 conjugated beads or other similar method of selection.

A variety of methods may be used to introduce CAR constructs into T cells including non-viral-based DNA transfection, transposon-based systems, viral-based systems and RNA-based systems. Non-viral-based DNA transfection has low risk of insertional mutagenesis. Transposon-based systems can integrate transgenes more efficiently than plasmids that do not contain an integrating element. Viral-based systems include the use of γ-retroviruses and lentiviral vectors. γ-Retroviruses are relatively easy to produce, efficiently and permanently transduce T cells, and have preliminarily proven safe from an integration standpoint in primary human T cells. Lentiviral vectors also efficiently and permanently transduce T cells but are more expensive to manufacture. They are also potentially safer than retrovirus based systems.

In one embodiment of the disclosure, T cells or T cell progenitors are transfected either *ex vivo* or *in vivo* with a nucleic acid encoding the CAR to provide T cells genetically modified to express a CAR.

CAR T cells may be produced *in vivo,* and therefore nearly instantaneously, using nanoparticles targeted to T cells. For example, poly(β-amino ester)-based nanoparticles may be coupled to anti-CD3e f(ab) fragments for binding to CD3 on T cells. Upon binding to T cells, these nanoparticles are endocytosed. Their contents, for example plasmid DNA encoding an anti-tumor antigen CAR, may be directed to the T cell nucleus due to the inclusion of peptides containing microtubule-associated sequences (MTAS) and nuclear localization signals (NLSs). The inclusion of transposons with inverted repeats (IRs) flanking the CAR gene expression cassette and a separate plasmid encoding a hyperactive transposase, may allow for the efficient integration of the CAR vector into chromosomes. Such system that allows for the *in vivo* production of CAR T cells following nanoparticle infusion is described in Smith et al. (2017) Nat. Nanotechnol. 12:813-820.

Another possibility is to use the CRISPR/Cas9 method to deliberately place a CAR coding sequence at a specific locus. For example, existing T cell receptors (TCR) may be knocked out, while knocking in the CAR and placing it under the dynamic regulatory control of the endogenous promoter that would otherwise moderate TCR expression; c.f., e.g., Eyquem et al. (2017) Nature 543:113-117.

In one embodiment of all aspects of the disclosure, the T cells genetically modified to express a CAR are stably or transiently transfected with nucleic acid encoding the CAR. Thus, the nucleic acid encoding the CAR is integrated or not integrated into the genome of the T cells.

In one embodiment of all aspects of the disclosure, the T cells or T cell progenitors are from the subject to be treated. In one embodiment of all aspects of the disclosure, the T cells or T cell progenitors are from a subject which is different to the subject to be treated.

In one embodiment of all aspects of the disclosure, the T cells may be autologous, allogeneic or syngeneic to the subject to be treated. The T cells may be genetically modified *in vitro* to express a chimeric antigen receptor (CAR).

In one embodiment of all aspects of the disclosure, the T cells genetically modified to express a CAR are inactivated for expression of an endogenous T cell receptor and/or endogenous HLA.

Activation and Expansion of T Cells

Whether prior to or after genetic modification of the T cells to express a desirable CAR, the T cells can be activated and expanded generally using methods known in the art.

Generally, the T cells of the disclosure are expanded by contact with a surface having attached thereto an agent that stimulates a CD3/TCR complex associated signal and a ligand that stimulates a co-stimulatory molecule on the surface of the T cells. In particular, T cell populations may be stimulated as described herein, such as by contact with an anti-CD3 antibody, or antigen binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells.

In certain embodiments, the primary stimulatory signal and the co-stimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one embodiment, the agent providing the co-stimulatory signal is bound to a cell surface and the agent providing the primary activation signal is in solution or coupled to a surface. In certain embodiments, both agents can be in solution.

In one embodiment, the two agents are immobilized on beads, either on the same bead, i.e., "cis", or to separate beads, i.e., "trans." By way of example, the agent providing the primary activation signal is an anti-CD3 antibody or an antigen binding fragment thereof and the agent providing the co-stimulatory signal is an anti-CD28 antibody or antigen binding fragment thereof; and both agents are co-immobilized to the same bead. In one embodiment, the ratio of CD3:CD28 antibody bound to the beads ranges from 100:1 to 1:100.

Ratios of particles to cells from 1:500 to 500:1 may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain embodiments the ratio of cells to particles ranges from 1:100 to 100:1 and in further embodiments the ratio comprises 1:9 to 9:1.

Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-$\gamma$, IL-4, IL-7, GM-CSF, IL- 10, IL-12, IL-15, TGF$\beta$, and TNF-$\alpha$ or any other additives for the growth of cells known to the skilled artisan. Other additives for the growth of cells include, but are not limited to, surfactant, plasmanate, and reducing agents such as N-acetyl-cysteine and 2-mercaptoethanol. The target cells are maintained under conditions necessary to support growth, for example, an appropriate temperature (e.g., 37° C) and atmosphere (e.g., air plus 5% $CO_2$).

Therapeutic Application

The present disclosure encompasses a cell (e.g., T cell) comprising a CAR molecule of the disclosure, e.g., transduced with a retroviral such as lentiviral vector (LV) that encodes a CAR of the disclosure. Therefore, in some instances, the transduced T cell can elicit a CAR-mediated T cell response.

The disclosure provides the use of a CAR to redirect the specificity of a primary T cell to the tumor antigen CLDN6. Thus, the present disclosure also provides a method for stimulating a T cell-mediated immune response to a target cell population or tissue in a mammal comprising the step of administering to the mammal a T cell that expresses a CAR of the disclosure, wherein the CAR comprises a binding moiety that specifically interacts with CLDN6 as a predetermined target.

In one embodiment, the present disclosure includes a type of cellular therapy where T cells are genetically modified to express a CAR of the disclosure and the CAR T cell is infused to a recipient in need thereof. The infused cell is able to kill tumor cells in the recipient. Unlike antibody therapies, CAR T cells are able to replicate *in vivo* resulting in long-term persistence that can lead to sustained tumor control.

In one embodiment, the CAR T cells of the disclosure can undergo robust *in vivo* T cell expansion and can persist for an extended amount of time. In another embodiment, the CAR T cells of the disclosure evolve into specific memory T cells that can be reactivated to inhibit any additional tumor formation or growth.

Cancers that may be treated include tumors that are not vascularized, or not yet substantially vascularized, as well as vascularized tumors. The cancers may comprise solid tumors. Types of cancers to be treated with the CARs of the disclosure include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors, such as sarcomas and carcinomas, include fibrosarcoma, myxosarcoma, liposarcoma, chondrosarcoma, osteosarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous eel! carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (such as a glioma (such as brainstem glioma and mixed gliomas), glioblastoma (also known as glioblastoma multiforme) astrocytoma, CNS lymphoma, germinoma, medulloblastoma, Schwannoma craniopharyogioma, ependymoma, pineaioma, hemangioblastoma, acoustic neuroma, oligodendroglioma, menangioma, neuroblastoma, retinoblastoma and brain metastases).

In one embodiment, the cancers that may be treated are CLDN6 expressing cancers such as those that are described herein.

In one embodiment of the disclosure, cells are isolated from a mammal (preferably a human) and genetically modified (i.e., transduced or transfected *in vitro*) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

The CAR-modified cells of the present disclosure may be administered either alone, or in combination with other components such as IL-2 or other cytokines or cell populations. In one embodiment, the CAR-modified cells of the present disclosure are administered in combination with a cognate antigen molecule, or a nucleic acid, in particular RNA, encoding a cognate antigen molecule. The cognate antigen molecule may be CLDN6, recombinant CLDN6, a CLDN6 fragment, or a variant of any of the foregoing. In one embodiment a nucleic acid, in particular RNA, encoding the cognate antigen molecule (e.g., CLDN6, recombinant CLDN6, a CLDN6 fragment, or a variant of any of the foregoing) is administered. Preferably, the nucleic acid encoding the cognate antigen molecule is expressed in cells of the subject being administered the CAR-modified cells of the present disclosure and the nucleic acid encoding the cognate antigen molecule to provide the cognate antigen molecule for binding by the CAR antigen binding domain. In one embodiment, expression of the cognate antigen molecule of the CAR antigen binding domain is at the cell surface. In one embodiment, the nucleic acid encoding the cognate antigen molecule is transiently expressed in cells of the subject. In one embodiment, the nucleic coding for the cognate antigen molecule is RNA. Preferably, contacting the CAR-modified cells of the present disclosure with the cognate antigen molecule results in expansion and/or activation of the cells.

The peptide and protein antigens suitable for use according to the disclosure typically include a peptide or protein comprising an epitope to which the CLDN6 antigen binding domain of the CAR of the disclosure binds. The peptide or protein or epitope may be derived from CLDN6. For example, the peptide or protein antigen or the epitope contained within the peptide or protein antigen may be CLDN6 or a fragment or variant of CLDN6.

A peptide and protein antigen provided to a subject according to the disclosure (either by administering the peptide and protein antigen or a nucleic acid, in particular RNA, encoding the peptide and protein antigen), i.e., a vaccine antigen, preferably results in stimulation, priming and/or expansion of CAR-modified cells in the subject being administered the CAR-modified cells and the antigen or nucleic acid. Said stimulated, primed and/or expanded CAR-modified cells are preferably directed against the CLDN6 target antigen, in particular the target antigen expressed by diseased cells, tissues and/or organs, i.e., the disease-associated antigen. Thus, a vaccine antigen may comprise the disease-associated antigen, or a fragment or variant thereof. In one embodiment, such fragment or variant is immunologically equivalent to the disease-associated antigen. In the context of the present disclosure, the term "fragment of an antigen" or "variant of an antigen" means an agent which results in stimulation, priming and/or expansion of CAR-modified cells which stimulated, primed and/or expanded CAR-modified cells target the disease-associated antigen, in particular when expressed on the surface of diseased cells, tissues and/or organs. Thus, the vaccine antigen administered according to the disclosure may correspond to or may comprise the disease-associated antigen, may correspond to or may comprise a fragment of the disease-associated antigen or may correspond to or may comprise an antigen which is homologous to the disease-associated antigen or a fragment thereof. If the vaccine antigen administered according to the disclosure comprises a fragment of the disease-associated antigen or an amino acid sequence which is homologous to a fragment of the disease-associated antigen said fragment or amino acid sequence may comprise an epitope of the disease-associated antigen or a sequence which is homologous to an epitope of the disease-associated antigen, wherein the CAR-modified cells bind to said epitope. Thus, according to the disclosure, an antigen may comprise an immunogenic fragment of the disease-associated antigen or an amino acid sequence being homologous to an immunogenic fragment of the disease-associated antigen. An "immunogenic fragment of an antigen" according to the disclosure preferably relates to a fragment of an antigen which is capable of stimulating, priming and/or expanding CAR-modified cells. It is preferred that the vaccine antigen (similar to the disease-associated antigen) provides the relevant epitope for binding by the CLDN6 antigen binding domain present in the CAR of the CAR-modified cells. It is also preferred that the vaccine antigen (similar to the disease-associated antigen) is expressed on the surface of a cell such as an antigen-presenting cell so as to provide the relevant epitope for binding by the CAR. The vaccine antigen according to the disclosure may be a recombinant antigen.

The term "immunologically equivalent" means that the immunologically equivalent molecule such as the immunologically equivalent amino acid sequence exhibits the same or essentially the same immunological properties and/or exerts the same or essentially the same immunological effects, e.g., with respect to the type of the immunological effect. In the context of the present disclosure, the term "immunologically equivalent" is preferably used with respect to the immunological effects or properties of antigens or antigen variants. For example, an amino acid sequence is immunologically equivalent to a reference amino acid sequence, e.g., CLDN6, if said amino acid sequence when exposed to CAR-modified cells binding to the reference amino acid sequence or cells expressing the reference amino acid sequence induces an immune reaction having a specificity of reacting with the reference amino acid sequence, in particular stimulation, priming and/or expansion of CAR-modified cells. Thus, a molecule which is immunologically equivalent to an antigen exhibits the same or essentially the same properties and/or exerts the same or essentially the same effects regarding the stimulation, priming and/or expansion of CAR-modified cells as the antigen to which the CAR-modified cells are targeted.

"Activation" or "stimulation", as used herein, refers to the state of a T cell that has been sufficiently stimulated to induce detectable cellular proliferation. Activation can also be associated with induced cytokine production, and detectable effector functions. The term "activated T cells" refers to, among other things, T cells that are undergoing cell division.

The term "priming" refers to a process wherein a T cell has its first contact with its specific antigen and causes differentiation into effector T cells.

The term "clonal expansion" or "expansion" refers to a process wherein a specific entity is multiplied. In the context of the present disclosure, the term is preferably used in the context of an immunological response in which lymphocytes are stimulated by an antigen, proliferate, and the specific lymphocyte recognizing said antigen is amplified. Preferably, clonal expansion leads to differentiation of the lymphocytes.

It is particularly preferred according to the disclosure that the cognate antigen is administered in the form of RNA encoding the antigen. After administration of the RNA, at least a portion of the RNA is delivered to a target cell. In one embodiment, at least a portion of the RNA is delivered to the cytosol of the target cell. In one embodiment, the RNA is translated by the target cell to produce the encoded peptide or protein. Some embodiments involve the targeted delivery of the RNA to certain tissues. In one embodiment, the delivery involves targeting the lymphatic system, in particular secondary lymphoid organs, more specifically spleen. In one embodiment, the target cell is a spleen cell. In one embodiment, the target cell is an antigen presenting cell such as a professional antigen presenting cell in the spleen. In one embodiment, the target cell is a dendritic cell in the spleen.

The "lymphatic system" is part of the circulatory system and an important part of the immune system, comprising a network of lymphatic vessels that carry lymph. The lymphatic system consists of lymphatic organs, a conducting network of lymphatic vessels, and the circulating lymph. The primary or central lymphoid organs generate lymphocytes from immature progenitor cells. The thymus and the bone marrow constitute the primary lymphoid organs. Secondary or peripheral lymphoid organs, which include lymph nodes and the spleen, maintain mature naive lymphocytes and initiate an adaptive immune response.

RNA may be delivered to spleen by so-called lipoplex formulations, in which the RNA is bound to liposomes comprising a cationic lipid and optionally an additional or helper lipid to form injectable nanoparticle formulations. The liposomes may be obtained by injecting a solution of the lipids in ethanol into water or a suitable aqueous phase. RNA lipoplex particles may be prepared by mixing the liposomes with RNA. Spleen targeting RNA lipoplex particles are described in WO 2013/143683. It has been found that RNA lipoplex particles having a net negative charge may be used to preferentially target spleen tissue or spleen cells such as antigen-presenting cells, in particular dendritic cells. Accordingly, following administration of the RNA lipoplex particles, RNA accumulation and/or RNA expression in the spleen occurs. Thus, RNA lipoplex particles of the disclosure may be used for expressing RNA in the spleen. In an embodiment, after administration of the RNA lipoplex particles, no or essentially no RNA accumulation and/or RNA expression in the lung and/or liver occurs. In one embodiment, after administration of the RNA lipoplex particles, RNA accumulation and/or RNA expression in antigen presenting cells, such as professional antigen presenting cells in the spleen occurs. Thus, RNA lipoplex particles of the disclosure may be used for expressing RNA in such antigen presenting cells. In one embodiment, the antigen presenting cells are dendritic cells and/or macrophages.

In the context of the present disclosure, the term "RNA lipoplex particle" relates to a particle that contains lipid, in particular cationic lipid, and RNA. Electrostatic interactions between positively charged liposomes and negatively charged RNA results in complexation and spontaneous formation of RNA lipoplex particles. Positively charged liposomes may be generally synthesized using a cationic lipid, such as DOTMA, and additional lipids, such as DOPE. In one embodiment, a RNA lipoplex particle is a nanoparticle.

As used herein, a "cationic lipid" refers to a lipid having a net positive charge. Cationic lipids bind negatively charged RNA by electrostatic interaction to the lipid matrix. Generally, cationic lipids possess a lipophilic moiety, such as a sterol, an acyl or diacyl chain, and the head group of the lipid typically carries the positive charge. Examples of cationic lipids include, but are not limited to 1,2-di-O-octadecenyl-3-trimethylammonium propane (DOTMA), dimethyldioctadecylammonium (DDAB); 1,2-dioleoyl-3-trimethylammonium propane (DOTAP); 1,2-dioleoyl-3-dimethylammonium-propane (DODAP); 1,2-diacyloxy-3-dimethylammonium propanes; 1,2-dialkyloxy-3- dimethylammonium propanes; dioctadecyldimethyl ammonium chloride (DODAC), 2,3-di(tetradecoxy)propyl-(2-hydroxyethyl)-dimethylazanium (DMRIE), 1,2-dimyristoyl-sn-glycero-3-ethylphosphocholine (DMEPC), 1,2-dimyristoyl-3-trimethylammonium propane (DMTAP), 1,2-dioleyloxypropyl-3-dimethyl-hydroxyethyl ammonium bromide (DORIE), and 2,3-dioleoyloxy- N-[2(spermine carboxamide)ethyl]-N,N-dimethyl-1-propanamium trifluoroacetate (DOSPA). Preferred are DOTMA, DOTAP, DODAC, and DOSPA. In specific embodiments, the cationic lipid is DOTMA and/or DOTAP.

An additional lipid may be incorporated to adjust the overall positive to negative charge ratio and physical stability of the RNA lipoplex particles. In certain embodiments, the additional lipid is a neutral lipid. As used herein, a "neutral lipid" refers to a lipid having a net charge of zero. Examples of neutral lipids include, but are not limited to, 1,2-di-(9Z-octadecenoyl)-sn-glycero-3-phosphoethanolamine (DOPE), 1,2-dioleoyl-sn-glycero-3-phosphocholine (DOPC), diacylphosphatidyl choline, diacylphosphatidyl ethanol amine, ceramide, sphingoemyelin, cephalin, cholesterol, and cerebroside. In specific embodiments, the additional lipid is DOPE, cholesterol and/or DOPC.

In certain embodiments, the RNA lipoplex particles include both a cationic lipid and an additional lipid. In an exemplary embodiment, the cationic lipid is DOTMA and the additional lipid is DOPE.

In some embodiments, the molar ratio of the at least one cationic lipid to the at least one additional lipid is from about 10:0 to about 1:9, about 4:1 to about 1:2, or about 3:1 to about 1:1. In specific embodiments, the molar ratio may be about 3:1, about 2.75:1, about 2.5:1, about 2.25:1, about 2:1, about 1.75:1, about 1.5:1, about 1.25:1, or about 1:1. In an exemplary embodiment, the molar ratio of the at least one cationic lipid to the at least one additional lipid is about 2:1.

RNA lipoplex particles described herein have an average diameter that in one embodiment ranges from about 200 nm to about 1000 nm, from about 200 nm to about 800 nm, from about 250 to about 700 nm, from about 400 to about 600 nm, from about 300 nm to about 500 nm, or from about 350 nm to about 400 nm. In specific embodiments, the RNA lipoplex particles have an average diameter of about 200 nm, about 225 nm, about 250 nm, about 275 nm, about 300 nm, about 325 nm, about 350 nm, about 375 nm, about 400 nm, about 425 nm, about 450 nm, about 475 nm, about 500 nm, about 525 nm, about 550 nm, about 575 nm, about 600 nm, about 625 nm, about 650 nm, about 700 nm, about 725 nm, about 750 nm, about 775 nm, about 800 nm, about 825 nm, about 850 nm, about 875 nm, about 900 nm, about 925 nm, about 950 nm, about 975 nm, or about 1000 nm. In an embodiment, the RNA lipoplex particles have an average diameter that ranges from about 250 nm to about 700 nm. In another embodiment, the RNA lipoplex particles have an average diameter that ranges from about 300 nm to about 500 nm. In an exemplary embodiment, the RNA lipoplex particles have an average diameter of about 400 nm.

The electric charge of the RNA lipoplex particles of the present disclosure is the sum of the electric charges present in the at least one cationic lipid and the electric charges present in the RNA. The charge ratio is the ratio of the positive charges present in the at least one cationic lipid to the negative charges present in the RNA. The charge ratio of the positive charges present in the at least one cationic lipid to the negative charges present in the RNA is calculated by the following equation: charge ratio=[(cationic lipid concentration (mol)) * (the total number of positive charges in the cationic lipid)] / [(RNA concentration (mol)) * (the total number of negative charges in RNA)].

The spleen targeting RNA lipoplex particles described herein at physiological pH preferably have a net negative charge such as a charge ratio of positive charges to negative charges from about 1.9:2 to about 1:2. In specific embodiments, the charge ratio of positive charges to negative charges in the RNA lipoplex particles at physiological pH is about 1.9:2.0, about 1.8:2.0, about 1.7:2.0, about 1.6:2.0, about 1.5:2.0, about 1.4:2.0, about 1.3:2.0, about 1.2:2.0, about 1.1:2.0, or about 1:2.0.

The CAR-modified cells and further agents described herein may be administered in pharmaceutical compositions or medicaments for therapeutic or prophylactic treatments and may be administered in the form of any suitable pharmaceutical composition.

The term "pharmaceutical composition" relates to a formulation comprising a therapeutically effective agent, preferably together with pharmaceutically acceptable carriers, diluents and/or excipients. Said pharmaceutical composition is useful for treating, preventing, or reducing the severity of a disease or disorder by administration of said pharmaceutical composition to a subject. A pharmaceutical composition is also known in the art as a pharmaceutical formulation.

The pharmaceutical compositions of the present disclosure may comprise one or more adjuvants or may be administered with one or more adjuvants. The term "adjuvant" relates to a compound which prolongs, enhances or accelerates an immune response. Adjuvants comprise a heterogeneous group of compounds such as oil emulsions (e.g., Freund's adjuvants), mineral compounds (such as alum), bacterial products (such as Bordetella pertussis toxin), or immune-stimulating complexes. Examples of adjuvants include, without limitation, LPS, GP96, CpG oligodeoxynucleotides, growth factors, and cyctokines, such as monokines, lymphokines, interleukins, chemokines. The chemokines may be IL1, IL2, IL3, IL4, IL5, IL6, IL7, IL8, IL9, IL10, IL12, IFN$\alpha$, IFN$\gamma$, GM-CSF, LT-a. Further known adjuvants are aluminium hydroxide, Freund's adjuvant or oil such as Montanide® ISA51. Other suitable adjuvants for use in the present disclosure include lipopeptides, such as Pam3Cys.

The pharmaceutical compositions according to the present disclosure are generally applied in a "pharmaceutically effective amount" and in "a pharmaceutically acceptable preparation".

The term "pharmaceutically acceptable" refers to the non-toxicity of a material which does not interact with the action of the active component of the pharmaceutical composition.

The term "pharmaceutically effective amount" or "therapeutically effective amount" refers to the amount which achieves a desired reaction or a desired effect alone or together with further doses. In the case of the treatment of a particular disease, the desired reaction preferably relates to inhibition of the course of the disease. This comprises slowing down the progress of the disease and, in particular, interrupting or reversing the progress of the disease. The desired reaction in a treatment of a disease may also be delay of the onset or a prevention of the onset of said disease or said condition. An effective amount of the compositions described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the compositions described herein may depend on various of such parameters. In the case that a reaction in a patient is insufficient with an initial dose, higher doses (or effectively higher doses achieved by a different, more localized route of administration) may be used.

The pharmaceutical compositions of the present disclosure may contain salts, buffers, preservatives, and optionally other therapeutic agents. In one embodiment, the pharmaceutical compositions of the present disclosure comprise one or more pharmaceutically acceptable carriers, diluents and/or excipients.

Suitable preservatives for use in the pharmaceutical compositions of the present disclosure include, without limitation, benzalkonium chloride, chlorobutanol, paraben and thimerosal.

The term "excipient" as used herein refers to a substance which may be present in a pharmaceutical composition of the present disclosure but is not an active ingredient. Examples of excipients, include without limitation, carriers, binders, diluents, lubricants, thickeners, surface active agents, preservatives, stabilizers, emulsifiers, buffers, flavoring agents, or colorants.

The term "diluent" relates a diluting and/or thinning agent. Moreover, the term "diluent" includes any one or more of fluid, liquid or solid suspension and/or mixing media. Examples of suitable diluents include ethanol, glycerol and water.

The term "carrier" refers to a component which may be natural, synthetic, organic, inorganic in which the active component is combined in order to facilitate, enhance or enable administration of the pharmaceutical composition. A carrier as used herein may be one or more compatible solid or liquid fillers, diluents or encapsulating substances, which are suitable for administration to subject. Suitable carrier include, without limitation, sterile water, Ringer, Ringer lactate, sterile sodium chloride solution, isotonic saline, polyalkylene glycols, hydrogenated naphthalenes and, in particular, biocompatible lactide polymers, lactide/glycolide copolymers or polyoxyethylene/polyoxy-propylene copolymers. In one embodiment, the pharmaceutical composition of the present disclosure includes isotonic saline.

Pharmaceutically acceptable carriers, excipients or diluents for therapeutic use are well known in the pharmaceutical art, and are described, for example, in Remington's Pharmaceutical Sciences, Mack Publishing Co. (A. R Gennaro edit. 1985).

Pharmaceutical carriers, excipients or diluents can be selected with regard to the intended route of administration and standard pharmaceutical practice.

In one embodiment, pharmaceutical compositions described herein may be administered intravenously, intraarterially, subcutaneously, intradermally or intramuscularly. In certain embodiments, the pharmaceutical composition is formulated for local administration or systemic administration. Systemic administration may include enteral administration, which involves absorption through the gastrointestinal tract, or parenteral administration. As used herein, "parenteral administration" refers to the administration in any manner other than through the gastrointestinal tract, such as by intravenous injection. In a preferred embodiment, the pharmaceutical compositions is formulated for systemic administration. In another preferred embodiment, the systemic administration is by intravenous administration. The compositions may be injected directly into a tumor or lymph node.

The term "co-administering" as used herein means a process whereby different compounds or compositions are administered to the same patient. For example, the CAR-modified cells and the antigen or nucleic acid coding therefor described herein may be administered simultaneously, at essentially the same time, or sequentially. If administration takes place sequentially, the CAR-modified cells may be administered before or after administration of the antigen or nucleic acid coding therefor. If administration takes place simultaneously the CAR-modified cells and the antigen or nucleic acid coding therefor need not be administered within the same composition. The CAR-modified cells and the antigen or nucleic acid coding therefor may be administered one or more times and the number of administrations of each component may be the same or different. In addition, the CAR-modified cells and the antigen or nucleic acid coding therefor need not be administered at the same site.

The term "disease" refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality.

In the present context, the term "treatment", "treating" or "therapeutic intervention" relates to the management and care of a subject for the purpose of combating a condition such as a disease or disorder. The term is intended to include the full spectrum of treatments for a given condition from which the subject is suffering, such as administration of the therapeutically effective compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of an individual for the purpose of combating the disease, condition or disorder and includes the administration of the active compounds to prevent the onset of the symptoms or complications.

The term "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual. The terms "prophylactic treatment" or "preventive treatment" are used herein interchangeably.

The terms "individual" and "subject" are used herein interchangeably. They refer to a human or another mammal (e.g. mouse, rat, rabbit, dog, cat, cattle, swine, sheep, horse or primate) that can be afflicted with or is susceptible to a disease or disorder (e.g., cancer) but may or may not have the disease or disorder. In many embodiments, the individual is a human being. Unless otherwise stated, the terms "individual" and "subject" do not denote a particular age, and thus encompass adults, elderlies, children, and newborns. In embodiments of the present disclosure, the "individual" or "subject" is a "patient".

The term "patient" means an individual or subject for treatment, in particular a diseased individual or subject.

Combination strategies in cancer treatment may be desirable due to a resulting synergistic effect, which may be considerably stronger than the impact of a monotherapeutic approach. In one embodiment, the pharmaceutical composition is administered with an immunotherapeutic agent. As used herein "immunotherapeutic agent" relates to any agent that may be involved in activating a specific immune response and/or immune effector function(s). The present disclosure contemplates the use of an antibody as an immunotherapeutic agent. Without wishing to be bound by theory, antibodies are capable of achieving a therapeutic effect against cancer cells through various mechanisms, including inducing apoptosis, block components of signal transduction pathways or inhibiting proliferation of tumor cells. In certain embodiments, the antibody is a monoclonal antibody. A monoclonal antibody may induce cell death via antibody-dependent cell mediated cytotoxicity (ADCC), or bind complement proteins, leading to direct cell toxicity, known as complement dependent cytotoxicity (CDC). Non-limiting examples of anti-cancer antibodies and potential antibody targets (in brackets) which may be used in combination with the present disclosure include: Abagovomab (CA-125), Abciximab (CD41), Adecatumumab (EpCAM), Afutuzumab (CD20), Alacizumab pegol (VEGFR2), Altumomab pentetate (CEA), Amatuximab (MORAb- 009), Anatumomab mafenatox (TAG-72), Apolizumab (HLA-DR), Arcitumomab (CEA), Atezolizumab (PD-L1), Bavituximab (phosphatidylserine), Bectumomab (CD22), Belimumab (BAFF), Bevacizumab (VEGF-A), Bivatuzumab mertansine (CD44 v6), Blinatumomab (CD 19), Brentuximab vedotin (CD30 TNFRSF8), Cantuzumab mertansin (mucin CanAg), Cantuzumab ravtansine (MUC1), Capromab pendetide (prostatic carcinoma cells), Carlumab (CNT0888), Catumaxomab (EpCAM, CD3), Cetuximab (EGFR), Citatuzumab bogatox (EpCAM), Cixutumumab (IGF-1 receptor), Claudiximab (Claudin), Clivatuzumab tetraxetan (MUC1), Conatumumab (TRAIL-R2), Dacetuzumab (CD40), Dalotuzumab (insulin-like growth factor I receptor), Denosumab (RANKL), Detumomab (B-lymphoma cell), Drozitumab (DR5), Ecromeximab (GD3 ganglioside), Edrecolomab (EpCAM), Elotuzumab (SLAMF7), Enavatuzumab (PDL192), Ensituximab (NPC-1C), Epratuzumab (CD22), Ertumaxomab (HER2/neu, CD3), Etaracizumab (integrin $\alpha\nu\beta3$), Farletuzumab (folate receptor 1), FBTA05 (CD20), Ficlatuzumab (SCH 900105), Figitumumab (IGF-1 receptor), Flanvotumab (glycoprotein 75), Fresolimumab (TGF-$\beta$), Galiximab (CD80), Ganitumab (IGF-I), Gemtuzumab ozogamicin (CD33), Gevokizumab (ILI$\beta$), Girentuximab (carbonic anhydrase 9 (CA-IX)), Glembatumumab vedotin (GPNMB), Ibritumomab tiuxetan (CD20), Icrucumab (VEGFR-1 ), Igovoma (CA-125), Indatuximab ravtansine (SDC1), Intetumumab (CD51), Inotuzumab ozogamicin (CD22), Ipilimumab (CD 152), Iratumumab (CD30), Labetuzumab (CEA), Lexatumumab (TRAIL-R2), Libivirumab (hepatitis B surface antigen), Lintuzumab (CD33), Lorvotuzumab mertansine (CD56), Lucatumumab (CD40), Lumiliximab (CD23), Mapatumumab (TRAIL-R1), Matuzumab (EGFR), Mepolizumab (IL5), Milatuzumab (CD74), Mitumomab (GD3 ganglioside), Mogamulizumab (CCR4), Moxetumomab pasudotox (CD22), Nacolomab tafenatox (C242 antigen), Naptumomab estafenatox (5T4), Namatumab (RON), Necitumumab (EGFR), Nimotuzumab (EGFR), Nivolumab (IgG4), Ofatumumab (CD20), Olaratumab (PDGF-R a), Onartuzumab (human scatter factor receptor kinase), Oportuzumab monatox (EpCAM), Oregovomab (CA-125), Oxelumab (OX-40), Panitumumab (EGFR), Patritumab (HER3), Pemtumoma (MUC1), Pertuzuma (HER2/neu), Pintumomab (adenocarcinoma antigen), Pritumumab (vimentin), Racotumomab (N- glycolylneuraminic acid), Radretumab (fibronectin extra domain-B), Rafivirumab (rabies virus glycoprotein), Ramucirumab (VEGFR2), Rilotumumab (HGF), Rituximab (CD20), Robatumumab (IGF-1 receptor), Samalizumab (CD200), Sibrotuzumab (FAP), Siltuximab (IL6), Tabalumab (BAFF), Tacatuzumab tetraxetan (alpha-fetoprotein), Taplitumomab paptox (CD 19), Tenatumomab (tenascin C), Teprotumumab (CD221), Ticilimumab (CTLA- 4), Tigatuzumab (TRAIL-R2), TNX-650 (IL13), Tositumomab (CD20), Trastuzumab (HER2/neu), TRBS07 (GD2), Tremelimumab (CTLA-4), Tucotuzumab celmoleukin (EpCAM), Ublituximab (MS4A1), Urelumab (4-1BB), Volociximab (integrin $\alpha 5\beta 1$), Votumumab (tumor antigen CTAA 16.88), Zalutumumab (EGFR), and Zanolimumab (CD4).

Citation of documents and studies referenced herein is not intended as an admission that any of the foregoing is pertinent prior art. All statements as to the contents of these documents are based on the information available to the applicants and do not constitute any admission as to the correctness of the contents of these documents.

EXAMPLES

The disclosure is further described in detail by reference to the following experimental examples. These examples are provided for purposes of illustration only.

Without further description, it is believed that one of ordinary skill in the art can, using the preceding description and the following illustrative examples, make and utilize the compounds of the present disclosure and practice the claimed methods. The following working examples therefore, specifically point out the preferred embodiments of the present disclosure, and are not to be construed as limiting in any way the remainder of the disclosure.

**Example 1: Materials and Methods**

The techniques and methods used herein are described herein or carried out in a manner known per se and as described, for example, in Sambrook et al., Molecular Cloning: A Laboratory Manual, 2nd Edition (1989) Cold Spring Harbor Laboratory Press, Cold Spring Harbor, N.Y. All methods including the use of kits and reagents are carried out according to the manufacturers' information unless specifically indicated.

**CAR construct**

A gamma-retroviral self-inactivating (SIN) vector pES.12-6 was used to stably overexpress the CLDN6-CAR-BBz in human T cells under the control of an internal eukaryotic promoter, the short intron-less version of the human elongation factor 1-alpha promoter (EFS - 213/+31). The vector backbone contains the MLV wild type sequences of the R- und U5-regions at the 5' and 3'-LTRs as well as the packaging region (psi and psi+). The enhancer elements in the U3-region of the 3'-LTR were eliminated (including CAAT-Box), and the TATA-Box sequence was mutated to prevent transcription initiation. The truncated version of the post-transcriptional regulatory element (PRE) of the Woodchuck Hepatitis Virus (WHV) is used to prevent the expression of unwanted viral proteins.

The CLDN6-CAR-BBz comprises a signaling peptide of human IgG, a single chain Fv-fragment of the CLDN6-specific antibody IMAB206 (Ganymed Pharmaceuticals) with a $(G_4S)_3$ linker between the heavy ($V_H$) and the light ($V_L$) chain containing a cysteine to serine substitution at position 46 of the $V_L$ (position 45 of the $V_L$ shown in the sequence listing). The scFv fragment is fused to human CD8$\alpha$ hinge and transmembrane region followed by human 4-1BB and human CD3zeta (Q14K) signaling moieties.

**Cell lines and reagents**

The human CLDN6 expressing teratoma cell line PA-1-SC12_A0201_luc_gfp was cultured in MEM-GlutaMAX medium supplemented with 10% *(v/v)* FCS (Biochrom), 1 mM Sodium pyruvate (Gibco), 1 mM MEM non-essential amino acids solution (Gibco) and 2% *(v/v)* sodium bicarbonate solution (Gibco). The cell line overexpresses HLA-A*0201, luciferase and GFP.

The ovarian carcinoma cell line OV-90-SC12 was cultured in 41.5% *(v/v)* MCDB 105 medium (Sigma-Aldrich), 41.5% *(v/v)* Medium 199 (Sigma Aldrich) supplemented with 15% *(v/v)* FCS and 2% *(v/v)* of 7.5% sodium bicarbonate solution.

The culture medium of the human melanoma cell line SK-MEL-37 is composed of 90% DMEM GlutaMAX™ (Gibco) supplemented with 10% *(v/v)* FCS.

The culture medium for MDA-MB-231 is composed of 88% (v/v) RPMI 1640 GlutaMAX™ (Gibco) and was supplemented with 10% FCS, 1 mM sodium pyruvate, 1mM MEM non-essential amino acids solution.

The human adenocarcinoma cell line 23132-87 and the human melanoma cell line MEL-526 was cultivated in 90% *(v/v)* RPMI 1640 GlutaMAX™ (Gibco) supplemented with 10% *(v/v)* heat inactivated FCS.

The culture medium for HEK-293 was composed of 90% *(v/v)* Eagle's Minimum Essential Medium (EMEM) (ATCC) and 10% *(v/v)* FCS.

SKOV-3 was cultured in 90% *(v/v)* McCoy's 5A Medium (ATCC) supplemented with 10% *(v/v)* FCS.

The human ovary cell line NIH-OVCAR-3 was cultivated in 80% *(v/v)* RPMI 1640 GlutaMAX™ (Gibco, Cat-No.

61870) supplemented with 20% *(v/v)* FCS and 0.1 % (w/v) Insulin (Sigma-Aldrich).

The human tumor cell lines LCLC-103H, COLO-699-N, JAR and NEC-8 were cultured in 90% *(v/v)* RPMI 1640 GlutaMAX™ (Gibco) supplemented with 10% *(v/v)* FCS.

Seeding and/ or splitting of the cell lines were done every 2 or 3 days.

## Peripheral blood mononuclear cells (PBMCs) and dendritic cells (DCs)

PBMCs were isolated by Ficoll®-Hypaque (1.077 g/mL Amersham Biosciences) density gradient centrifugation from buffy coats. Monocytes were enriched with anti-CD14 microbeads (Miltenyi Biotec). Immature DCs (iDCs) were differentiated by culture in DC medium consisting of RPMI 1640 GlutaMAX™, 100 U/mL penicillin, 100 µg/mL streptomycin, 1 mM sodium pyruvate, nonessential amino acids, and 5% *(v/v)* heat-inactivated human AB serum (all from Invitrogen, Karlsruhe, Germany) supplemented with 1000 U/mL h GM-CSF (Essex, Lucerne, Switzerland) and 1000 U/mL h IL-4 (Strathmann Biotech, Hamburg, Germany).

## Transduction of T cells

T cells were enriched from PBMCs by magnetic separation of CD3$^+$/CD28$^{high+}$ T cells using Dynabeads® Human T-Expander CD3/CD28 CTS. Cells were incubated with beads to CD3$^+$ T cell ratio of three to one and separated using a CTS DynaMag magnet. Enriched T cells were cultured in X-VIVO 15 medium supplemented with 5% *(v/v)* human serum in the presence of 450 U/mL rh IL-7 and 50 U/mL rh IL-15 (both from Miltenyi Biotec). Three days later CD3/CD28 beads were removed using a magnet and pre-activated T cells were transduced with retroviral vectors in the presence of Protransduzin®-A at a final concentration of 25 µg/mL. Cells were expanded until day 7 or day 10 in complete culture medium and were either directly used to assess CAR surface expression, T cell phenotype and effector functions or were cryopreserved.

## Flow cytometric analyses *in vitro* cultured cells

Cell surface expression of transduced CARs was analyzed using an Alexa-Fluor-647-conjugated idiotype-specific antibody (Ganymed Pharmaceuticals) recognizing the *scFv* fragment contained in all CLDN6-CAR constructs. CLDN6 surface expression on target cells was analyzed by staining with an Alexa-Fluor647-conjugated CLDN6-specific antibody IMAB027 (Ganymed Pharmaceuticals). Flow cytometric measurement was performed on a FACSCanto™ II flow cytometer using the FACSDiva™ software (BD Biosciences) and analysis performed using FlowJo® V10 (treestar inc.).

## Quantitative Real-Time PCR (qRT-PCR)

Total RNA was isolated from indicated cell lines using RNeasy® Mini Kit (QIAGEN). For reverse transcription of RNA in order to obtain cDNA for qRT-PCR, PrimeScript™ RT Reagent Kit with gDNA Eraser (TAKARA) was used starting with 1 µg total RNA. Quantitative Real-Time PCR was performed using QuantiTect SYBR® Green PCR kit (QIAGEN) with following primers (5'-3'): *CLDN6-for:* CTT ATC TCC TTC GCA GTG CAG, *CLDN6-rev:* AAG GAG GGC GAT GAC ACA GAG, *HPRT1-for:* TGA CAC TGG CAA AAC AAT GCA, *HPRT1-rev:* GGT CCT TTT CAC CAG CAA GCT (annealing temperature: 62°C).

## xCELLigence cytotoxicity assay

For assessment of CAR-mediated cytotoxicity the xCELLigence system (OMNI Life Science) was used. Cell index (CI) impedance measurements were performed according to the instructions of the supplier. The optimal cell density resulting in an exponential growth curve was determined for each tumor cell line. Target cells were seeded at concentrations of 2-10$^4$ cells per well in E-plate 96 PET (ACEA Biosciences Inc.). After 24 h varying numbers of CAR-transduced T cells were added in a final volume of 200 µL and monitored every 30 min for a period of up to 48 h by the xCELLigence system.

The percent specific lysis was calculated as follows:

$$(CI\ L_{min} - CI\ _{sample})/CI\ L_{min}\ \times\ 100$$

The standard deviation was calculated as follows:

$$100 \times (CI_{sample} / CI\ L_{min}) \times \{ \sqrt{[ (STDEV\ of\ CI_{sample}/ CI_{sample})^2 + (STDEV\ of\ CI\ L_{min} / CI\ L_{min})^2 ]}\}$$

The maximum cell index corresponding to the minimal lysis ($L_{min}$) was assessed after incubating target cells with effector T cells expressing a control antigen (e.g. eGFP, control-CAR).

**CFSE (Carboxyfluorescein succinimidyl ester) proliferation assay**

To determine the percentage of proliferating T cells after antigen-specific stimulation CAR-expressing T cells were labeled with 1.6 $\mu$M of the fluorescent dye carboxyfluorescein diacetate succinimidyl ester (CFSE) for 10 min at 37° C (protected from light). To remove any free dye, pure FCS was added to the cells and incubated for further 5 minutes. Cells were washed, resuspended in culture medium and cocultured with target cells using different effector to target (E:T) ratios in a total volume of 200 $\mu$L DC medium in 96-well round bottom plates. After 5 days of co-culture, cells were stained with fluorochrome-conjugated antibodies directed e.g. against CD4, CD8 and the CAR. The percentage of proliferating T cells was analyzed by flow cytometry based on the progressive halving of CFSE fluorescence within daughter cells following cell divisions using a BD FACSCanto™II flow cytometer (Becton Dickinson).

**Spheroid assay (IncuCyte®)**

Tumor spheroids were generated by culturing 1x10$^4$ PA1-SC12-A2-eGFP cells per well in MEM-GlutaMAX media supplemented with 10% *(v/v)* FCS, 1% *(v/v)* Na-Pyruvat, 1% *(v/v)* MEM NEAA and 2% *(v/v)* Na-Bicarbonat in a Corning® Costar® Ultra-Low Attachment 96 round bottom well plate for 48 h after centrifugation. CAR T cells were added (1x10$^5$/well) and eGFP-expressing tumor spheroids were imaged at 4-fold magnification and an exposure time of 300 ms to detect green fluorescence in an IncuCyte Zoom Live-content imaging system (Essen Bioscience) at 37 °C, five % $CO_2$. Images were acquired every hour for 10 days. Data was analyzed using IncuCyte analysis software to detect and quantify the total green object integrated intensity (GCU x $\mu m^2$/Image). Averages of green object counts with SD at each time point were plotted using IncuCyte analysis software.

**Generation of *in vitro* transcribed (IVT) mRNA**

*In vitro* transcriptions of antigen encoding mRNAs were based on the pST1-T7-GG-hAg-MCS-2hBg-A30LA70 plasmid backbones and derivative DNA-constructs. These plasmid constructs contain beside the full length ORF the 5' human $\alpha$-globin, two serial 3' human $\beta$-globin UTR and a poly(A) tail of 100 nucleotides, with a linker after 70 nucleotides. Antigen encoding mRNAs were generated by *in vitro* transcription as described by Holtkamp S. et al. (2006) Blood 108(13):4009-17. *In vitro* transcription of all described mRNA constructs was carried out at BioNTech RNA Pharmaceuticals GmbH.

**Generation of liposomal formulated antigen coding IVT RNA (RNA(LIP)) and *in vitro* transfection of dendritic cells**

Complexing of antigen encoding IVT RNA with liposomes was previously described in Kranz et al (2016) Nature 534(7607):396-401. A charge ratio of 1.3 to 2 of cationic DOTMA and RNA was used. Besides DOTMA the lipid fraction does contain the helper lipid DOPE in a molar ratio of 2:1 DOTMA per DOPE.

**Animal Experimental Techniques**

**Animals**

Nine to 21 week old female immunodeficient NOD.Cg-*Prkdc$^{scid}$ Il2rg$^{tm1Wjl}$*/SzJ (NSG) mice were used for *in vivo* studies. Breeding pairs were purchased from Jackson laboratory (Bar Harbour, ME, USA) and bred in the animal facility of the BioNTech AG, Germany. C57BL/6BrdCrHsd-*Tyr$^c$* mice were purchased from Envigo Labs. Age (8-10 weeks old) and sex (male or female) matched animals were used throughout the experiments. Congenic C57Bl/6-Thy1.1 mice were bred in the animal facility of the BioNTech AG, Germany. All experiments were performed under specific-pathogen-free (SPF) conditions and according to German animal experimentation regulations.

**Engraftment of Tumor Cells**

Either 5x10^6 OV90-SC12 or 5x10^5 CT26 tumor cells were subcutaneous injected (in 100 $\mu$L PBS) into the

right-side back flank of mice. Tumor growth monitoring and volume calculation were determined using a caliper and inserted into the formula V = ½ (length x width$^2$). Prior adoptive transfer of human CAR-engineered T cells, tumor-bearing were stratified using Daniels's XL Toolbox Add-in for Microsoft Excel to achieve humongous tumor volume distributions between different treatment groups.

### Adoptive Cell Transfer (ACT) of Human T cells

Different amounts of gamma-retroviral transduced total human T cells (number and frequency of CAR or GFP transgene expressing T cells is indicated in respective figures) were intravenously injected into the retrobular plexus in 200 $\mu$L PBS. Dependent on the experimental setting, either transduced T cells were directly used after *in vitro* activation and transduction process or cryopreserved transduced T cells were thawed and directly adoptively transferred after 2 times of washing with PBS into mice, respectively. Viability of every T cell product used in the experiment was >90%.

### Monitoring of human CAR T cells *in vivo* in blood

At indicated time points 50 $\mu$L peripheral blood was retrieved from the retro-orbital vein and collected into heparin containing reactions tubes (Sarstedt). Red blood cells were lysed using BD FACS Lysing solution (BD). CAR expression on surface of transferred human T cells were analyzed using hCD45-PE-Cy7 (HI30, BD), hCD4-APC-Cy7 (OKT4, BioLegend), hCD8-BV421 (RPA-T8, BD) and Alexa-Fluor 647-conjugated idiotype-specific antibody (Ganymed Pharmaceuticals). Dead cells were discriminated from the analysis using 7-AAD (Beckmann Coulter). Flow cytometric measurement was performed on a FACSCanto™ II flow cytometer using the FACSDiva™ software (BD Biosciences) and analysis performed using FlowJo® V10 (Treestar inc.).

### Retroviral gene manipulation and preparation CAR T cells for adoptive T cell transfer

Splenocytes of either naïve C57Bl/6-Thy1.1+ or Balb/c-Thy1.1+ were isolated and pre-activated by Dynabeads™ Mouse T-Activator CD3/CD28 in a bead to T cell ratio of 1:1 (Invitrogen) in the presences of 5 ng/mL recombinant human (rh) IL-7 and 5 ng/mL rh IL-15 (Miltenyi Biotec). For transduction of murine cells, MLV-E-pseudotyped retroviral supernatants were loaded onto RetroNectin (2 $\mu$g/cm$^2$)-coated non-tissue culture treated well plates according to the manufacturer's instruction (Takara Bio Inc., Otsu, Japan), with 3 repeated cycles of virus loading and centrifugation (1,300 xg, 15 °C, 15 min) for increased binding. 24h after pre-activation, 0.5-0.6x10$^6$ cells/cm$^2$ has been spun down (300 xg, 37°C, 1h) onto viral particle coated wells. After overnight cultivation, spin-down transduction was repeated with freshly viral particles coated plates. 72h after pre-activation, Dynabeads™ Mouse T-Activator CD3/CD28 were removed from culture and cells were expanded in the presence of 5 ng/mL rh IL-7 and 5 ng/mL rh IL-15. After ficoll cleaning, cells were washed twice with PBS to remove serum proteins and were then prepared for adoptive cell transfer (ACT). pES12.6 based retroviral vectors containing CLDN6-CAR-BBz encoded as well enhanced firefly luciferase (effLuc; Rabinovich et al.BA, PNAS (2008) PNAS 105(38):14342-6) and eGFP (enhanced green fluorescence protein) reporter gene, which expressed separately using 2A-splice elements (Szymczak et al. AL, Nature Biotechnology, (2004) Nat Biotechnol. 22(5):589-94) were used for transduction.

### Adoptive T cells transfer of murine T cells and RNA$_{(LIP)}$ vaccination

Gamma-retroviral transduced CAR congenic Thy1.1$^+$ T cells were intravenously *(i.v.)* transferred into total body irradiated (XRAD320) C57BL/6BrdCrHsd-*Tyr$^c$* or BALB/c donor mice, respectively. Subsequently, mice were intravenously *(i.v.)* vaccinated with an F12:RNA ratio of 1.3 : 2 of antigen encoding RNA$_{(LIP)}$ at various time points after ACT. CAR *in vivo* expansion was analyzed via whole body bioluminescence imaging and anti-tumoral efficacy was analyzed by tumor monitoring.

### *In vivo* luciferase imaging (BLI)

Expansion and distribution of CAR-effLuc-GFP transduced T cells were evaluated by *in vivo* bioluminescence imaging using the IVIS Lumina imaging system (Caliper Life Sciences). Briefly, an aqueous solution of D-luciferin (80 mg/kg body weight; Perkin Elmer) was injected *i.p.* at indicated time points after adoptive transfer of transduced T cells. 5 min thereafter, emitted photons were quantified (integration time of 1 min, binning 8). *In vivo* bioluminescence in regions of interest (ROI) were quantified as total flux (photons/sec) using IVIS Living Image 4.0 Software. The intensity of transmitted light originating from luciferase expressing cells within the animal was represented as a greyscale image, where black is the least intense and white to dark-grey the most intense bioluminescence signal. Greyscale reference images of mice were obtained under LED low light illumination. The images were superimposed using the Living Image 4.0 software.

**Statistical Analysis and Depiction of Data**

All results are represented with mean +/- SD of technical replicates or mean +/- SEM of biological replicates. The number of replicate-samples is stated in the figure description of each experiment. Unpaired two-tailed student's t-test was used for area under the curve (AUC)-comparison of two groups. All statistical analyses were performed using GraphPad PRISM 6.04. *** P $\leq$ 0.001, **** P $\leq$ 0.0001.

**Example 2: Generation and *in vitro* characterization of CLDN6-specific CARs**

For lead structure selection we generated different CAR backbones that all share the same scFv fragment derived from the CLDN6-specific antibody IMAB206-C46S, but differ in their hinge and costimulatory domains (Figure 1A). As 4-1BB co-stimulation has been shown to increase the persistence and anti-tumoral efficacy of CAR T cells, we included 2nd and 3rd generation CAR backbones containing the 4-1BB endodomain. Alternatively or in addition a modified CD28 domain (Kofler D.M. et al., (2011) Molecular Therapy 19 (4), 760-767) was incorporated known to deliver CD28-mediated co-stimulation to the engineered T cell in the absence of agonistic CD28 ligands such as B7.1 and B7.2. The scFv fragments were fused to the 4-1BB or CD28 costimulatory domains either via a modified IgG Fc (Hombach A. et al., (2010) Gene Therapy 17, 1206-1213) or a CD8$\alpha$ hinge region.

The different CLDN6-CARs were extensively characterized *in vitro* for their potential to sensitively and specifically recognize and kill CLDN6-expressing tumor cells. Among other experiments a tumor spheroid assay was performed using CAR-transduced T cells in combination with eGFP expressing PA1 tumor spheroids. Indeed an accelerated lysis of CLDN6-expressing tumor spheroids by 4-1BB containing CARs compared to a CAR carrying the CD28 domain could be detected using IncuCyte® real-time imaging (Figure 1B).

Based on the summary of results of different functional characterization studies we selected the CLDN6-CAR-CD8h-BBz as lead structure for preclinical and clinical testing as we could demonstrate highly specific and sensitive recognition of CLDN6 as well as a high potential for survival and repetitive stimulation of engineered CAR T cells. Importantly, this CAR backbone was already successfully used in several CD19-CAR T cell trials. For stable integration of our CLDN6-CAR into the T cell genome we selected the $\gamma$-retroviral self-inactivating (SIN) vector pES12.6 for stable integration of the therapeutic CLDN6-CAR into the T cell genome (Loew et al., Gene Therapy (2010) 17, 272-280).

**Example 3: Sensitivity of the CLDN6-CAR-BBz**

In order to analyze the sensitivity of CAR mediated recognition in more detail, a CLDN6-RNA titration experiment was conducted. To that aim the CLDN6-negative lung carcinoma cell line Colo699-N was transfected with titrated amounts of CLDN6-RNA, and CLDN6-CAR-mediated target cell lysis was assessed by xCELLigence cytotoxicity assay. CLDN6-CAR surface expression on transduced T cells (Figure 2A) and CLDN6 protein expression levels on transfected Colo699-N cells (Figure 2B) were assessed by flow cytometry after staining with CLDN6-CAR- and CLDN6-specific antibodies. Specific killing of CLDN6-RNA-transfected target cells by CLDN6-CAR-expressing T cells could be observed dependent on the CLDN6-RNA dose used for transfection of Colo699-N cells correlating with the number of CLDN6 molecules on the target cell surface as assessed by flow cytometry (Figure 2C). CAR T cells even mediated lysis, if very low numbers of CLDN6 molecules were expressed on the target cell surface after transfection of only 0.01 $\mu$g CLDN6-RNA, which were hardly detectable by flow cytometry.

**Example 4: Safety of the CLDN6-CAR-BBz**

In order to evaluate the safety of the CLDN6-CAR-BBz a cell line screening assay was performed. To that aim CLDN6-CAR-BBz-transduced T cells (Figure 3A) were cultured with a panel of CLDN6-positive and CLDN6-negative tumor cell lines of different tissue origins (Figure 3B) and recognition and lysis of target cells was analyzed by xCELLigence cytotoxicity assay (Figure 3C). In parallel CLDN6-mRNA and protein expression level in the target cell lines were assessed by qRT-PCR and flow cytometry, respectively (Figure 3D, E). It could be demonstrated that recognition and lysis of target cell lines is strictly correlated with CLDN6 mRNA and protein expression levels.

**Example 5: Proliferation of CAR T cells**

An essential prerequisite for the anti-tumoral efficacy of CLDN6-CAR-BBz-engineered T cells is their ability to proliferate and persist in the patient. In order to analyze, if CLDN6-CAR T cells efficiently proliferate in response to CLDN6 ectopically expressed in iDCs, a CFSE-based *in vitro* co-culture assay was performed. CLDN6-CAR-transduced T cells were labeled with CFSE and co-cultured with autologous iDCs transfected with titrated amounts of RNA lipoplexes (RNA$_{(LIP)}$) encoding either CLDN6 or a control antigen. Surface expression of the CLDN6-CAR on T cells and CLDN6 on target cells was verified by flow cytometry (Figure 4A, B). After five days of co-culture, the antigen-specific proliferation of CFSE-labeled CAR-expressing CD4+ and CD8+ T cells was analyzed by flow cytometry (Figure 4C).

The CLDN6-CAR mediated dose-dependent proliferation correlating with the amount of transfected CLDN6-RNA$_{(LIP)}$, while only background proliferation could be observed, if iDCs were transfected with control RNA$_{(LIP)}$. These data confirm that efficient antigen-specific expansion of CLDN6-CAR-BBz T cells is induced after antigen-specific stimulation.

**Example 6: *In vivo* anti-tumoral efficacy of the CLDN6-CAR-BBz**

After CAR-mediated antigen-specific induction of effector functions has been demonstrated *in vitro,* the therapeutic potential of human CLDN6-CAR-BBz T cells was investigated *in vivo* in an advanced xenograft tumor model. To this extent, immunodeficient NOD.Cg-Prkdcscid Il2rgtm1Wjl/SzJ (NSG) mice were subcutaneously engrafted with endogenously CLDN6-expressing human ovarian carcinoma cells (OV90). OV90 tumor-bearing NSG mice were then treated with a single dose of $1 \times 10^7$ CLDN6-CAR or eGFP transduced T cells (approx. $5 \times 10^8$ T cells/kg) administered i.v. (Figure 5 A). The CLDN6-CAR was expressed on about 16-18% of human CD4+ and CD8+ T cells (Figure 5B). Of note, adoptive transfer of CLDN6-CAR T cells led to complete regression of large tumors with a mean tumor volume of 170 mm$^3$ at the day of adoptive transfer, while no anti-tumoral effect could be observed in the control group (Figure 5C). This significant anti-tumoral effect correlated with persistence of CLDN6-CAR-BBz T cells in the peripheral blood of treated mice (Figure 5D).

**Example 7: *In vitro* functionality of short-term and long-term cultured CAR T cells**

As reproducible manufacturing of high-quality, clinical-grade CAR T cell products is a prerequisite for clinical testing, the GMP manufacturing process was optimized to achieve high transduction efficiencies and sufficient numbers and quality of CLDN6-CAR-expressing T cells. The transduction procedure was also simplified by reducing the number of transductions from two to one and by shorting the *ex vivo* culture time from 10 to 7 days. It has been shown that T cells cultured for a short period of time display improved efficacy compared with long-term expanded, exhausted T cells (PMID: 30030295). Accordingly, the shorting of the culture time not only saves time and cost, but most importantly should lead to an enhanced potential of engineered T cells to expand and persist in the patient.

In order to compare the *in vitro* anti-tumoral efficacy of short-term (7 days) and long-term (10 days) cultured CLDN6-CAR-BBz T cells, a long-term spheroid experiment was conducted. Assessment of CAR surface expression by flow cytometry revealed nearly comparable CAR expression levels, whereas the frequency of CAR-positive T cells was slightly lower in 7-day-cultured T cell sample (Figure 6A). In order to analyze the potential for repetitive killing, CAR T cells were co-cultured with CLDN6 and eGFP expressing PA1-SC12-A2-eGFP tumor spheroids and killing of tumor spheroids was monitored in real-time based on the eGFP signal using the IncuCyte® system. After complete eradication of the tumor spheroid a novel tumor spheroid was added (Figure 6B). It could be demonstrated that short-term cultured CAR T cells have a comparable potential for repetitive killing as long-term cultured T cells.

**Example 8: *In vivo* functionality of thawed GMP-manufactured CAR T cells**

As the GMP transduction process intended for clinical use generates a cryopreserved CAR T cell product, it was of seminal importance to validate the anti-tumoral potential of thawed CLDN6-CAR-BBz T cells engineered at the GMP facility. Furthermore, as the final GMP manufacturing process could be shortened (harvest at day 7 instead of day 10), this modification in the final protocol had to be assessed by conducting an *in vivo* study. Most importantly, thawed human CLDN6-CAR-BBz T cells demonstrated significant anti-tumoral effectivity comparable to freshly generated CAR T cells and eradicated advanced tumors with a mean tumor volume of 160 mm$^3$ in the ovarian carcinoma xenograft model (Figure 7A)., Moreover, CAR T cells harvested at day 7 or 10 that showed similar CAR surface expression (Figure 7B) were compared for their anti-tumoral efficacy in this experiment (Figure 7C). In correlation with the results of the long-term tumor spheroid experiment both CAR T cell products mediated complete tumor rejection with comparable kinetics. The anti-tumoral responses of both products correlated with the persistence of the CLDN6-CAR-BBz T cells in peripheral blood two weeks after adoptive transfer (Figure 7D).

**Example 9: Increased persistence of CLDN6-CAR-BBz transduced T cells**

The clinical success of adoptively transferred tumor reactive T cell therapy has been also positively correlated with the persistence of those cells *in vivo* (Robbins et al. (2004) J Immunol. 173(12):7125-30, Huang et al. (2005) 28(3):258-67). We therefor analyzed whether *in situ* antigen exposure could enhance the persistence of CLDN6-CAR-BBz T cells *in vivo*. Since a xenograft model is not sufficient to study long term persistence of CAR T cells due to graft-versus-host disease of human T cell to murine tissues as well as lacking of competing endogenous immune cells, persistence studies of CLDN6-CAR-BBz T cells were conducted in a murine syngeneic mouse model. Therefore, luciferase co-expressing CLDN6-CAR-BBz CAR-transduced murine T cells were adoptively transferred into mildly irritated (2.5 Gy) mice followed by repetitive administration of RNA$_{(LIP)}$ encoding either CLDN6 or a control antigen and expansion of the CAR T cell population was monitored sequentially by bioluminescence imaging (Figure 8 A) .

CLDN6-CAR-BBz T cells are able to persist (over 3 month) *in vivo* after repetitive vaccination with liposomally formulated CAR antigen *in vivo.* CAR T cells disappear over time in the control group while antigen specific restimulated CAR T cells proliferate after every RNA$_{(LIP)}$ treatment, even after a 3-4 weeks treatment pause after 3° and 4° boosting round (Fig 8 B and C). These data demonstrate that BioNTech's RNA$_{(LIP)}$ technology supports adequate CLDN6-CAR-BBz T cell activation and proliferation by providing natural co-stimulation *in situ,* which also lead to an increased persistence of expanded CLDN6-CAR-BBz T cells *in vivo.*

**Example 10: Improved anti-tumoral activity of *in vivo* expanded CLDN6-CAR-BBz T cells**

After CAR-mediated antigen-specific expansion and persistence has been demonstrated, the question arose, whether those *in* vivo-expanded CLDN6-CAR-BBz T cells also show an enhanced anti-tumoral potential compared to the non-expanded counterparts. For this purpose Balb/c mice inoculated with CLDN6$^{dim}$ - expressing colon carcinoma cell line CT26 were treated with a moderate dose of either 1x10$^6$ CLDN6-CAR-BBz or Control-CAR-BBz transduced murine T cells (3-4x10$^5$ CAR-expressing T cells). After *i.v.* administration of CAR T cells, mice received an additional vaccination with 20 μg RNA$_{(LIP)}$ encoding either for full-length CLDN6 or full-length control antigen (Figure 9A). No anti-tumoral effect has been observed in Control-CAR-BBz treated animals treated with CLDN6-RNA$_{(LIP)}$ and only a slight tumor regression could be achieved when mice were treated with the moderate dose of CLDN6-CAR-BBz T cells in combination with non-relevant ctrl RNA$_{(LIP)}$. However, mice that received the combination of the moderate CAR T cell dose together with the CLDN6-RNA$_{(LIP)}$ vaccination to achieve *in vivo* expansion of CLDN6-CAR-BBz T cells showed a remarkably enhanced tumor regression (Figure 9B).

**Example 11: Restored anti-tumoral efficacy of low-dose *in vivo* expanded CAR T cells**

Besides the potential of RNA$_{(LIP)}$-based *in vivo* CAR T cell expansion to improve an ongoing anti-tumoral response, it could also be shown that this technology is well-suited to restore the anti-tumoral potential of an insufficient CAR T cell dose. Tumor-bearing mice were treated with a 10-fold lower CAR T cell dose than needed for tumor rejection (Figure 10A). While tumor outgrowth was observed in mice treated with control RNA$_{(LIP)}$, all animals showed tumor rejection that received RNA$_{(LIP)}$ encoding for the CAR antigen after the initial transplantation (Figure 10C). Accordingly, frequencies of transplanted CAR T cells in peripheral blood were remarkably higher after vaccination (Figure 10D). Compensating an insufficient CAR T cell dose after transplantation by additional treatment with RNA$_{(LIP)}$ to achieve an *in vivo* expansion can be highly useful for at least two different scenarios: 1) poor yield of GMP-manufactured CAR T cell product (e.g. due to low lymphocyte count in the initial apheresis or other non-influenceable reasons) or 2) avoidance of SAEs by reduction of starting CAR T cell dose (especially, if the needed CAR is known to cause toxicities or in case of first-in-human dose-escalation studies).

**Claims**

1.    A chimeric antigen receptor (CAR) molecule comprising:

    i) a CLDN6 antigen binding domain, which comprises the amino acid sequence of SEQ ID NO: 35;
    ii) a hinge domain, which comprises the amino acid sequence of SEQ ID NO: 27;
    iii) a transmembrane domain, which comprises the amino acid sequence of SEQ ID NO: 28; and
    iv) an intracellular domain that comprises a 4-1BB costimulatory domain comprising the amino acid sequence of SEQ ID NO: 30, and a CD3-zeta signaling domain comprising the amino acid sequence of SEQ ID NO: 31.

2.    A nucleic acid encoding the CAR molecule of claim 1.

3.    The nucleic acid of claim 2, which is DNA or RNA.

4.    A vector comprising the nucleic acid of claim 2 or 3.

5.    The vector of claim 4, wherein the vector is selected from the group consisting of a DNA vector, an RNA vector, a plasmid, a lentivirus vector, an adenoviral vector, and a retrovirus vector.

6. The vector of claim 4 or 5, further comprising a promoter.

7. The vector of claim 6, wherein the promoter is chosen from an EF-1 promoter, a CMV IE gene promoter, an EF-1$\alpha$ promoter, an ubiquitin C promoter, or a phosphoglycerate kinase (PGK) promoter.

8. An immune effector cell, comprising:

the CAR molecule of claim 1;
the nucleic acid of claim 2 or 3; or
the vector of any one of claims 4 to 7.

9. The immune effector cell of claim 8, which is genetically modified to express the CAR.

10. A population of immune effector cells comprising multiple immune effector cells of claim 8 or 9.

11. The immune effector cell of claims 8 or 9 or the population of immune effector cells of claim 10, wherein the cells lack expression or have low expression of a functional TCR or a functional HLA.

12. A method of making an immune effector cell or a population of immune effector cells, comprising introducing the nucleic acid of claim 2 or 3 or the vector of any one of claims 4 to 7 into an immune effector cell, under conditions such that the CAR molecule is expressed.

13. The nucleic acid of claim 2 or 3, the vector of any one of claims 4 to 7, the immune effector cell of any one of claims 8, 9 and 11, or the population of immune effector cells of claim 10 or 11 for use as a medicament.

14. The nucleic acid of claim 2 or 3, the vector of any one of claims 4 to 7, the immune effector cell of any one of claims 8, 9 and 11, or the population of immune effector cells of claim 10 or 11 for use in the treatment of a cancer expressing CLDN6, wherein the treatment comprises providing an effective amount of the immune effector cell or population of immune effector cells to the subject.

15. The nucleic acid, vector, immune effector cell or population of immune effector cells for use of claim 14, wherein the cancer expressing CLDN6 is a cancer selected from the group consisting of ovarian cancer, lung cancer, gastric cancer, breast cancer, hepatic cancer, pancreatic cancer, skin cancer, melanomas, head neck cancer, sarcomas, bile duct cancer, renal cell cancer, and urinary bladder cancer.

16. The nucleic acid, vector, immune effector cell or population of immune effector cells for use of claim 14 or 15, wherein the treatment comprises contacting the immune effector cell or the population of immune effector cells with a cognate antigen molecule binding to the CLDN6 antigen binding domain.

17. A kit comprising the nucleic acid of claim 2 or 3, the vector of any one of claims 4 to 7, the immune effector cell of any one of claims 8, 9 and 11, or the population of immune effector cells of claim 10 or 11.

**Patentansprüche**

1. Chimärer Antigenrezeptor (CAR)-Molekül, das umfasst:

i) eine CLDN6-Antigenbindungsdomäne, die die Aminosäuresequenz von SEQ ID NO: 35 umfasst,
ii) eine Gelenkdomäne, die die Aminosäuresequenz von SEQ ID NO: 27 umfasst,
iii) eine Transmembrandomäne, die die Aminosäuresequenz von SEQ ID NO: 28 umfasst, und
iv) eine intrazelluläre Domäne, die eine 4-1BB-kostimulatorische Domäne, die die Aminosäuresequenz von SEQ ID NO: 30 umfasst, und eine CD3-Zeta-Signaldomäne, die die Aminosäuresequenz von SEQ ID NO: 31 umfasst, umfasst.

2. Nukleinsäure, die das CAR-Molekül nach Anspruch 1 kodiert.

3. Nukleinsäure nach Anspruch 2, die DNA oder RNA ist.

4. Vektor, der die Nukleinsäure nach Anspruch 2 oder 3 umfasst.

5. Vektor nach Anspruch 4, wobei der Vektor aus der Gruppe ausgewählt ist, die aus einem DNA-Vektor, einem RNA-Vektor, einem Plasmid, einem Lentivirus-Vektor, einem Adenovirus-Vektor und einem Retrovirus-Vektor besteht.

6. Vektor nach Anspruch 4 oder 5, der ferner einen Promotor umfasst.

7. Vektor nach Anspruch 6, wobei der Promotor aus einem EF-1-Promotor, einem CMV-IE-Gen-Promotor, einem EF-1α-Promotor, einem Ubiquitin-C-Promotor oder einem Phosphoglyceratkinase (PGK)-Promotor ausgewählt ist.

8. Immuneffektorzelle, die umfasst:

   das CAR-Molekül nach Anspruch 1,
   die Nukleinsäure nach Anspruch 2 oder 3 oder
   den Vektor nach einem der Ansprüche 4 bis 7.

9. Immuneffektorzelle nach Anspruch 8, die genetisch verändert ist, um den CAR zu exprimieren.

10. Population von Immuneffektorzellen, die mehrere Immuneffektorzellen nach Anspruch 8 oder 9 umfasst.

11. Immuneffektorzelle nach Anspruch 8 oder 9 oder Population von Immuneffektorzellen nach Anspruch 10, wobei die Zellen keine oder eine geringe Expression eines funktionellen TCR oder eines funktionellen HLA aufweisen.

12. Verfahren zum Herstellen einer Immuneffektorzelle oder einer Population von Immuneffektorzellen, das das Einbringen der Nukleinsäure nach Anspruch 2 oder 3 oder des Vektors nach einem der Ansprüche 4 bis 7 in eine Immuneffektorzelle unter Bedingungen, so dass das CAR-Molekül exprimiert wird, umfasst.

13. Nukleinsäure nach Anspruch 2 oder 3, Vektor nach einem der Ansprüche 4 bis 7, Immuneffektorzelle nach einem der Ansprüche 8, 9 und 11 oder Population von Immuneffektorzellen nach Anspruch 10 oder 11 zur Verwendung als Medikament.

14. Nukleinsäure nach Anspruch 2 oder 3, Vektor nach einem der Ansprüche 4 bis 7, Immuneffektorzelle nach einem der Ansprüche 8, 9 und 11 oder Population von Immuneffektorzellen nach Anspruch 10 oder 11 zur Verwendung bei der Behandlung von Krebs, der CLDN6 exprimiert, wobei die Behandlung die Bereitstellung einer wirksamen Menge der Immuneffektorzelle oder der Population von Immuneffektorzellen an den Patienten umfasst.

15. Nukleinsäure, Vektor, Immuneffektorzelle oder Population von Immuneffektorzellen zur Verwendung nach Anspruch 14, wobei der Krebs, der CLDN6 exprimiert, ein Krebs ist, der aus der Gruppe ausgewählt ist, die aus Eierstockkrebs, Lungenkrebs, Magenkrebs, Brustkrebs, Leberkrebs, Bauchspeicheldrüsenkrebs, Hautkrebs, Melanomen, Kopf-Hals-Krebs, Sarkomen, Gallengangskrebs, Nierenzellkrebs und Harnblasenkrebs besteht.

16. Nukleinsäure, Vektor, Immuneffektorzelle oder Population von Immuneffektorzellen zur Verwendung nach Anspruch 14 oder 15, wobei die Behandlung das Inkontaktbringen der Immuneffektorzelle oder der Population von Immuneffektorzellen mit einem kognaten Antigenmolekül umfasst, das an die CLDN6-Antigenbindungsdomäne bindet.

17. Kit, der die Nukleinsäure nach Anspruch 2 oder 3, den Vektor nach einem der Ansprüche 4 bis 7, die Immuneffektorzelle nach einem der Ansprüche 8, 9 und 11 oder die Population von Immuneffektorzellen nach Anspruch 10 oder 11 umfasst.

**Revendications**

1. Molécule de récepteur antigénique chimérique (CAR) comprenant :

   i) un domaine de liaison à l'antigène CLDN6, qui comprend la séquence d'acides aminés de SEQ ID N° : 35 ;
   ii) un domaine charnière, qui comprend la séquence d'acides aminés de SEQ ID N° : 27 ;

iii) un domaine transmembranaire, qui comprend la séquence d'acides aminés de SEQ ID N° : 28 ; et

iv) un domaine intracellulaire qui comprend un domaine co-stimulateur 4-1BB comprenant la séquence d'acides aminés de SEQ ID N° : 30, et un domaine de signalisation CD3-zéta comprenant la séquence d'acides aminés de SEQ ID N° : 31.

2. Acide nucléique codant pour la molécule CAR de la revendication 1.

3. Acide nucléique de la revendication 2, qui est de l'ADN ou de l'ARN.

4. Vecteur comprenant l'acide nucléique de l'une des revendications 2 ou 3.

5. Vecteur de la revendication 4, ledit vecteur étant choisi dans le groupe constitué par un vecteur ADN, un vecteur ARN, un plasmide, un vecteur lentivirus, un vecteur adénoviral et un vecteur rétrovirus.

6. Vecteur de l'une des revendications 4 ou 5, comprenant en outre un promoteur.

7. Vecteur de la revendication 6, dans lequel le promoteur est choisi parmi un promoteur EF-1, un promoteur du gène CMV IE, un promoteur EF-1$\alpha$, un promoteur de l'ubiquitine C ou un promoteur de la phosphoglycérate kinase (PGK).

8. Cellule effectrice immunitaire comprenant :

    la molécule CAR de la revendication 1 ;
    l'acide nucléique de l'une des revendications 2 ou 3 ; ou
    le vecteur de l'une quelconque des revendications 4 à 7.

9. Cellule effectrice immunitaire de la revendication 8, qui est génétiquement modifiée pour exprimer la CAR.

10. Population de cellules effectrices immunitaires comprenant de multiples cellules effectrices immunitaires de l'une des revendications 8 ou 9.

11. Cellule effectrice immunitaire de l'une des revendications 8 ou 9 ou population de cellules effectrices immunitaires de la revendication 10, lesdites cellules étant dépourvues d'expression d'un TCR fonctionnel ou d'un HLA fonctionnel, ou présentant une faible expression de ceux-ci.

12. Procédé de fabrication d'une cellule effectrice immunitaire ou d'une population de cellules effectrices immunitaires, comprenant l'introduction de l'acide nucléique de l'une des revendications 2 ou 3 ou du vecteur de l'une quelconque des revendications 4 à 7 dans une cellule effectrice immunitaire, dans des conditions telles que la molécule CAR est exprimée.

13. Acide nucléique de l'une des revendications 2 ou 3, vecteur de l'une quelconque des revendications 4 à 7, cellule effectrice immunitaire de l'une quelconque des revendications 8, 9 et 11, ou population de cellules effectrices immunitaires de l'une des revendications 10 ou 11, destiné(e) à être utilisé(e) en tant que médicament.

14. Acide nucléique de l'une des revendications 2 ou 3, vecteur de l'une quelconque des revendications 4 à 7, cellule effectrice immunitaire de l'une quelconque des revendications 8, 9 et 11 ou population de cellules effectrices immunitaires de l'une des revendications 10 ou 11 destiné(e) à être utilisé(e) dans le traitement d'un cancer exprimant CLDN6, ledit traitement comprenant la fourniture d'une quantité efficace de la cellule effectrice immunitaire ou de la population de cellules effectrices immunitaires au sujet.

15. Acide nucléique, vecteur, cellule effectrice immunitaire ou population de cellules effectrices immunitaires destiné(e) à être utilisé(e) selon la revendication 14, ledit cancer exprimant CLDN6 étant un cancer choisi dans le groupe constitué par le cancer de l'ovaire, le cancer du poumon, le cancer de l'estomac, le cancer du sein, le cancer du foie, le cancer du pancréas, le cancer de la peau, les mélanomes, le cancer de la tête et du cou, les sarcomes, le cancer des voies biliaires, le cancer des cellules rénales et le cancer de la vessie urinaire.

16. Acide nucléique, vecteur, cellule effectrice immunitaire ou population de cellules effectrices immunitaires destiné(e) à être utilisé(e) selon l'une des revendications 14 ou 15, ledit traitement comprenant la mise en contact de la cellule effectrice immunitaire ou de la population de cellules effectrices immunitaires avec une molécule d'antigène cognat se liant au domaine de liaison à l'antigène CLDN6.

17. Kit comprenant l'acide nucléique de l'une des revendications 2 ou 3, vecteur de l'une quelconque des revendications 4 à 7, cellule effectrice immunitaire de l'une quelconque des revendications 8, 9 et 11 ou population de cellules effectrices immunitaires de l'une des revendications 10 ou 11.

# Figure 1

**A)**

CLDN6-CAR-IgG-28z    CLDN6-CAR-CD8-BBz    CLDN6-CAR-IgG-BBz    CLDN6-CAR-IgG-28BBz

**B)**

CLDN6-CAR-IgG-28z    CLDN6-CAR-CD8-BBz    CLDN6-CAR-IgG-BBz    CLDN6-CAR-IgG-28BBz    untransduced

**Figure 2**

A)

|  | FSC-W/ FSC-A | SSC/ FSC | CD4/ CD8 | CD4/ CAR | CD8/ CAR |

Untransduced: 92.8 | 90.2 | 70.6 / 25 | 0.2 | 0.3

CLDN6-CAR-BBz: 93.1 | 91.9 | 70.1 / 25.3 | 22.7 | 23

B)

☐ 0 µg CLDN6-RNA
☐ 0.001µg CLDN6-RNA
☐ 0.01 µg CLDN6-RNA
☐ 0.1 µg CLDN6-RNA
☐ 1 µg CLDN6-RNA
■ 10 µg CLDN6-RNA

CLDN6 →

C) Specific lysis [%] vs Amount of CLDN6-mRNA [µg]

EP 4 501 351 B1

# Figure 3

**A)**

CLDN6-CAR-BBz surface expression

| SSC/ FSC | CD4/ CD8 | CD4/ CAR | CD8/ CAR |

**B)**

CLDN6+ and CLDN6- tumor cell lines

| | Name | CLDN6 pos/neg | Origin |
|---|---|---|---|
| 1 | LCLC-103H | negative | Lung |
| 2 | Mel-526 | negative | Skin |
| 3 | SK-MEL-37 | negative | Skin |
| 4 | SK-OV-3 | negative | Ovary |
| 5 | 23132-87 | negative | Gastic |
| 6 | Colo-699-N | negative | Lung |
| 7 | HEK-293 | negative | Embryonic kidney |
| 8 | MDA-MB-231 | negative | Breast |
| 9 | PA-1-SC12 | positive | Ovary |
| 10 | NIH-OvCar-3 | positive | Ovary |
| 11 | JAR | positive | Embryo |
| 12 | NEC-8 | positive | Embryo |

# Figure 3

# Figure 4

**A)**

**C)**

**B)**

**Figure 5**

A)

Tumor volume (mean): 170 mm³

NSG mice (10/group) → Tumor engraftment ← s.c. 5x 10⁶ OV90-SC12 → Adoptive T-cell transfer ← i.v. 1x 10⁷ human T cells → Monitoring:
• Tumor size
• CAR-T frequency in blood

B) **CAR surface expression** (ACT)

C) **Tumor growth** (mean±SEM)

D) **CAR-T persistence** (3 wks post ACT)

# Figure 6

A)

| | SSC/ FSC | CD4/ CD8 | CD4/ CAR | CD8/ CAR |
|---|---|---|---|---|

Day 7 CLDN6-CAR-BBz — 96.7 | 57.2 / 37.2 | 18 | 20.7

Day 10 CLDN6-CAR-BBz — 96.4 | 45.2 / 49.8 | 23.1 | 28

B)

Addion of new tumor spheroid

Green Object Integrated Intensity (GCU x µm²/lImage)

- CLDN6-CAR-BBz d7
- untransduced d7
- CLDN6-CAR-BBz d10
- untransduced d10

Time [h]

EP 4 501 351 B1

Figure 7

A)

Tumor volume (mean): 160 mm$^3$

Tumor engraftment ⟹ Adoptive T-cell transfer ⟹ Monitoring:
- Tumor size
- CAR-T frequency in blood

NSG mice (12/ group)

s.c. 5x 10$^6$ OV90-SC12

i.v. thawed 1x 10$^7$ human T cells

B) CAR surface expression (ACT)

CD4$^+$  CD8$^+$
GFP/ CAR

CLDN6-CAR-BBz
d7   21.5   24.9
d10  20.1   24.9

C) Tumor growth (mean±SEM)

☐ d7 - untransd. T cells
● d7 - CLDN6-CAR-BBz
▼ d10 - CLDN6-CAR-BBz

**** ***

D) CAR-T persistence (2 wks post ACT)

CD4$^+$  CD8$^+$
FSC/CD45  CD4/CD8  GFP/ CAR

Untransd. T cells
d7    1.2    30.5  37.3    1.4    0.0

CLDN6-CAR-BBz
d7    31.8   38.0  57.0    17.2   10.6
d10   18.1   16.8  75.5    15.0   14.7

# Figure 8

**A)**

2.5 Gy TBI

*i.v.*
5x10e6
CLDN6-CAR-BBz-Luc

*i.v.*
20 µg CLDN6/ctrl RNA$_{(LIP)}$
vaccination

Bioluminescence

d0    8    15    22    50    85

**B)**

d0    CART cells

d7

d8    CLDN6$_{(LIP)}$    Ctrl$_{(LIP)}$    1° RNA$_{(LIP)}$ vacc.

d11

d15    CLDN6$_{(LIP)}$    Ctrl$_{(LIP)}$    2° RNA$_{(LIP)}$ vacc.

d17

d22    CLDN6$_{(LIP)}$    Ctrl$_{(LIP)}$    3° RNA$_{(LIP)}$ vacc.

d25

**C)**

1° 2° 3°    4°    5° RNA$_{(LIP)}$vaccination

total flux [p/s] vs days post ACT

- ● CLDN6 RNA$_{(LIP)}$
- △ ctrl RNA$_{(LIP)}$

**Figure 9**

A)

| Tumor inocul. | TBI | ACT | Vacc. |
|---|---|---|---|
| s.c. 5x10e5 CT26 | 4 Gy | i.v. 3-4 x10e5 CAR+ T cells | i.v. 20 µg RNA(LIP) |

BALB/c mice

Tumor monitoring

-26    -6    0    1    Days post ACT

B)

Tumor volume / mm$^{-3}$ vs Days after tumor inoculation

ACT, vacc

- ●— CLDN6-CAR-BBz  + CLDN6 RNA(LIP)
- □— CLDN6-CAR-BBz  + ctrl RNA(LIP)
- ✳— Control-CAR-BBz  + CLDN6 RNA(LIP)

# Figure 10

A)

Tumor inocul. | ACT | Vacc.

s.c.
5x10e6
OV90-SC12

i.v.
10e5 CAR+
10e6 CAR+
10e7 ctrl hum T cells

i.v.
+/-20 µg
RNA(LIP)

NSG mice

-30  0  3  10  17  Tumor volume
Days post ACT

B) CAR surface expression (ACT)

CD4/CAR   CD8/CAR

Untransd. T cells — 0.3 | 0.3

CLDN6-CAR-BBz — 9.6 | 12.6

C) Tumor growth (mean±SEM)

- 1e7 ctrl T cells + CLDN6/ctrl RNA(LIP)
- 1e6 CLDN6-CAR-BBz
- 1e5 CLDN6-CAR-BBz + CLDN6 RNA(LIP)
- 1e5 CLDN6-CAR-BBz + ctrl RNA(LIP)

***

# Figure 10

D)

**CAR-T persistence (2.5 wks post ACT)**

## REFERENCES CITED IN THE DESCRIPTION

### Patent documents cited in the description

- WO 2016180778 A
- WO 2015150327 A
- WO 2016180467 A
- WO 2016150400 A
- WO 0196584 A
- WO 0129058 A
- US 6326193 B
- WO 2013143683 A

### Non-patent literature cited in the description

- **ROBBINS et al.** *J Immunol.*, 2004, vol. 173 (12), 7125-30
- **HOMBACH A. et al.** *Gene Therapy*, 2010, vol. 17, 1206-1213
- **KOFLER D.M. et al.** *Molecular Therapy*, 2011, vol. 19 (4), 760-767
- **HARLOW et al.** In: Using Antibodies: A Laboratory Manual. Cold Spring Harbor Laboratory Press, 1999
- **HARLOW et al.** Antibodies: A Laboratory Manual. Cold Spring Harbor, 1989
- **HOUSTON et al.** *Proc. Natl. Acad. Sci. USA*, 1988, vol. 85, 5879-5883
- **BIRD et al.** *Science*, 1988, vol. 242, 423-426
- **TURKSEN, K. et al.** *Dev Dyn*, 2001, vol. 222, 292-300
- **ANDERSON WJ. et al.** *Dev Dyn*, 2008, vol. 237, 504-12
- **TURKSEN K. et al.** *Development*, 2002, vol. 129, 1775-84
- **ASSOU S. et al.** *Stem Cells*, 2007, vol. 25, 961-73
- **ABUAZZA G. et al.** *Am J Physiol Renal Physiol*, 2006, vol. 291, 1132-1141
- **TROY T.C. et al.** *Molecular Biotechnology*, 2007, vol. 36, 166-74
- **ZHAO L. et al.** *Am J Physiol Regul Integr Comp Physiol*, 2008, vol. 294, 1856-1862
- **SMITH ; WATERMAN**. *Ads App. Math.*, 1981, vol. 2, 482
- **NEDDLEMAN ; WUNSCH**. *J. Mol. Biol.*, 1970, vol. 48, 443
- **PEARSON ; LIPMAN**. *Proc. Natl Acad. Sci. USA*, 1988, vol. 85, 2444
- **MILONE M.C. et al.** *Molecular Therapy*, 2009, vol. 17 (8), 1453-1464
- **SAMBROOK et al.** Molecular Cloning: A Laboratory Manual. Cold Spring Harbor Laboratory, 2001
- **SMITH et al.** *Nat. Nanotechnol.*, 2017, vol. 12, 813-820
- **EYQUEM et al.** *Nature*, 2017, vol. 543, 113-117
- Remington's Pharmaceutical Sciences. Mack Publishing Co., 1985
- **SAMBROOK et al.** Molecular Cloning: A Laboratory Manual. Cold Spring Harbor Laboratory Press, 1989
- **HOLTKAMP S. et al.** *Blood*, 2006, vol. 108 (13), 4009-17
- **KRANZ et al.** *Nature*, 2016, vol. 534 (7607), 396-401
- **RABINOVICH et al.** BA, PNAS. *PNAS*, 2008, vol. 105 (38), 14342-6
- **SZYMCZAK et al.** Nature Biotechnology. *Nat Biotechnol.*, 2004, vol. 22 (5), 589-94
- **LOEW et al.** *Gene Therapy*, 2010, vol. 17, 272-280